(19) Europäisches Patentamt
European Patent Office
Office européen des brevets

(11) EP 0 408 461 B1

(12) **FASCICULE DE BREVET EUROPEEN**

(45) Date de publication et mention de la délivrance du brevet:
**16.10.1996 Bulletin 1996/42**

(51) Int Cl.[6]: **C12N 15/53**, C12N 9/06, A61K 38/44, C12N 1/19, C12N 1/21, C12N 5/16 // (C12N1/19, C12R1:865)

(21) Numéro de dépôt: **90402023.7**

(22) Date de dépôt: **13.07.1990**

(54) **Protéine à activité urate oxydase, gène recombinant codant pour celle-ci, vecteur d'expression, micro-organismes et cellules transformées**

Protein mit Urate-Oxidase-Aktivität, dafür kodierendes rekombinantes Gen, Expressionsvektor, Mikroorganismen und Transformanten

Protein with urate oxidase activity, recombinant gene coding therefor, expression vector, micro-organisms and transformed cells

(84) Etats contractants désignés:
**AT BE CH DE DK ES FR GB GR IT LI LU NL SE**

(30) Priorité: **13.07.1989 FR 8909550**
**29.12.1989 FR 8917466**
**06.02.1990 FR 9001368**

(43) Date de publication de la demande:
**16.01.1991 Bulletin 1991/03**

(73) Titulaire: **SANOFI**
**75008 Paris (FR)**

(72) Inventeurs:
- **Caput, Daniel**
  **F-31000 Toulouse (FR)**
- **Ferrara, Pascual**
  **F-31290 Villefranche de Lauragais (FR)**
- **Guillemot, Jean Claude**
  **F-31400 Toulouse (FR)**
- **Kaghad, Mourad**
  **F-31520 Ramonville St. Agne (FR)**
- **Legoux, Richard**
  **F-31460 Caraman (FR)**
- **Loison, Gérard**
  **F-31300 Toulouse (FR)**
- **Larbre, Elizabeth**
  **F-84000 Avignon (FR)**
- **Lupker, Johannes**
  **F-31320 Castanet-Tolosan (FR)**
- **Leplatois, Pascal**
  **F-81470 Cuq Toulza (FR)**
- **Salomé, Marc**
  **F-31320 Castanet-Tolosan (FR)**
- **Laurent, Patrick**
  **F-31320 Pechbusque (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 173 378** **WO-A-88/08450**
**DE-A- 3 927 061** **GB-A- 1 198 764**

- **BIOCHEMICAL AND BIOPHYSICAL RESEARCH COMMUNICATIONS, vol. 158, no. 3, 15 février 1989, pages 991-995, Academic Press, Inc.; K. ALVARES et al.: "The nucleotide sequence of a full length cDNA clone encoding rat liver urate oxidase"**
- **PROCEEDINGS OF THE NATIONAL ACADEMY OF SCIENCES USA, vol. 85, décembre 1988, pages 9081-9085; P. GOPAL REDDY et al.: "Isolation and sequence determination of a cDNA clone for rat peroxisomal urate oxidase: Liver-specific expression in the rat"**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 408 461 B1

Printed by Jouve, 75001 PARIS (FR)

## Description

L'invention concerne une nouvelle protéine à activité urate oxydase, un médicament contenant celle-ci et les outils de génie génétique pour produire cette protéine et notamment un gène recombinant, un vecteur d'expression portant ce gène, les micro-organismes procaryotes et le cellules eucaryotes transformées par ce vecteur d'expression.

L'urate oxydase (EC 1.7.3.3.), également dénommée uricase, est une enzyme de la voie de dégradation des purines. Cette enzyme n'existe pas chez les primates (comme l'Homme), les oiseaux, quelques reptiles ni chez la plupart des insectes. Certains chiens (comme le dalmatien) en sont également dépourvus.

Chez l'homme, les bases puriques, adénine et guanine, sont converties en xanthine. La xanthine est oxydée par la xanthine oxydase pour former l'acide urique selon la réaction :

$$\text{xanthine} + H_2O + O_2 \rightarrow \text{acide urique} + O_2^-.$$

Le radical $O_2^-$, substrat de la superoxyde dismutase, est transformé par cette dernière en peroxyde d'hydrogène.

L'acide urique, métabolite présent dans le sang, se trouve normalement essentiellement sous forme de sel monosodique soluble. Toutefois, il peut arriver que, chez certaines personnes, l'acide urique précipite et forme des calculs. L'hyperuricémie, augmentation de la quantité d'acide urique en circulation dans le sang, cause le dépôt d'acide urique dans les tissus cartilagineux, ce qui entraîne la goutte. L'hyperuricémie peut également avoir des conséquences sur les reins : un excès d'acide urique dans l'urine et dans les reins peut conduire à une néphrolithiase à acide urique, c'est-à-dire à l'accumulation de calculs rénaux qui sont très douloureux et peuvent endommager le rein. Ces calculs sont composés d'acide urique éventuellement associé avec des sels phosphates et oxalates.

La surproduction d'acide urique peut avoir des origines diverses : défauts métaboliques congénitaux, syndrome de Lesch-Nyhan, l'ingestion en excès de purine ou de protéines, traitements avec des drogues uricosuriques, traitement des hémopathies, notamment cancéreuses à l'aide des cytolytiques (chimiothérapie) ou par radiothérapie, etc. (Gutman, AB et YU, T.F. (1968) Am. J. Med. 45 - 756-779).

L'urate oxydase, enzyme qui catalyse la dégradation de l'acide urique en allantoïne (composé beaucoup plus soluble que l'acide urique, ne cristallisant pas aux concentrations atteintes dans les liquides biologiques), présente donc un intérêt thérapeutique. Utilisée en injection, elle présente un grand nombre d'avantages dans le traitement de l'hyperuricémie et des néphrolithiases : rapidité de l'effet hypo-uricémiant (diminution de l'ordre de 50 % de l'hyperuricémie en moins de 24 h), meilleure protection du rein contre les lithiases par rapport à d'autres drogues comme l'allopurinol (inhibiteur de la xanthine oxydase), etc. Cette enzyme est actuellement principalement utilisée comme adjuvant des cytolytiques en chimiothérapie.

L'urate oxydase utilisée actuellement comme médicament est obtenue selon un procédé comprenant la culture d'un mycélium d'Aspergillus flavus et l'isolement de l'urate oxydase par extraction du milieu de culture ainsi que plusieurs étapes de purification partielle de cette protéine. Ce procédé, qui permet l'obtention d'urate oxydase d'activité spécifique urate oxydase voisine de 8 U/mg, dépourvue de contaminants toxiques, présente toutefois des inconvénients. En effet, la physiologie et surtout la génétique d'A. flavus ne sont pas aisément travaillables (WOLOSHUK et al, (1989) Applied environ. microbiol. vol. 55, p. 86-90). Il n'est donc pas possible d'obtenir des souches qui produisent cette enzyme en quantités importantes. D'autre part, A. flavus est susceptible de produire des aflatoxines, lesquelles sont parfois difficiles à séparer. Il convient donc de vérifier que le produit purifié est exempt de ces toxines.

Il existe donc un besoin pour une urate oxydase d'A. flavus plus pure ainsi que des outils et des techniques de génie génétique permettant de s'affranchir de ces inconvénients.

La demanderesse a purifié l'urate oxydase extraite d'A. flavus, appelée ci-après urate oxydase extractive, a un degré de pureté très supérieur à celui déjà connu de cette protéine, déterminé la séquence partielle de celle-là et construit deux pools de sondes marquées susceptibles de s'hybrider avec les nucléotides codant pour deux portions de cette protéine. Elle a ensuite construit un vecteur d'expression comprenant cet ADNc, transformé une souche d'E. coli K12 avec ce dernier, cultivé celle-ci et vérifié que le lysat des cellules contenait une protéine recombinante de la masse moléculaire attendue, qui possède une activité urate oxydase (capacité de dégrader l'acide urique en allantoine).

La demanderesse a également construit plusieurs vecteurs d'expression dans les cellules eucaryotes comprenant un gène recombinant codant pour l'urate oxydase dont la séquence comporte des variations par rapport à l'ADNc isolé, introduites dans le but de mettre en place des codons usuels dans les cellules eucaryotes, transformé différentes cellules eucaryotes à l'aide de ces vecteurs, cultivé celles-ci en faible volume ainsi qu'en volume plus important (fermenteur) et constaté que les lysats des cellules contenaient un taux important de protéine recombinante de la masse moléculaire attendue possédant une activité urate oxydase.

Elle a purifié cette protéine recombinante et caractérisé partiellement celle-ci, de façon comparative vis-à-vis de l'urate oxydase extractive.

L'invention concerne donc une nouvelle protéine caractérisée en ce qu'elle présente une activité urate oxydase

spécifique d'au moins 16 U/mg, en ce qu'elle a la séquence ci-après :

Ser Ala Val Lys Ala Ala Arg Tyr Gly Lys Asp Asn Val Arg Val Tyr Lys
Val His Lys Asp Glu Lys Thr Gly Val Gln Thr Val Tyr Glu Met Thr Val
Cys Val Leu Leu Glu Gly Glu Ile Glu Thr Ser Tyr Thr Lys Ala Asp Asn
Ser Val Ile Val Ala Thr Asp Ser Ile Lys Asn Thr Ile Tyr Ile Thr Ala

Lys Gln Asn Pro Val Thr Pro Pro Glu Leu Phe Gly Ser Ile Leu Gly Thr
His Phe Ile Glu Lys Tyr Asn His Ile His Ala Ala His Val Asn Ile Val
Cys His Arg Trp Thr Arg Met Asp Ile Asp Gly Lys Pro His Pro His Ser
Phe Ile Arg Asp Ser Glu Glu Lys Arg Asn Val Gln Val Asp Val Val Glu
Gly Lys Gly Ile Asp Ile Lys Ser Ser Leu Ser Gly Leu Thr Val Leu Lys
Ser Thr Asn Ser Gln Phe Trp Gly Phe Leu Arg Asp Glu Tyr Thr Thr Leu
Lys Glu Thr Trp Asp Arg Ile Leu Ser Thr Asp Val Asp Ala Thr Trp Gln
Trp Lys Asn Phe Ser Gly Leu Gln Glu Val Arg Ser His Val Pro Lys Phe
Asp Ala Thr Trp Ala Thr Ala Arg Glu Val Thr Leu Lys Thr Phe Ala Glu
Asp Asn Ser Ala Ser Val Gln Ala Thr Met Tyr Lys Met Ala Glu Gln Ile
Leu Ala Arg Gln Gln Leu Ile Glu Thr Val Glu Tyr Ser Leu Pro Asn Lys
His Tyr Phe Glu Ile Asp Leu Ser Trp His Lys Gly Leu Gln Asn Thr Gly
Lys Asn Ala Glu Val Phe Ala Pro Gln Ser Asp Pro Asn Gly Leu Ile Lys
Cys Thr Val Gly Arg Ser Ser Leu Lys Ser Lys Leu,

précédée éventuellement d'une méthionine.

De préférence, cette protéine a une activité urate oxydase spécifique d'environ 30 U/mg.

Une protéine de ce type appréciée est celle qui, par analyse sur un gel bidimensionnel présente un spot de masse moléculaire d'environ 33,5 kDa et de point isoélectrique voisin de 8,0 représentant au moins 90 % de la masse protéique.

De préférence le taux de pureté de cette protéine déterminé par chromatographie liquide sur une colonne de silice greffée C8 est supérieur à 80 %.

Une protéine intéressante de ce type est celle qui a un point isoélectrique voisin de 8,0. On apprécie que la sérine amino-terminale porte un groupement bloquant, de préférence de masse voisine de 43 unités de masse atomique, tel que par exemple le groupe acétyle.

L'invention a également trait au médicament qui contient, dans un véhicule pharmaceutiquement acceptable, la protéine définie précédemment. Celle-ci peut remplacer avantageusement, dans ses différentes indications, l'urate oxydase extractive d'activité urate oxydase spécifique voisine de 8 U/mg vendue en préparation injectable sous la marque Uricozyme (Vidal 1990).

L'invention concerne aussi un gène recombinant caractérisé en ce qu'il comporte une séquence d'ADN codant pour la protéine de séquence ci-après :

Met Ser Ala Val Lys Ala Ala Arg Tyr Gly Lys Asp Asn Val Arg Val Tyr
Lys Val His Lys Asp Glu Lys Thr Gly Val Gln Thr Val Tyr Glu Met Thr
Val Cys Val Leu Leu Glu Gly Glu Ile Glu Thr Ser Tyr Thr Lys Ala Asp
Asn Ser Val Ile Val Ala Thr Asp Ser Ile Lys Asn Thr Ile Tyr Ile Thr
Ala Lys Gln Asn Pro Val Thr Pro Pro Glu Leu Phe Gly Ser Ile Leu Gly
Thr His Phe Ile Glu Lys Tyr Asn His Ile His Ala Ala His Val Asn Ile
Val Cys His Arg Trp Thr Arg Met Asp Ile Asp Gly Lys Pro His Pro His
Ser Phe Ile Arg Asp Ser Glu Glu Lys Arg Asn Val Gln Val Asp Val Val
Glu Gly Lys Gly Ile Asp Ile Lys Ser Ser Leu Ser Gly Leu Thr Val Leu
Lys Ser Thr Asn Ser Gln Phe Trp Gly Phe Leu Arg Asp Glu Tyr Thr Thr
Leu Lys Glu Thr Trp Asp Arg Ile Leu Ser Thr Asp Val Asp Ala Thr Trp
Gln Trp Lys Asn Phe Ser Gly Leu Gln Glu Val Arg Ser His Val Pro Lys
Phe Asp Ala Thr Trp Ala Thr Ala Arg Glu Val Thr Leu Lys Thr Phe Ala
Glu Asp Asn Ser Ala Ser Val Gln Ala Thr Met Tyr Lys Met Ala Glu Gln
Ile Leu Ala Arg Gln Gln Leu Ile Glu Thr Val Glu Tyr Ser Leu Pro Asn
Lys His Tyr Phe Glu Ile Asp Leu Ser Trp His Lys Gly Leu Gln Asn Thr
Gly Lys Asn Ala Glu Val Phe Ala Pro Gln Ser Asp Pro Asn Gly Leu Ile
Lys Cys Thr Val Gly Arg Ser Ser Leu Lys Ser Lys Leu

A cause de la dégénérescence du code génétique, il existe un grand nombre de séquences d'ADN codant pour une protéine dont la séquence répond à la formule donnée ci-dessus. Parmi celles-ci une séquence préférée particulièrement adaptée pour une expression dans les micro-organismes procaryotes est la suivante :

ATGTCTGCGG TAAAAGCAGC GCGCTACGGC AAGGACAATG TTCGCGTCTA
CAAGGTTCAC AAGGACGAGA AGACCGGTGT CCAGACGGTG TACGAGATGA
CCGTCTGTGT GCTTCTGGAG GGTGAGATTG AGACCTCTTA CACCAAGGCC
GACAACAGCG TCATTGTCGC AACCGACTCC ATTAAGAACA CCATTTACAT
CACCGCCAAG CAGAACCCCG TTACTCCTCC CGAGCTGTTC GGCTCCATCC
TGGGCACACA CTTCATTGAG AAGTACAACC ACATCCATGC CGCTCACGTC
AACATTGTCT GCCACCGCTG GACCCGGATG GACATTGACG GCAAGCCACA
CCCTCACTCC TTCATCCGCG ACAGCGAGGA GAAGCGGAAT GTGCAGGTGG

ACGTGGTCGA GGGCAAGGGC ATCGATATCA AGTCGTCTCT GTCCGGCCTG
ACCGTGCTGA AGAGCACCAA CTCGCAGTTC TGGGGCTTCC TGCGTGACGA
GTACACCACA CTTAAGGAGA CCTGGGACCG TATCCTGAGC ACCGACGTCG
ATGCCACTTG GCAGTGGAAG AATTTCAGTG GACTCCAGGA GGTCCGCTCG
CACGTGCCTA AGTTCGATGC TACCTGGGCC ACTGCTCGCG AGGTCACTCT
GAAGACTTTT GCTGAAGATA ACAGTGCCAG CGTGCAGGCC ACTATGTACA
AGATGGCAGA GCAAATCCTG GCGCGCCAGC AGCTGATCGA GACTGTCGAG
TACTCGTTGC CTAACAAGCA CTATTTCGAA ATCGACCTGA GCTGGCACAA
GGGCCTCCAA AACACCGGCA AGAACGCCGA GGTCTTCGCT CCTCAGTCGG
ACCCCAACGG TCTGATCAAG TGTACCGTCG GCCGGTCCTC TCTGAAGTCT
AAATTG.

Une autre séquence d'ADN préférée qui convient notamment pour l'expression dans les cellules eucaryotes, telles que la levure, est la suivante :

ATGTCTGCTG TTAAGGCTGC TAGATACGGT AAGGACAACG TTAGAGTCTA
CAAGGTTCAC AAGGACGAGA AGACCGGTGT CCAGACGGTG TACGAGATGA
CCGTCTGTGT GCTTCTGGAG GGTGAGATTG AGACCTCTTA CACCAAGGCC
GACAACAGCG TCATTGTCGC AACCGACTCC ATTAAGAACA CCATTTACAT
CACCGCCAAG CAGAACCCCG TTACTCCTCC CGAGCTGTTC GGCTCCATCC
TGGGCACACA CTTCATTGAG AAGTACAACC ACATCCATGC CGCTCACGTC
AACATTGTCT GCCACCGCTG GACCCGGATG GACATTGACG GCAAGCCACA
CCCTCACTCC TTCATCCGCG ACAGCGAGGA GAAGCGGAAT GTGCAGGTGG
ACGTGGTCGA GGGCAAGGGC ATCGATATCA AGTCGTCTCT GTCCGGCCTG
ACCGTGCTGA AGAGCACCAA CTCGCAGTTC TGGGGCTTCC TGCGTGACGA
GTACACCACA CTTAAGGAGA CCTGGGACCG TATCCTGAGC ACCGACGTCG
ATGCCACTTG GCAGTGGAAG AATTTCAGTG GACTCCAGGA GGTCCGCTCG
CACGTGCCTA AGTTCGATGC TACCTGGGCC ACTGCTCGCG AGGTCACTCT
GAAGACTTTT GCTGAAGATA ACAGTGCCAG CGTGCAGGCC ACTATGTACA
AGATGGCAGA GCAAATCCTG GCGCGCCAGC AGCTGATCGA GACTGTCGAG
TACTCGTTGC CTAACAAGCA CTATTTCGAA ATCGACCTGA GCTGGCACAA
GGGCCTCCAA AACACCGGCA AGAACGCCGA GGTCTTCGCT CCTCAGTCGG
ACCCCAACGG TCTGATCAAG TGTACCGTCG GCCGGTCCTC TCTGAAGTCT
AAATTG.

Une autre séquence d'ADN préférée qui convient notamment pour une expression dans les cellules animales est la suivante :

5'-ATGTC CGCAGTAAAA GCAGCCCGCT ACGGCAAGGA
CAATGTCCGC GTCTACAAGG TTCACAAGGA CGAGAAGACC GGTGTCCAGA
CGGTGTACGA GATGACCGTC TGTGTGCTTC TGGAGGGTGA GATTGAGACC
TCTTACACCA AGGCCGACAA CAGCGTCATT GTCGCAACCG ACTCCATTAA
GAACACCATT TACATCACCG CCAAGCAGAA CCCCGTTACT CCTCCCGAGC
TGTTCGGCTC CATCCTGGGC ACACACTTCA TTGAGAAGTA CAACCACATC
CATGCCGCTC ACGTCAACAT TGTCTGCCAC CGCTGGACCC GGATGGACAT
TGACGGCAAG CCACACCCTC ACTCCTTCAT CCGCGACAGC GAGGAGAAGC
GGAATGTGCA GGTGGACGTG GTCGAGGGCA AGGGCATCGA TATCAAGTCG
TCTCTGTCCG GCCTGACCGT GCTGAAGAGC ACCAACTCGC AGTTCTGGGG
CTTCCTGCGT GACGAGTACA CCACACTTAA GGAGACCTGG GACCGTATCC
TGAGCACCGA CGTCGATGCC ACTTGGCAGT GGAAGAATTT CAGTGGACTC
CAGGAGGTCC GCTCGCACGT GCCTAAGTTC GATGCTACCT GGGCCACTGC
TCGCGAGGTC ACTCTGAAGA CTTTTGCTGA AGATAACAGT GCCAGCGTGC
AGGCCACTAT GTACAAGATG GCAGAGCAAA TCCTGGCGCG CCAGCAGCTG
ATCGAGACTG TCGAGTACTC GTTGCCTAAC AAGCACTATT TCGAAATCGA
CCTGAGCTGG CACAAGGGCC TCCAAAACAC CGGCAAGAAC GCCGAGGTCT
TCGCTCCTCA GTCGGACCCC AACGGTCTGA TCAAGTGTAC CGTCGGCCGG
TCCTCTCTGA AGTCTAAATT G

précédée d'une séquence 5′ non traduite favorable à l'expression dans les cellules animales. Une telle séquence 5′ non traduite préférée est celle qui comprend la séquence : AGCTTGCCGCCACT, située immédiatement en amont de la séquence explicitée ci-dessus.

On notera que la protéine codée par les séquences d'ADNc données ci-dessus peut subir une maturation par la méthionyl-amino-peptidase, qui la clive de son résidu méthionine amino-terminal.

L'invention concerne également un vecteur d'expression qui porte, avec les moyens nécessaires à son expression, le gène recombinant défini précédemment.

Pour une expression dans les micro-organismes procaryotes, en particulier dans Escherichia coli, la séquence codante doit être insérée dans un vecteur d'expression comportant notamment un promoteur efficace, suivi d'un site de fixation des ribosomes en amont du gène à exprimer, ainsi qu'une séquence d'arrêt de transcription efficace en aval du gène à exprimer. Ce plasmide doit également comporter une origine de réplication et un marqueur de sélection. Toutes ces séquences doivent être choisies en fonction de la cellule hôte.

Pour une expression dans les cellules eucaryotes, le vecteur d'expression selon l'invention porte le gène recombinant défini précédemment avec les moyens nécessaires à son expression, à sa réplication dans les cellules eucaryotes et à la sélection des cellules transformées. De préférence, ce vecteur porte un marqueur de sélection, choisi par exemple pour complémenter une mutation des cellules eucaryotes réceptrices, qui permet la sélection des cellules qui ont intégré le gène recombinant en un nombre de copies élevé soit dans leur génome, soit dans un vecteur multicopie.

Pour une expression dans les cellules animales, notamment dans les cellules d'ovaires de hamster chinois CHO, la séquence codante est insérée dans un plasmide (par exemple dérivé du pBR 322) comportant deux unités d'expression, une première unité dans laquelle est inséré le gène recombinant devant un promoteur efficace (par exemple le promoteur précoce de SV40). La séquence autour de l'ATG d'initiation est de préférence choisie en fonction de la séquence consensus décrite par KOZAK (M. KOZAK (1978) Cell., 15, 1109-1123). Une séquence intronique, par exemple l'intron de l'α-globine de souris, peut être insérée en amont du gène recombinant ainsi qu'une séquence comportant un site de polyadénylation, par exemple une séquence de polyadénylation du SV40, en aval du gène recombinant. La deuxième unité d'expression comporte un marqueur de sélection (par exemple une séquence d'ADN) codant pour la dihydrofolate réductase (enzyme ci-après abrégée DHFR). Le plasmide est transfecté dans les cellules animales, par

exemple les cellules CHO DHFR$^-$ (incapables d'exprimer la DHFR). Une lignée est sélectionnée pour sa résistance au méthotréxate : elle a intégré dans son génome un nombre élevé de copies du gène recombinant et exprime ce dernier à un niveau suffisant.

Pour une expression dans les cellules eucaryotes telles que la levure, par exemple Saccharomyces cerevisiae, il convient d'insérer la séquence codante entre, d'une part, des séquences reconnues comme promoteur efficace, d'autre part, un terminateur de transcription. L'ensemble promoteur-séquence codante-terminateur, appelé cassette d'expression, est cloné soit dans un vecteur plasmidique (monocopie ou polycopie pour la levure, soit intégré en multicopie dans le génome de la levure.

L'invention a également trait aux cellules eucaryotes transformées par le vecteur d'expression précédent. Parmi celles-ci, on apprécie les souches de l'espèce Saccharomyces cerevisiae, en particulier celles qui comportent une mutation sur l'un des gènes responsables de la synthèse de la leucine ou de l'uracile, par exemple le gène LEU2 ou le gène URA3.

L'invention a également trait aux cellules animales contenant, avec les moyens nécessaires à son expression, ce gène recombinant. Ce dernier peut, par exemple, avoir été introduit dans les cellules par transfection par le vecteur d'expression ci-dessus, par infection au moyen d'un virus ou d'un rétro-virus le portant, ou par microinjection.

L'invention a également pour objet un procédé d'obtention d'urate oxydase recombinante qui comprend les étapes de :

1) mise en culture d'une souche telle que définie ci-dessus :
2) lyse de cellules ;
3) isolement et purification de l'urate oxydase recombinante contenue dans le lysat.

L'invention sera mieux comprise à l'aide des exemples ci-après :

une grande partie de l'ensemble des techniques ci-après, bien connues de l'homme de l'art, est exposée en détail dans l'ouvrage de Maniatis et al : "Molecular cloning : a laboratory manual" publié en 1984 par les éditions Cold Spring Harbor Press à New York.

EXEMPLE 1 : Isolement des ARN messagers d'Aspergillus flavus

La souche d'A. flavus productrice d'urate oxydase a été cultivée dans des conditions de production d'urate oxydase, c'est-à-dire dans un milieu contenant de l'acide urique de composition suivante : glucose 15 g/l, $MgSO_4$,$7H_2O$ 1 g/l, $KH_2PO_4$ 0,75 g/l, $CaCO_3$ 1,2 g/l, acide urique 1,2 g/l, KOH 0,5 g/l, huile de soja 0,66 ml/l, $FeSO_4$,$7H_2O$ 10 mg/l, $CuSO_4$, $5H_2O$ 1 mg/l, $ZnSO_4$,$7H_2O$ 3 mg/l, $MnSO_4$,$H_2O$ 1 mg/l.

Le milieu est ajusté à pH 7 avec $H_2SO_4$ 1M et est stérilisé à 120°C pendant 80 min.

Dans un erlenmeyer de 5 1 on ensemence 1,5 l de milieu avec environ 1 à $3.10^7$ spores.

La culture est incubée pendant environ 40 h à 30°C sous agitation (120 tr/min). Le mycélium est récupéré par filtration sur gaze, lavé avec de l'eau et congelé dans l'azote liquide.

15 g de mycélium (poids humide) sont décongelés et resuspendus dans 45 ml de tampon de lyse puis repris dans un même volume de billes (0,45 µm de diamètre). Le tampon de lyse est constitué de thiocyanate de guanidine 4 M, Tris-HCl 10 mM pH 7,6, EDTA 10 mM, β-mercaptoéthanol 50 ml/l. La suspension mycélienne est broyée au broyeur Zellmühle (vibrogène) pendant 5 min.

Le broyat est récupéré et les billes décantées. Le surnageant est prélevé (environ 45 ml) et ramené à 3 M final en chlorure de lithium et conservé à 0°C.

Après deux jours, on le centrifuge pendant 60 min à 10 000 tr/min. Le surnageant est écarté et le culot est repris dans 40 ml de LiCl 3M et recentrifugé à 10 000 tr/min pendant 1 h 30.

On ajoute la protéinase K (SIGMA), 40 µg/ml du SDS (0,1 % P/V) et de l'EDTA à 20 mM. On incube à 37°C pendant 3 h. On précipite avec 2 volumes d'éthanol, puis on effectue un lavage à l'éthanol 70 %. On reprend le culot dans 0,5 ml de tampon TE (tris HCl 10 mM EDTA, 1 mM pH 7,5), on extrait 2 fois au chloroforme, on précipite à l'éthanol. Les ARN sont conservés à -80°C dans l'alcool.

EXEMPLE 2 : Purification de la fraction poly $A^+$ des ARN

Environ 1 mg d'ARN est précipité 20 min à 4°C (15 000 tr/min) puis lavé avec de l'éthanol 70 % puis séché.

Le culot est repris dans 1 ml de tampon TE et resuspendu par agitation au vortex. L'oligo dT-cellulose type 3 (commercialisé par Collaborative Research Inc, Biomedicals Product Division) est préparé suivant les recommandations du fabricant. L'ARN est déposé sur l'oligo dT, agité doucement pour remettre en suspension les billes, puis chauffé pendant 1 min à 65°C.

La suspension est ajustée à 0,5 M NaCl puis mise à agiter doucement pendant 10 min. La suspension est alors

centrifugée pendant 1 min à 1 000 tr/min, le surnageant est éliminé, le culot est lavé 2 fois avec 1 ml de tampon TE contenant 0,5 M NaCl. Les surnageants sont éliminés. L'élution de la fraction polyadénylée des ARN (constituée des ARN messagers) est obtenue par suspension des billes dans 1 ml de tampon TE, puis chauffage de cette suspension à 60°C pendant 1 min, suivi d'une agitation pendant 10 min sur plaque basculante. On centrifuge ensuite 1 min à 1 000 tr/min, ce qui permet de récupérer d'une part le surnageant contenant des ARNm libres en solution et d'autre part le culot de billes de cellulose. L'ensemble des opérations ci-dessus (à partir de l'élution) est répété. Les surnageants ainsi obtenus sont rassemblés, on élimine l'excès de billes par centrifugation et on précipite le surnageant à l'éthanol contenant du NaCl suivant les techniques habituelles. (Maniatis : op. prec.).

EXEMPLE 3 : Constitution de la banque des ADNc

A partir des ARN messagers isolés comme décrit dans l'exemple précédent, on a réalisé une banque d'ADNc dans le vecteur pTZ19R (commercialisé par PHARMACIA). Ce vecteur est un plasmide comprenant un polylinker contenant des sites uniques de restriction.

La technique de clonage utilisée est celle décrite par Caput et al, (technique de l'amorce-adapteur (Primer-adapter) : (Caput et al, Proc. Natl. Acad. Sci. (U.S.A.) (1986) 83, 1670-1674)).

Elle consiste d'une part à digérer le vecteur par Pst1, ajouter une queue de polydC sur l'extrémité 3' protubérante, puis à digérer les plasmides ainsi obtenus par BamHI. Le fragment correspondant au vecteur est purifié sur colonne de Sépharose CL4B (Pharmacia). Il comprend donc une queue polydC à une extrémité, l'autre extrémité étant cohésive, du type BamHI. D'autre part, les ARN messagers sont soumis à la transcription inverse à partir d'une amorce dont la séquence est la suivante 5'<GATCCGGGCCCT$_{(12)}$)<3. Ainsi, les ADNc présentent-ils à leur extrémité 5' la séquence GATCC complémentaire de l'extrémité cohésive BamHI.

Les hybrides ARN-ADN obtenus par action de la transcriptase inverse sont soumis à une hydrolyse alcaline qui permet de se débarrasser de l'ARN. Les ADNc simple brin sont alors purifiés par 2 cycles sur colonne de Sépharose CL4B et soumis à un traitement à la terminal transférase de façon à ajouter des polydG en 3'. Les ADNc sont insérés sous forme simple brin dans le vecteur préparé comme décrit plus haut. Un second oligonucléotide "l'adapter" complémentaire de l'amorce est nécessaire pour générer un site BamHI "ouvert" à l'extrémité 5' des ADNc. Après hybridation du vecteur, de l'ADNc et de l'"adapter", les molécules recombinantes sont circularisées par l'action de la ligase du phage T4. Les régions simple brin sont alors réparées grâce à l'ADN polymérase du phage T4. Le pool de plasmides ainsi obtenu sert à transformer la souche MC 1061 pour la résistance à l'ampicilline (Casabadan Chou et Cohen J. Bact. (1980) 143 pages 971-980).

EXEMPLE 4 : Purification de l'urate oxydase extractive d'A. flavus et caractérisation de celle-ci

1) Purification de l'urate oxydase extractive d'A. flavus.

Une préparation d'urate oxydase extractive d'A. flavus (uricozyme - Laboratoires Clin Midy) présentant une activité urate oxydase spécifique de 8 U/mg (l'activité spécifique urate oxydase est le rapport entre l'activité urate oxydase mesurée selon le test décrit à l'exemple 9 et la masse de protéines totales mesurée par la méthode de Bradford : Anal. Biochem., 72, 248-254) a été repurifiée par chromatographie sur une colonne d'agarose greffé Redagarose 120 (SIGMA), concentration par ultrafiltration et filtration sur un gel polyacrylamide-agarose Ultrogel ACA 44 (IBF), selon le protocole ci-après :

Etape 1 : Chromatographie d'affinité sur agarose greffé

| | |
|---|---|
| Température : | 4°C |
| Colonne : | PHARMACIA K50/30<br>- diamètre = 50 mm<br>- longueur = 33 cm |
| Résine : | Red 120 Agarose (3 000 CL/R-0503 SIGMA) (volume de gel = 410 ml, hauteur de gel = 20 cm) |
| Tampon d'équilibrage : | glycine/NaOH 20 mM pH 8,3 |
| Tampon d'élution : | glycine/NaOH 20 mM, NaCl 2M pH 8,3 |
| Débit de mise en conditionnement : | 250 $ml.h^{-1}$ |
| Débit de fonctionnement : | 160 $ml.h^{-1}$ |
| Débit d'élution : | 60 $ml.h^{-1}$ |

1) Déposer en tête de colonne, à l'aide d'une pompe à débit constant la solution d'Uricozyme
2) Après adsorption, laver la colonne par 2 fois son volume en tampon d'équilibrage
3) Eluer par un gradient de force ionique ayant la composition suivante :
glycine, NaOH, 20 mM pH 8,3/glycine, NaOH, 20 mM + NaCl 2M pH 8,3
Le volume total du gradient est égal à 10 fois le volume de la colonne, réparti pour moitié dans chacun des constituants.
L'enregistrement chromatographique est opéré à $\lambda$ = 280 nm ; le pool urate oxydase est collecté après regroupement des fractions présentant une activité urate oxydase spécifique supérieure ou égale à 16 U/mg.

Etape 2 : Concentration du pool urate oxydase par ultrafiltration à l'aide d'un système Biopass comportant une membrane d'ultrafiltration de 10 kDa.

Etape 3 :

| | |
|---|---|
| Température : | 4°C |
| Colonne : | PHARMACIA K 50/100<br>- diamètre = 50 mm<br>- longueur = 100 cm |
| Résine : | Polyacrylamide agarose avec groupements amine et hydroxyle : l'Ultrogel ACA 44 (IBF)<br>- volume de gel = 1,6 l<br>- hauteur de gel = 80 cm |
| Tampon d'équilibrage : | glycine/NaOH 20 mM pH 8,3 |
| Débit de mise en conditionnement : | 40 $ml.h^{-1}$ |
| Débit de fonctionnement : | 24 $ml.h^{-1}$ |

1) Déposer en tête de colonne, à l'aide d'une pompe à débit constant, le pool urate oxydase concentré.
2) Après dépôt de l'échantillon, continuer d'alimenter la colonne avec du tampon glycine/NaOH 20 mM pH 8,3.
3) Après chromatographie, laver avec du NaCl 2M jusqu'à une valeur d'absorbance U.V. ($\lambda$ = 280 nm) < 0,05.
Stocker sous NaCl 2 M à 4°C.
L'enregistrement chromatographique est opéré à $\lambda$ = 280 nm ;
le pool urate oxydase est collecté après regroupement des fractions présentant conjointement :

- une activité urate oxydase spécifique supérieure ou égale à 20 U/mg.
- 2 bandes seulement en électrophorèse dans des conditions dénaturantes (présence de S.D.S) et révélation au nitrate d'argent (kit de coloration Biorad), avec :
  . une bande majeure de 33-34 kDa
  . une bande mineure de 70-71 kDa

2) Caractérisation de l'urate oxydase extractive d'A. flavus purifiée :

a) séquençage partiel

Afin d'obtenir des informations sur la séquence des acides aminés de l'urate oxydase extractive purifiée, permettant la synthèse des sondes nécessaires au clonage de l'ADNc, un séquençage amino-terminal direct de la protéine a été tenté. Ce dernier n'a pas abouti, à cause d'un blocage amino-terminal de la protéine (Cf. f) ci-dessous).

La stratégie ci-après a donc été développée pour l'obtention de la séquence partielle de l'urate oxydase :

- coupure de la protéine par des enzymes protéolytiques (à l'aide des enzymes trypsine et protéase V8 de Staphylococcus aureus)
- séparation des polypeptides obtenus par HPLC phase inverse
- séquençage des peptides purifiés.

α) Hydrolyse de l'urate oxydase par la trypsine, purification et séquençage des peptides :

l'urate oxydase à 9 mg/ml dans un tampon de carbonate d'ammonium 100 mM pH 8,9 a été digérée par la trypsine (Worthington, TPCK) avec un rapport urate oxydase/trypsine de 30/1 en poids à 30°C pendant 24 h. Après l'hydrolyse tryptique, 60 µg d'urate oxydase digérée ont été directement injectés sur une colonne HPLC phase inverse de silice greffée Brownlee G18 (colonne 10 x 0,2 cm), équilibrée en acétonitrile 1 % (v/v), acide trifluoroacétique 0,1 % (v/v) dans l'eau. Les peptides ont été ensuite élués par un gradient linéaire d'acétonitrile dans une solution d'acide trifluoroacétique (0,1 % v/v) dans l'eau variant de 1 % à 60 % d'acétonitrile en 60 min, avec un débit de 150 µl/min. Les peptides à la sortie de la colonne ont été détectés par mesure de la densité optique à 218 nm.

Le profil d'élution est présenté dans la figure 1, dans laquelle les numéros suivant la lettre T (Trypsine) correspondent aux pics identifiés.

Chaque pic a été collecté et conservé à -20°C jusqu'au moment de l'analyse sur un séquenceur de protéines (le modèle 470 A d'Applied Biosystems), équipé d'un chromatographe (modèle 430 A d'Applied Biosystems) qui analyse en continu les dérivés phénylthiohydantoïques formés, après chaque cycle de dégradation.

Le tableau (1) ci-après présente les séquences peptidiques des 9 pics identifiés.

β) Hydrolyse de l'urate oxydase par la protéase V8, purification et séquençage des peptides.

L'urate oxydase, à une concentration de 2 mg/ml dans un tampon acétate d'ammonium 100 mM pH 6,8, a été digérée par la protéase V8 de Staphylococcus aureus (Boehringer-Mannheim) avec un rapport urate oxydase/protéase V8 de 60/1 à 30°C pendant 72 h. 160 µg d'urate oxydase digérée ont ensuite été injectés sur une colonne HPLC phase inverse de silice greffée Brownlee G18 (colonne 10 x 0,2 cm) ; particules (7 x 0,03 µm) équilibrée en acétonitrile 1 %, acide trifluoroacétique 0,1 % (v/v) dans l'eau. Les peptides ont ensuite été élués par un gradient linéaire d'acétonitrile dans une solution d'acide trifluoroacétique dans l'eau (0,1 % (v/v)) variant de 1 % à 60 % d'acétonitrile en 60 min, avec un débit de 150 µl/ml. Les peptides à la sortie de la colonne ont été détectés par mesure de la densité optique à 218 nm.

Le profil d'élution est présenté dans la figure 2, dans laquelle les numéros suivant la lettre V (protéase V8) correspondent aux pics identifiés.

Chaque pic a été collecté et conservé à -20°C jusqu'au moment de l'analyse sur le séquenceur de protéines déjà mentionné.

Le tableau (1) ci-après présente les séquences peptidiques des 5 pics identifiés.

TABLEAU (1)

| | | Séquençage des produits obtenus par hydrolyse |
|---|---|---|
| A l'aide de la trypsine | T 17 | Asn - Val - Gln - Val - Asp - Val - Val - Glu - Gly - Lys |
| | T 20 | Asn - Phe - Ser - Gly - Leu - Gln - Glu - Val |
| | T 23 | Phe - Asp - Ala - Thr - Trp - Ala |
| | T 27 | His - Tyr - Phe - Glu - Ile - Asp - Leu - Ser |
| | T 28 | Ile - Leu - Ser - Thr - Asp - Val - Asp - Ala - Thr - Trp - Gln - Trp - Lys |
| | T 29 | His - Tyr - Phe - Glu - Ile - Asp - Leu - Ser - Trp - His - Lys |
| | T 31 | Ser - Thr - Asn - Ser - Gln - Phe - Trp - Gly - Phe - Leu - Arg |
| | T 32 | Gln - Asn - Pro - Val - Thr - Pro - Pro - Glu - Leu - Phe - Gly - Ser - Ile - Leu - Gly - Thr |
| | T 33 | Gln - Asn - Pro - Val - Thr - Pro - Pro - Glu - Leu - Phe - Gly - Ser - Ile - Leu - Gly - Thr |
| A l'aide de la protéase V8 | V 1 | Tyr - Ser - Leu - Pro - Asn - Lys - His - Tyr - Phe - Glu - Ile - Asp - Leu - Ser - Trp - His - Lys |
| | V 2 | Val - Thr - Leu - Lys - Thr - Phe - Ala - Glu - Asp - Asn - Ser - Ala - Ser - Val - Gln - Ala |
| | V 3 | Thr - Ser - Tyr - Thr - Lys - Ala - Asp - Asn - Ser - Val - Ile - Val - Ala - Thr - Asp - Ser - Ile - Lys - Asn - Thr - Ile - Tyr - Ile - Thr |
| | V 5 | Gly - Lys - Gly - Ile - Asp - Ile - Lys - Ser - Ser - Leu - Ser - Gly - Leu - Thr - Val - Leu - Lys - Ser - Thr - Asn - Ser - Gln - Phe - Trp - Gly - Phe - Leu - Arg |
| | V 6 | Gly - Lys - Gly - Ile - Asp - Ile - Lys - Ser - Ser - Leu - Ser - Gly - Leu - Thr - Val - Leu - Lys |

b) Activité spécifique :

L'urate oxydase extractive purifiée présente une activité spécifique d'environ 30 U/mg.

c) Electrophorèse en conditions dénaturantes

L'électrophorèse de l'urate oxydase extractive purifiée sur gel de polyacrylamide en présence de SDS (sodium dodécylsulfate), suivie d'une révélation à l'argent, permet de voir une bande de forte intensité d'environ 33-34 kDa et une bande de très faible intensité d'environ 70-71 kDa.

d) Détermination du point isoélectrique :

mode opératoire :

- Utilisation des gels prêts à l'emploi, les LKB Ampholines Gel Plates de Pharmacia de gammes de pH : (3,5-9,5) et (5-8).
- Dépot de 10 µl de protéines de témoins LKB (gamme des points isoélectriques des protéines témoins : 3,5-9,5) et d'urate oxydase purifiée 4 µg et 8 µg (sur deux pistes différentes).
- Run 1 h 30, 12V, 6°C.
- Puis coloration au bleu de Commassie (0,1 %) dans (25 % Ethanol, 8 % Acide Acétique) de façon à colorer les protéines, suivie d'une décoloration à l'aide d'une solution contenant 25 % Ethanol et 8 % Acide Acétique (pour éliminer le bruit de fond).
- Résultats : Observation sur chacune de deux pistes de deux bandes rapprochées (doublet) de points isoélectriques 8,1 et 7,9.

e) Analyse en gel bidimensionnel

L'analyse en gel bidimensionnel permet de séparer les protéines dans un premier temps d'après leurs points isoélectriques et dans un deuxième temps d'après leurs masses moléculaires.

Protocole

Echantillon : solution d'urate oxydase extractive purifiée dans un tampon glycine 20 mM de pH 8,3.

Préparation de l'échantillon

- Deux échantillons de 5 µg et 10 µg d'urate oxydase ;
- Séchage par centrifugation sous vide, reprise dans 5 µl du tampon de lyse de composition suivante :

urée 2,5 M, / 3-(3-cholamidopropyl) diméthylammonio) 1-propane sulfonate CHAPS (Sigma) / 2 % (v/v), / Amphotères Ampholines (LKB) de gamme de pH 5-8 et 3,5-9,5 / 0,4 % et β mercaptoéthanol 5 %.

Gel d'isoélectrofocalisation

- Préparation d'une solution contenant : urée 9,5 M, CHAPS 5 %, Ampholines LKB (pH (3,5-9,5) 1 % ; pH (5-8) 1 %), Acrylamide/bisacrylamide (28,4 %/1,7 %) 3,5 % final, $H_2O$.
- Solution filtrée et dégazée puis addition de 0,075 % de tétraméthyléthylène diamine Temed (Pharmacia) et de 0,015 % persulfate d'ammonium.
- Solution coulée dans des tubes (16 x 0,12 cm) - polymérisation une nuit à 20°C.
- Solution cathodique : NaOH 0,1 M dégazée. Solution anodique : $H_3PO_4$ 25 mM.
- Pré run 45 min 4 mA (voltage 300 V → 1 000 V).
- Dépôt des échantillons au niveau de la cathode.
- Run 19 h à 1 000 V à 20°C.
- Gels démoulés et équilibrés 10 min à 20°C dans un tampon (Tris 0,375 M pH 8,8 ; SDS 3 %, Dithiothréitol DTT 50 mM).

Gel dénaturant PAGE/SDS

- Préparation d'une solution contenant : Acrylamide/bisacrylamide (30 %/0,8 %) 15 % final, tris-HCl (pH 8,8) 0,375 M, $H_2O$.
- Solution filtrée et dégazée puis addition de SDS (0,1 %), de persulfate d'ammonium 0,05 % et de Temed 0,05 %.
- Polymérisation une nuit à 4°C (gel 16 x 20 x 0,15 cm).

- Le gel d'isoélectrofocalisation après équilibration est déposé à la surface du gel PAGE/SDS qui scellé par de l'agarose.
- Tampon d'électrophorèse : (Tris-HCl 25 mM pH 8,3, glycine 0,192 M, SDS 0,1 %).
- Run 100 mA - 6 h à 6°C.
- Gel fixé dans 50 % de méthanol, 10 % acide acétique puis coloré au nitrate d'argent (Méthode de Blum. H., Electrophoresis 1987, 8 p. 93-99).
- Gel scanné sur un analyseur d'image visage 2000/Kodak pour déterminer la densité optique et la surface de chaque spot, donc permettre de calculer le rapport quantitatif entre les spots.
- La masse molaire de la protéine est déterminée en réalisant un gel bidimensionnel en présence de protéines témoins Amersham.

<u>Résultat :</u>

On observe deux spots de masse moléculaire voisine de 33,5 kDa, l'un majoritaire de point isoélectrique voisin de 8,0 d'intensité 5,2 (représentant environ 93 % de la masse protéique), l'autre minoritaire de point isoélectrique voisin de 7,4 d'intensité 0,41 (représentant environ 7 % de la masse protéique).

f) Détermination de la séquence amino-terminale et de la masse du groupement amino-terminal bloquant :

α) mise en évidence du caractère bloqué de la séquence amino-terminale :

La séquence amino-terminale a été analysée à l'aide d'un séquenceur Applied Biosystem modèle 470 A, couplé à un analyseur de dérivés phénylthiohydantoïques Applied Biosystem modèle 120A. L'urate oxydase purifiée (200 pmoles contrôlées par analyse d'acides aminés) a été déposée sur le séquenceur en présence de 20 pmoles de β-lactoglobuline, protéine témoin.

Aucune séquence amino-terminale correspondant à une séquence de l'urate oxydase n'a été détectée (par contre la séquence amino-terminale de la protéine témoin a été détectée, donc le séquenceur fonctionne).

L'urate oxydase d'A. flavus a donc l'extrémité amino-terminale bloquée.

β) détermination de la séquence d'un peptide amino-terminal de 32 acides aminés et de la masse du groupement amino-terminal bloquant :

<u>Méthode</u> : <u>Digestion par le bromure de cyanogène</u>

L'urate oxydase extractive purifiée est soumise à un gel filtration sur un gel obtenu par réticulation du dextran avec de l'épichlorhydrine, le Sephadex G25 (PD10 - Pharmacia), équilibré avec une solution contenant 7 % d'acide formique, ce qui permet d'éliminer les sels et de changer le tampon. Par centrifugation sous vide, la concentration en acide formique est augmentée à 70 %. On ajoute alors du bromure de cyanogène à 0,2 M final et on laisse réagir 20 h sous argon, en absence de lumière, et à température ambiante.

- <u>Séparation par chromatographie d'échanges d'ions des peptides issus de la digestion au bromure de cyanogène de la protéine</u>

Les peptides ont été séparés sur une colonne d'échanges d'ions à base de résine hydrophyle mono S (Pharmacia).
Tampon A : Acétate d'ammonium 10 mM pH 6,2
Tampon B : Acétate d'ammonium 1 M pH 6,2
Débit : 0,6 ml/min, détection des pics par mesure de la densité optique à 278 nm
Gradients : à 0 % de B à 100 % B en 30 min - collecte de fractions de 1 ml.

Les fractions issues de l'étape d'échanges d'ions ont été analysées par gel PAGE/SDS suivant la méthode décrite par Schagger et Von Jagow (1987) Anal. Biochem 166 - p. 368-379.

- <u>Purification du peptide amino-terminal par HPLC phase inverse et analyse par spectrométrie de masse de ce dernier</u>

Le peptide issu de l'étape d'échanges d'ions et ayant une masse molaire voisine de 4 000 Da (sur gel PAGE/SDS) a été purifié sur une colonne d'HPLC phase inverse à base de silice greffée C18, la colonne Beckman Altex C18 (250 x 2,1 mm)
Débit : 0,3 ml/min, détection des pics par mesure de la densité optique à 218 nm
Tampon A : $H_2O$/0,1 % TFA (acide trifluoroacétique)
Tampon B : Acétonitrile/0,1 % TFA

Gradient de 1 à 50 % de B en 60 min.

Le peptide collecté après une première étape d'HPLC phase inverse a été repurifié sur la même colonne d'HPLC phase inverse mais avec un gradient différent.

Gradient de 1 à 50 % de B en 10 min.

Le pic collecté a été soumis à une analyse par spectrométrie de masse à bombardement par atomes rapides (FAB/MS) avec une matrice glycérol + thioglycérol.

- Digestion par la chymotrypsine du peptide amino-terminal et analyse des acides aminés des peptides chymotryptiques séparés par HPLC phase inverse

Afin d'établir la séquence du peptide purifié par HPLC phase inverse, celui-ci a été digéré par la chymotrypsine. Les peptides chymotryptiques ont été séparés par HPLC phase inverse sur colonne Beckman Altex C18 (250 x 2,1 mm). Débit 0,3 ml/min, détection des pics par mesure de la densité optique à 218 nm,

Tampon A $H_2O$/0,11 % TFA

Tampon B Acétonitrile/0,08 % TFA

Gradient de 1 % de B à 50 % B en 60 min - collecte des pics.

Les peptides chymotryptiques ont été identifiés par analyse d'acides aminés sur Analyseur Applied Biosystem (modèle 420-130A).

Résultats :

Les résultats présentés ci-après, établis postérieurement à la détermination de la séquence de l'ADNc de l'urate oxydase d'A. flavus et de la séquence d'acides aminés déduite (Cf. exemple 6), ne peuvent être compris qu'à la lumière de ceux-ci.

- Analyse par spectrométrie de masse du peptide amino-terminal.

On observe une différence d'environ 42 unités de masse atomique entre les deux masses moléculaires déterminées par spectrométrie de masse, 3684 et 3666, et les masses moléculaires théoriques déterminées à partir de la séquence suivante (séquence d'acides aminés déduite de l'ADNc de l'urate oxydase d'A. flavus avec clivage du groupe méthionine amino-terminal et coupure peptidique par le bromure de cyanogène après le premier résidu méthionine) :

```
Ser Ala Val Lys Ala Ala Arg Tyr Gly Lys Asp Asn Val Arg Val Tyr
Lys Val His Lys Asp Glu Lys Thr Gly Val Gln Thr Val Tyr Gly (1)
```

avec un résidu méthionine carboxyterminal modifié par action du bromure de cyanogène soit en homosérine, 3642, soit en homosérine lactone, 3624.

Il y a donc un groupement bloquant sur la sérine amino-terminale qui confère une masse supplémentaire d'environ 42 unités de masse atomique, correspondant probablement à une acétylation de ce dernier (masse de $CH_3CO$-masse H = 42 unités de masse atomique).

- Analyse des acides aminés des peptides chymotryptiques:

Cette analyse a permis de montrer sans ambiguïté que la séquence du peptide amino-terminal obtenu par digestion au bromure de cyanogène comprend la séquence (1), explicitée ci-dessus.

La séquence complète d'acides aminés de l'urate oxydase est indiquée ci-après :

Ser Ala Val Lys Ala Ala Arg Tyr Gly Lys Asp Asn Val Arg Val Tyr Lys
Val His Lys Asp Glu Lys Thr Gly Val Gln Thr Val Tyr Glu Met Thr Val
Cys Val Leu Leu Glu Gly Glu Ile Glu Thr Ser Tyr Thr Lys Ala Asp Asn
Ser Val Ile Val Ala Thr Asp Ser Ile Lys Asn Thr Ile Tyr Ile Thr Ala
Lys Gln Asn Pro Val Thr Pro Pro Glu Leu Phe Gly Ser Ile Leu Gly Thr
His Phe Ile Glu Lys Tyr Asn His Ile His Ala Ala His Val Asn Ile Val
Cys His Arg Trp Thr Arg Met Asp Ile Asp Gly Lys Pro His Pro His Ser
Phe Ile Arg Asp Ser Glu Glu Lys Arg Asn Val Gln Val Asp Val Val Glu
Gly Lys Gly Ile Asp Ile Lys Ser Ser Leu Ser Gly Leu Thr Val Leu Lys
Ser Thr Asn Ser Gln Phe Trp Gly Phe Leu Arg Asp Glu Tyr Thr Thr Leu
Lys Glu Thr Trp Asp Arg Ile Leu Ser Thr Asp Val Asp Ala Thr Trp Gln
Trp Lys Asn Phe Ser Gly Leu Gln Glu Val Arg Ser His Val Pro Lys Phe
Asp Ala Thr Trp Ala Thr Ala Arg Glu Val Thr Leu Lys Thr Phe Ala Glu
Asp Asn Ser Ala Ser Val Gln Ala Thr Met Tyr Lys Met Ala Glu Gln Ile
Leu Ala Arg Gln Gln Leu Ile Glu Thr Val Glu Tyr Ser Leu Pro Asn Lys
His Tyr Phe Glu Ile Asp Leu Ser Trp His Lys Gly Leu Gln Asn Thr Gly
Lys Asn Ala Glu Val Phe Ala Pro Gln Ser Asp Pro Asn Gly Leu Ile Lys
Cys Thr Val Gly Arg Ser Ser Leu Lys Ser Lys Leu

EXEMPLE 5 : Criblage des bactéries

1) Préparation des sondes marquées

Deux pools de sondes déduites de séquences en aminoacides de la protéine ont été synthétisés à l'aide d'un synthétiseur d'ADN Biosearch 4600. Le premier pool correspond à la séquence de résidus His-Tyr-Phe-Glu-Ile-Asp (partie de la séquence de T 27), soit de 5′ vers 3′ :

```
    A     T     G     G     G
T C G A T   T C   A A   T A   T G
    T     C     A     A     A
```

Ce pool est en fait constitué de $2^4 \times 3 = 48$ oligonucléotides différents représentant toutes les combinaisons possibles. Le deuxième pool correspond à la séquence en résidus aminoacides Gln-Phe-Trp-Gly-Phe-Leu (partie de la séquence de V5), soit de 5′ vers 3′ :

```
 GG  A     G  T
A  AAGCCCCA AA TG
 AA  C     A  C
     T
```

Ce pool est constitué de $2^4 \times 4 = 64$ combinaisons.

Les sondes sont marquées à la terminale désoxynucléotide transférase (TdT) (commercialisé par IBI, Inc).

La réaction s'effectue sur 100 ng d'un mélange d'oligonucléotides en solution (100 mg/ml) dans du tampon de réaction "Cobalt" (fourni 10 fois concentrée par IBI inc. : 1,4 M de cocodylate de potassium-pH 7,2, 300 mM de dithiothréitol, 1 µl d'enzyme désoxynucléotidyl-terminal-transférase (IBI inc) et 50 µCi de désoxycytidyltriphosphate dCTP marqué au P32.

La réaction se fait à 37°C pendant 10 min puis est arrêtée en ajoutant 1 µl d'EDTA 0,5 M.

On extrait au phénol et on dialyse sur colonne de polyacrylamide Biogel P 10 (Biorad : 150-1050).

2) Hybridation et détection des colonies contenant l'ADNc de l'urate oxydase :

Environ 40 000 colonies sont criblées par la technique d'hybridation in situ mise au point par Grunstein et Hogness (1975, Proc. Natl. Acad. Sci. (U.S.A.), 72 3961). Environ 6 000 bactéries sont étalées sur boîtes de Pétri de façon à obtenir des colonies isolées. Après incubation pendant 24 h à 37°C, chaque boîte est repliquée sur 2 filtres, chaque filtre étant destiné à être traité avec un des 2 pools de sondes, de façon que toutes les colonies obtenues soient testées avec les 2 pools de sondes en parallèle.

Les filtres sont mis à hybrider avec un des 2 pools de sondes dans un tampon contenant 6 x SSC, 10 x Denhardt's et 100 µg/ml d'ADN de sperme de saumon soniqué et dénaturé (SIGMA). La température d'hybridation est de 42°C et la durée de 16 h. La solution 6 x SSC s'obtient par dilution d'une solution 20 x SSC. La préparation du tampon 20 x SSC est décrite dans Maniatis, Fritsch et Sambrook (op. cité). En résumé, ce tampon contient 175,3 g/l de NaCl ; 88,2 g/l de citrate de sodium et est ajusté à pH 7 par quelques gouttes de NaOH 10N. La solution 10 x Denhardt's contient 1 g de Ficoll, 1 g de polyvinylpyrrolidone, 1 g de sérum-albumine humaine pour 500 ml de volume final.

Après lavage dans la solution 6 x SSC à 42°C (3 h avec 5 changements de bain), les filtres sont essuyés au papier Joseph et mis en autoradiographie. Les filtres sont révélés après 16 h. Il est apparu qu'une fraction d'environ 0,5 % des colonies hybridait avec les 2 pools de sondes.

5 colonies parmi celles-ci ont été reprises et purifiées. On a préparé l'ADN plasmidique de chacune de ces colonies, et l'on a analysé cet ADN par digestion avec soit BamHI, soit HindIII, soit à la fois BamHI et HindIII.

Après analyse sur gel d'agarose, il est apparu que les 5 plasmides obtenus sont linéarisés par BamHI et par HindIII. Les doubles digestions permettent de libérer un fragment correspondant à l'intégralité de l'ADNc cloné. La taille de ce fragment est d'environ 1,2 Kb dans 3 cas, d'environ 0,9 Kb dans les 2 autres cas. Pour la détermination ci-après on a sélectionné un des fragments de 0,9 Kb et un des fragments de 1,2 Kb qui ont été reclonés (voir exemple 6 ci-après).

EXEMPLE 6 : Détermination de la séquence de l'ADNc de l'urate oxydase

On a recloné un des fragments de 0,9 Kb d'une part (clone 9A) et un des fragments de 1,2 Kb (clone 9C) d'autre part, dans l'ADN de la forme replicative de phage simple-brin M13. L'ADN des clones M13 contenant d'une part le fragment de 0,9 Kb et d'autre part le fragment de 1,2 Kb a été soumis à une digestion exonucléasique de manière à générer une série de clones M13 chevauchants (procédure "Cyclone I Biosystem" de IBI). Ces derniers ont été séquencés par la méthode des didéoxyribonucléotides (Sanger et al, PNAS-U.S.A.-1977, 14, 5463-5467).

La séquence nucléotidique du clone 9C est représentée sur la figure 3 laquelle indique également par une flèche le début du clone 9A et par un symbole nucléotidique muni d'un astérisque * les nucléotides séquencés du clone 9A non identiques à ceux du clone 9C (quand on fait correspondre les deux séquences et les sites de restriction AccI et BamHI utilisés dans les constructions ultérieures (Cf. exemple 10).

On constate que :

- la séquence nucléotidique du fragment le plus long (clone 9C) recouvre celle du fragment le plus court (clone 9A), à deux différences près (v figure 3). Une des différences est silencieuse, l'autre correspond à un changement d'un résidu tryptophane en résidu glycine. Ces différences peuvent être dues, soit à des différences dans les ARN messagers isolés, (Cf. exemple 2 ci-dessus), soit à des erreurs de la transcriptase inverse utilisée lors de la constitution de la banque des ADNc (Cf. exemple 3 ci-dessus). Le séquençage de l'ADN génomique, de l'urate oxydase d'A. flavus a permis de lever cette ambiguïté : il s'agit d'un résidu tryptophane (et donc probablement d'une erreur de la transcriptase inverse).

Dans le cas du fragment le plus long, un codon ATG (en position 109 sur la figure 3) ouvre une phase ouverte correspondant à un polypeptide de 302 aminoacides, de masse moléculaire voisine de 34240 Da, dont la séquence correspond à la séquence partielle de l'urate oxydase d'A. flavus purifiée (Cf. exemple 4).

On a représenté sur la figure 4 la séquence d'ADN ouverte par le codon ATG et le polypeptide codé, ainsi que par des flèches en vis-à-vis avec le polypeptide codé les peptides séquencés (Cf. exemple 4) obtenus par hydrolyse de l'urate oxydase d'A. flavus à l'aide de la trypsine et de la protéase V8.

On constate que la séquence du polypeptide se termine par Le triplet Ser-Lys-Leu, typique des enzymes à localisation peroxisomale (Gould S.J. et al, J. Cell, Biology 108 (1989) 1657-1664).

EXEMPLE 7 : Construction d'un vecteur d'expression de l'ADNc de l'urate oxydase

On a préparé le plasmide p466, vecteur d'expression dans E. coli. Ce dernier comprend un fragment de pBR327 incluant l'origine de réplication et le gène de résistance à l'ampicilline, il comprend également un promoteur synthétique d'E. coli (R. RODRIGUEZ et M. CHAMBERLIN "Promoters-Structure and function (1982) Preager), une séquence de Shine-Dalgarno, suivie d'un polylinker présentant les sites uniques NdeI et KpnI, un terminateur de transcription (dérivé du phage fd) et le gène lac i.

Ce plasmide a été construit à partir d'un plasmide d'expression de l'hGH dans E. coli (p462) par substitution d'un fragment portant le gène de l'hGH par l'ADNc de l'urate oxydase.

La construction du plasmide p466 va maintenant être décrite plus en détail dans l'exposé ci-après dans lequel il sera fait référence aux figures 5, 6, 7, 8, 9.

La figure 5 représente une carte de restriction du plasmide p163,1. Les différents segments de restriction sont marqués de manière arbitraire selon la légende ci-dessous :

= Segment d'ADN issu du plasmide pBR322

= Localisation de l'origine de réplication (ORI)

= Segment d'ADN contenant la séquence codant pour un précurseur naturel de l'hGH

= Segment d'ADN du phage fd contenant un terminateur de transcription

= Segment d'ADN contenant un promoteur-opérateur hybride tryptophane-lactose UV5

= Segment d'ADN codant pour la β-lactamase (ApR : résistance à l'ampicilline).

La figure 6 représente la carte de restriction d'un plasmide p160 dont les fragments PvuI-XhoI-BamHI(1) et PvuI-ORI-BamHI(2) proviennent respectivement des plasmides p163,1 et pBR327 et dont le petit fragment BamHI(2)-BamHI (1) est le fragment 3 décrit ci- après.

La figure 7 représente la carte de restriction du plasmide p373,2. Les différents segments de restriction sont marqués de manière arbitraire selon la légende ci-dessous :

= Séquence PvuI-BamHI issue du plasmide pBR327

= Séquence PvuI-XhoI issue du plasmide p163,1

= Séquence XhoI-HincII issue du plasmide p163,1

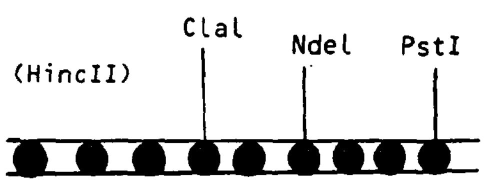


Fragment 4 décrit ci-après

= Fragment 3 décrit ci-après

= Segment d'ADN du phage fd contenant un terminateur de transcription

La figure 8 représente une carte de restriction du plasmide p462, le fragment synthétique BglII-HindIII défini ci-après étant représenté par :

La figure 9 représente une carte de restriction du plasmide p466, le fragment NdeI-KpnI comprenant le gène codant pour l'urate oxydase étant représenté par :

1) Construction du plasmide p373,2

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS EF, FRITSCH et J. SAMBROOK, Cold Spring Harbor Laboratory (1982). La synthèse des oligonucléotides est réalisée à l'aide d'un synthétiseur d'ADN Biosearch 4 600.

On a soumis le plasmide p163,1 (figure 5), décrit dans la demande de brevet EP-A-0245138 et déposée à la CNCM sous la référence I-530 le 17 février 1986, à une digestion par les enzymes PvuI et BamHI. Ce plasmide contient le gène codant pour l'hGH. Le fragment PvuI-BamHI - ci-après fragment 1 - contenant le site d'action de l'enzyme de restriction XhoI, représenté sur la figure 5, a été purifié.

De même, on a soumis le plasmide pBR327, bien connu de l'homme de l'art, (cf. SOBERON, X et al., Gene, 9 (1980) 287-305) à une digestion par les enzymes PvuI et BamHI. Le fragment PvuI-BamHI

- ci-après fragment 2 -, contenant l'origine de réplication, a été purifié.

Puis on a préparé le fragment 3, qui est un fragment synthétique BamHI(1)-BamHI(2) contenant le gène lac i et son promoteur dont la séquence est la suivante sur laquelle les deux extrémités du brin sont repérées par les nombres 1 et 2 pour préciser l'orientation du fragment dans les plasmides décrits aux figures 6 et 7.

<u>FRAGMENT 3</u>

```
   BamHI(1)

5'      GATCC GCGGAAGCAT AAAGTGTAAA GCCTGGGGTG CCTAATGAGT
   GAGCTAACTT ACATTAATTG CGTTGCGCTC ACTGCCCGCT TTCCAGTCGG
   GAAACCTGTC GTGCCAGCTG CATTAATGAA TCGGCCAACG CGCGGGGAGA
   GGCGGTTTGC GTATTGGGCG CCAGGGTGGT TTTTCTTTTC ACCAGTGAGA
   CGGGCAACAG CTGATTGCCC TTCACCGCCT GGCCCTGAGA GAGTTGCAGC
   AAGCGGTCCA CGCTGGTTTG CCCCACCACC CGAAAATCCT GTTTGATGGT
   GGTTAACGGC GGGATATAAC ATGAGCTGTC TTCGGTATCG TCGTATCCCA
   CTACCGAGAT ATCCGCACCA ACGCGCAGCC CGGACTCGGT AATGGCGCGC
   ATTGCGCCCA GCGCCATCTG ATCGTTGGCA ACCAGCATCG CAGTGGGAAC
   GATGCCCTCA TTCAGCATTT GCATGGTTTG TTGAAAACCG GACATGGCAC
   TCCAGTCGCC TTCCCGTTCC GCTATCGGCT GAATTTGATT GCGAGTGAGA
   TATTTATGCC AGCCAGCCAG ACGCAGACGC GCCGAGACAG AACTTAATGG
   GCCCGCTAAC AGCGCGATTT GCTGGTGACC CAATGCGACC AGATGCTCCA
   CGCCCAGTCG CGTACCGTCT TCATGGGAGA AAATAATACT GTTGATGGGT
   GTCTGGTCAG AGACATCAAG AAATAACGCC GGAACATTAG TGCAGGCAGC
   TTCCACAGCA ATGGCATCCT GGTCATCCAG CGGATAGTTA ATGATCAGCC
   CACTGACGCG TTGCGCGAGA AGATTGTGCA CCGCCGCTTT ACAGGCTTCG
   ACGCCGCTTC GTTCTACCAT CGACACCACC ACGCTGGCAC CCAGTTGATC
```

```
GGCGCGAGAT TTAATCGCCG CGACAATTTG CGACGGCGCG TGCAGGGCCA
GACTGGAGGT GGCAACGCCA ATCAGCAACG ACTGTTTGCC CGCCAGTTGT
TGTGCCACGC GGTTGGGAAT GTAATTCAGC TCCGCCATCG CCGCTTCCAC
TTTTTCCCGC GTTTTCGCAG AAACGTGGCT GGCCTGGTTC ACCACGCGGG
AAACGGTCTG ATAACAGACA CCGGCATACT CTGCGACATC GTATAACGTT
ACTGGTTTCA CATTCACCAC CCTGAATTGA CTCTCTTCCG GGCGCTATCA
TGCCATACCG CGAAAGGTTT TGCGCCATTC GATGGTGTCC G            3'
                                            BamHI(2)
```

Les fragments 1, 2 et 3 ont été alors ligués de manière à obtenir le plasmide p160 représenté sur la figure 6.

Ce plasmide a été soumis à une digestion partielle par les enzymes de restriction HincII et PstI. Le grand fragment HincII-PstI, contenant l'origine de réplication et représenté sur la figure 6, a été ensuite ligué au fragment 4, représenté ci-après, qui est un fragment synthétique d'ADN portant une séquence codant pour les 44 premiers acides aminés d'un précurseur naturel de l'hGH et en amont de cette séquence des signaux de régulation.

FRAGMENT 4

```
                                                    ClaI
              5'    TCGAGCTGACTGACCTGTTGCTTATATTACATCGA
                    -----------------------------------
                    AGCTCGACTGACTGGACAACGAATATAATGTAGCT
                                                    NdeI
TAGCGTATAATGTGTGGAATTGTGAGCGATAACAATTTCACACAGTTTAACTTTAAGAAGGAGATATACAT
-----------------------------------------------------------------------
ATCGATATTACACACCTTAACACTCGCCTATTGTTAAAGTGTGTCAAATTGAAATTCTTCCTCTATATGTA

ATG GCT ACC GGA TCC CGG ACT AGT CTG CTC CTG GCT TTT GGC CTG CTC TGC CTG
-----------------------------------------------------------------------
TAC CGA TGG CCT AGG GCC TGA TCA GAC GAG GAC CGA AAA CCG GAC GAC ACG GAC
 M   A   T   G   S   R   T   S   L   L   L   A   F   G   L   L   C   L
-26
```

```
                                                               XbaI
                                                                ▼
CCC TGG CTT CAA GAG GGC AGT GCC TTC CCA ACC ATT CCC TTA TCT AGA CTT TTT
-----------------------------------------------------------------------
GGG ACC GAA GTT CTC CCG TCA CGG AAG GGT TGG TAA GGG AAT AGA TCT GAA AAA
                                                                    ▲
 P   W   L   Q   E   G   S   A   F   P   T   I   P   L   S   R   L   F
                            -1   1

GAC AAC GCT ATG CTC CGC GCC CAT CGT CTG CAC CAG CTG GCC TTT GAC ACC TAC
-----------------------------------------------------------------------
CTG TTG CGA TAC GAG GCG CGG GTA GCA GAC GTG GTC GAC CGG AAA CTG TGG ATC
 D   N   A   M   L   R   A   H   R   L   H   Q   L   A   F   L   T   Y
                                                                   PstI
CAG GAG TTT GAA GAA GCC TAT ATC CCA AAG GAA CAG AAG TAT TCA TTC CTG CA
-----------------------------------------------------------------------
GTC CTC AAA CTT CTT CGG ATA TAG GGT TTC CTT GTC TTC ATA AGT AAG G
 Q   E   F   E   E   A   Y   I   P   K   E   Q   K   Y   S   F
                                                              44
```

Dans ce fragment, les acides aminés sont désignés par des lettres selon le code suivant :

| | |
|---|---|
| A = Alanine | M = Méthionine |
| C = Cystéine | N = Asparagine |
| D = Acide aspartique | P = Proline |
| E = Acide glutamique | Q = Glutamine |
| F = Phénylalanine | R = Arginine |
| G = Glycine | S = Sérine |
| H = Histidine | T = Thréonine |
| I = Isoleucine | V = Valine |
| K = Lysine | W = Tryptophane |
| L = Leucine | Y = Tyrosine |

Sont successivement soulignées dans ce fragment les séquences -35 (TTGCTT) et -10 (TATAAT) de la séquence promotrice, et la séquence de Shine et Dalgarno bien connue de l'homme de l'art.

Le plasmide p380,1 a été ainsi obtenu.

Le plasmide p380,1 a été ensuite soumis à une digestion par les enzymes de restriction ClaI et NdeI de manière à en éliminer le petit fragment ClaI-NdeI du fragment 4 ci-dessus et à lui substituer le fragment ClaI-NdeI ci-après :

```
                ClaI
5'              CGATAGCGTATAATGTGTGGAATTGTGAGCGGATAACA
                  TATCGCATATTACACACCTTAACACTCGCCTATTGT

                                                     NdeI
                ATTTCACACAGTTTTTCGCGAAGAAGGAGATATACA
                TAAAGTGTGTCAAAAAGCGCTTCTTCCTCTATATGTAT          5'
```

Le plasmide résultant est le plasmide p373,2 (figure 7).

2) Construction du plasmide p466 :

Le plasmide p373,2 a été soumis à une double digestion par les enzymes BglII et HindIII. Le grand fragment issu de cette digestion a été purifié et ligué avec un fragment d'ADN synthétique, dont la séquence donnée ci-après est destinée à reconstituer la fin du gène de l'hGH suivie en 3′ des sites de clonage KpnI et SnaBI.

```
          B
          g
          l
          I
          I
           GATCTTCAAGCAGACCTACAGCAAGTTCGACACAAACTCACACAACGAT
               ----+---------+---------+---------+---------+
               AAGTTCGTCTGGATGTCGTTCAAGCTGTGTTTGAGTGTGTTGCTA
GACGCACTACTCAAGAACTACGGGCTGCTCTACTGCTTCAGGAAGGACATGGACAAGGTC
---------+---------+---------+---------+---------+---------+
CTGCGTGATGAGTTCTTGATGCCCGACGAGATGACGAAGTCCTTCCTGTACCTGTTCCAG

             F
             s
             p
             I
GAGACATTCCTGCGCATCGTGCAGTGCCGCTCTGTGGAGGGCAGCTGTGGCTTCTAGTAA
---------+---------+---------+---------+---------+---------+
CTCTGTAAGGACGCGTAGCACGTCACGGCGAGACACCTCCCGTCGACACCGAAGATCATT
```

```
                        H
                        i
                  S     n
      K           n     d
      p           a     I
      n           B     I
      I           I     I
GGTACCCTGCCCTACGTACCA
---------+---------+-----
CCATGGGACGGGATGCATGGTTCGA
```

Ce fragment comprend les extrémités cohésives BglII et HindIII. Le nouveau plasmide ainsi formé p462 (Cf. Fig. 8) comprend ainsi un site KpnI et un site NdeI qui vont servir au clonage du fragment portant l'ADNc de l'urate oxydase dans le vecteur d'expression.

Le plasmide hybride dérivé de pTZ19R portant l'ADNc d'environ 1,2 Kb (clone 9C), de l'urate oxydase (voir exemple 3) comprend un site KpnI unique. Ce site est localisé quelques paires de bases en aval du site de clonage de l'ADNc. D'autre part l'ADNc de l'urate oxydase contient un site AccI situé à proximité de l'extrémité 5'.

On a donc isolé et purifié le fragment AccI-KpnI comprenant la plus grande partie de cet ADNc. D'autre part on a synthétisé deux oligonucléotides complémentaires dont la séquence donnée ci-après :

```
5'-TATGTCTGCGGTAAAAGCAGCGCGCTACGGCAAGGACAATGTTCGCGT
       ACAGACGCCATTTTCGTCGCGCGATGCCGTTCCTGTTACAAGCGCAGA-5'
```

est destinée à reconstituer l'extrémité 5' de l'ADNc. Ce fragment synthétique ainsi obtenu possède une extrémité NdeI et une autre AccI. Le fragment et la séquence synthétique ont été ligués avec le vecteur d'expression coupé par KpnI et par NdeI. Cette ligation à trois fragments permet d'obtenir le vecteur d'expression nommé p466, de l'urate oxydase pour E. coli (Cf. figure 9). Ce plasmide a été soumis à une série d'hydrolyses enzymatiques par des enzymes de restriction, qui a permis de vérifier la présence des sites de restriction attendus, en particulier ceux portés par le gène codant pour l'urate oxydase.

Le plasmide p466 contient donc par construction un gène codant pour l'urate oxydase de séquence ci-après :

```
ATGTCTGCGG TAAAAGCAGC GCGCTACGGC AAGGACAATG TTCGCGTCTA
CAAGGTTCAC AAGGACGAGA AGACCGGTGT CCAGACGGTG TACGAGATGA
CCGTCTGTGT GCTTCTGGAG GGTGAGATTG AGACCTCTTA CACCAAGGCC
GACAACAGCG TCATTGTCGC AACCGACTCC ATTAAGAACA CCATTTACAT
CACCGCCAAG CAGAACCCCG TTACTCCTCC CGAGCTGTTC GGCTCCATCC
TGGGCACACA CTTCATTGAG AAGTACAACC ACATCCATGC CGCTCACGTC
AACATTGTCT GCCACCGCTG GACCCGGATG GACATTGACG GCAAGCCACA
CCCTCACTCC TTCATCCGCG ACAGCGAGGA GAAGCGGAAT GTGCAGGTGG
ACGTGGTCGA GGGCAAGGGC ATCGATATCA AGTCGTCTCT GTCCGGCCTG
ACCGTGCTGA AGAGCACCAA CTCGCAGTTC TGGGGCTTCC TGCGTGACGA
GTACACCACA CTTAAGGAGA CCTGGGACCG TATCCTGAGC ACCGACGTCG
ATGCCACTTG GCAGTGGAAG AATTTCAGTG GACTCCAGGA GGTCCGCTCG
CACGTGCCTA AGTTCGATGC TACCTGGGCC ACTGCTCGCG AGGTCACTCT
GAAGACTTTT GCTGAAGATA ACAGTGCCAG CGTGCAGGCC ACTATGTACA
AGATGGCAGA GCAAATCCTG GCGCGCCAGC AGCTGATCGA GACTGTCGAG
TACTCGTTGC CTAACAAGCA CTATTTCGAA ATCGACCTGA GCTGGCACAA
GGGCCTCCAA AACACCGGCA AGAACGCCGA GGTCTTCGCT CCTCAGTCGG
ACCCCAACGG TCTGATCAAG TGTACCGTCG GCCGGTCCTC TCTGAAGTCT
AAATTG.
```

(Les nucléotides différents des nucléotides de l'ADNc isolé d'A. flavus sont soulignés dans la séquence ci-dessus. Ces différences ont été introduites sur le fragment synthétique AccI-KpnI de façon à avoir en aval de l'ATG une séquence nucléotidique plus conforme à celles habituellement rencontrées dans un gène procaryote).

EXEMPLE 8 : Expression de l'ADNc de l'urate oxydase

La souche E. coli K12 RR1 (Bethesda research lab. Inc.) a été transformée pour la résistance à l'ampicilline avec le plasmide p466 et avec un plasmide témoin négatif pBR322.
Des colonies résistantes à l'ampicilline ont été obtenues dans les 2 cas.
1 colonie de chaque type a été mise en culture dans du milieu (LB + ampicilline 100 μg/ml). Après une nuit à 37°C sous agitation, les deux cultures ont été diluées 100 fois dans du milieu (LB + ampicilline 100 μg/ml). Après 1 h de culture on y ajoute de l'IPTG (Isopropyl β-DThiogalactoside) 1 mM pendant 3 h.
Immunodétection de l'urate oxydase par Western blot :

1) Mode opératoire :

On prélève du milieu de culture obtenu après 3 h d'induction à l'IPTG une fraction aliquote correspondant à 0,2 ml à DO = 1. On centrifuge cette dernière et on élimine le surnageant. On soumet ensuite le culot à un Western blot, technique bien connue de l'homme de l'art, qui comprend les étapes suivantes :

- Solubilisation du culot par ébullition pendant 10 min dans un tampon dénommé tampon de charge constitué de Tris HCl 0,125 M pH 6,8 SDS 4 %, bleu de bromophénol 0,002 %, glycérol 20 %, β-mercaptoéthanol 10 % (selon le protocole décrit par LAEMMLI (U.K. LAEMMLI, Nature, 227 (1970), 680-685)),
- séparation électrophorétique des différentes protéines contenues dans le solubilisat selon le protocole décrit par LAEMMLI (U.K. LAEMMLI, Nature, 227 (1970), 680-685),
- transfert desdites protéines contenues dans le gel sur un filtre de nitrocellulose (selon la technique de H. TOWBIN et al. Proc. Natl. Acad. Sci. USA 76 (1979) 4350-4354),

Immunodétection, réalisée selon la technique de BURNETTE (W.W. BURNETTE Ana. Biochem. 112 (1981) 195-203), elle implique successivement :

. Le rinçage du filtre de nitrocellulose pendant 10 min avec un tampon A (Tris-HCl 10 mM, NaCl 170 mM, KCl 1 mM).
. La mise en contact du filtre de nitrocellulose pendant 30 min à 37°C avec un tampon B (tampon A additionné de sérum-albumine bovine à raison de 3 g pour 100 ml).
. La mise en contact du filtre de nitrocellulose pendant 1 h à 37°C avec un immunsérum (des anticorps polyclonaux reconnaissant l'urate oxydase d'A. flavus).
. Le rinçage du filtre de nitrocellulose avec le tampon B.
. La mise en contact du filtre de nitrocellulose pendant 1 h à 37°C avec une solution de protéine G marquée à l'iode 125 à 0,1 microcurie/ml.
. Le rinçage du filtre avec le tampon A.
. Le séchage du filtre entre deux feuilles absorbantes.
. La mise en contact d'un film radiographique.
. La révélation du film.

2) Résultats :

On constate que la souche transformée par le plasmide p466 surproduit une protéine de poids moléculaire apparent d'environ 33 KDa qui est reconnue par les anticorps dirigés contre l'urate oxydase d'A. flavus et qui est absente dans la souche témoin.

EXEMPLE 9 : Dosage de l'activité urate oxydase

On prélève du milieu de culture obtenu après 3 h d'induction à l'IPTG dans les conditions de culture décrites à l'exemple précédent une fraction aliquote correspondant à l'équivalent de 0,5 ml à DO = 1. On centrifuge cette dernière et on élimine le surnageant. On reprend les culots dans 1 ml de tampon TEA (Triéthanolamine) 0,05 M pH 8,9. La suspension cellulaire est soniquée 2 fois pendant 30 s dans la glace avec un sonicateur ultrasonic W10 (réglé à puissance 8 et intensité 4). Les extraits sont centrifugés à 10 000 g pendant 10 min, les surnageants sont utilisés pour le dosage.

On effectue les opérations ci-dessus pour quatre colonies prises au hasard d'E. coli K12 transformé par le plasmide p466 (colonies $A_1$, $B_1$, $C_1$ et $D_1$) et une colonie transformée par le plasmide pBR322).

1) Principe

On suit la transformation de l'acide urique en allantoïne par la diminution de l'absorbance à 292 nm. La réaction est la suivante :

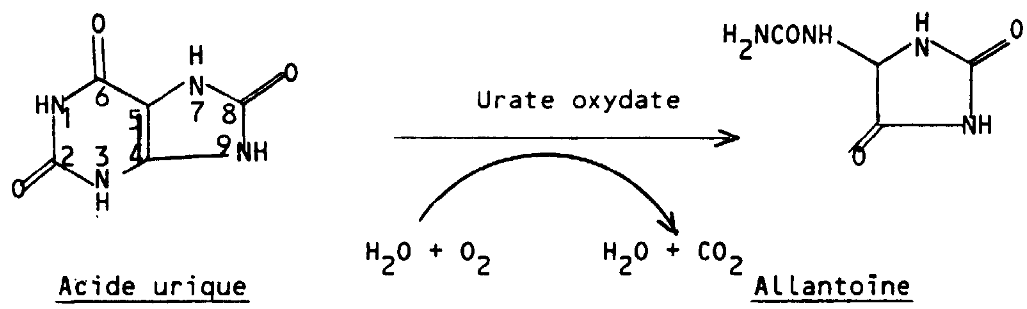


Acide urique

(absorbant à 292 nm)

Allantoïne

2) Réactifs

a) Tampon TEA 0,05 M pH 8,9/EDTA

- dissoudre 7,5 g de TEA (réactif pour analyse-Prolabo réf. 287.46.266) dans 400 ml d'eau distillée,
- dissoudre 0,372 g de Complexon III (Merck-réf. 8418) dans 50 ml d'eau distillée,
- réunir les deux solutions et compléter à 500 ml (solution 1),

- ajuster le pH de cette solution à 8,9 par HCl 0,2N,
- compléter à 1 000 ml avec de l'eau distillée (solution 2).

b) Acide urique Solution stock

- dissoudre 100 mg d'acide urique (Carbiochem réf. 6671) dans 50 ml de la solution 1,
- ajuster par HCl 0,2N à pH 8,9,
- compl`8e ter à 100 ml par de l'eau distillée.

La solution obtenue peut se conserver une semaine à 4°C.

c) Acide urique Solution Substrat

- prélever 1,5 ml de solution stock d'acide urique (carbiochem réf. 6671) et diluer à 100 ml avec du tampon TEA/ réactif pour analyse-Prolabo réf. 287.46.266).

Cette solution doit être utilisée dans la journée.

3) Mode opératoire

On introduit dans la cuve de quartz d'un spectrophotomètre réglé à 292 nm et thermostaté à 30°C les volumes suivants :

- 600 µl d'acide urique solution substrat (préchauffés à 30°C),
- 100 µl des surnageants ci-dessus auxquels ont été ajoutés 200 µl de TEA pH 8,9 (préchauffés à 30°C).

On mélange et on lit le changement de densité optique toutes les 30 s pendant 5 min. On en déduit ΔE, variation de la densité optique par minute.

4) Résultats :

L'activité A enzymatique urate oxydase exprimée en U/ml DO 1 est calculée à partir de la mesure de ΔE à l'aide de la formule :

$$A = \frac{\Delta E \text{ x } Vr \text{ x } d}{l \text{ x } V_{PE}}$$

dans laquelle les symboles Vr, d, l et $V_{PE}$ représentent respectivement le volume réactionnel (0,9 ml), le taux de dilution (2), le coefficient d'extinction de l'acide urique à 292 nm (12,5) et le volume de la prise d'essai (0,3 ml).

Les résultats obtenus sont rassemblés dans le tableau (II) ci-après :

TABLEAU (II)

| Souche d'E. coli K12 transformée par | | Activité urate oxydase (U/ml DO 1) |
|---|---|---|
| pBR322 | | < 0,001 |
| p466 | colonie $A_1$ | 0,086 |
| | colonie $B_1$ | 0,119 |
| | colonie $C_1$ | 0,135 |
| | colonie $D_1$ | 0,118 |

Il apparaît clairement sur le tableau ci-dessus que les cellules d'E. coli transformées par le plasmide p466 sont capables en présence d'IPTG de produire une activité urate oxydase.

EXEMPLE 10 : Construction de trois vecteurs d'expression de l'ADNc de l'urate oxydase dans la levure, les plasmides pEMR469, pEMR473 et pEMR515

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS, EF. FRITSCH et J. SAMBROOK dans "Molecular Cloning, a laboratory manual" (Cold Spring Harbor Laboratory, 1984). La synthèse des oligonucléotides est réalisée à l'aide d'un synthétiseur d'ADN Biosearch 4 600.

La description ci-après sera mieux comprise à la lecture des figures 10, 11 et 12 qui représentent respectivement des cartes de restriction du plasmide pEMR414, et des plasmides pEMR469 ainsi que pEMR473. Les symboles utilisés sur ces figures seront précisés dans l'exposé ci-après. Dans le cas où un site a été rendu franc par la polymérase de Kleenow, il lui est affecté l'indice "°"; lorsque les sites ont été supprimés par ligation, ils sont indiqués entre parenthèses.

1) Construction du plasmide pEMR469 :

Ce plasmide a été construit à partir du vecteur navette E. coli-levure pEMR414, construit par ligations successives des éléments ci-après :

- le fragment PstI-HindIII° - symbolisé par ++++ sur la figure 10 - du plasmide pJDB207 (BEGGS, 1978 : Gene cloning in yeast-p. 175-203 dans : Genetic Engineering vol 2 - WILLIAMSON - Academic Press - London UK) comprenant la partie amont du gène de résistance à l'ampicilline $Amp^R$ de pBR322 (Sutcliffe 1979 Cold Spring Symp. Quart. Biol. 43, 779) et un fragment de 2 µ endogène forme B portant le gène LEU2 de S. cerevisiae partiellement délété de son promoteur (appelé LEU2d), le locus STB (REP3) et l'origine de réplication du 2 µ (HARTLEY et DONELSON, 1980, Nature, 286, 860-865). L'extrémité HindIII de ce fragment a été rendue franche par action de la polymérase de Kleenow. Elle est notée HindIII° sur la figure 10.
- le fragment HindIII-SmaI - représenté par /////  sur la figure 10 - du chromosome V de levure contenant le gène URA3 avec son promoteur (ROSE et al., 1984, Gene, 29, p. 113-124). Ce fragment HindIII-SmaI provient du plasmide pFL1 (CHEVALLIER et al., 1980, Gene 11, 11-19)). L'extrémité HindIII de ce plasmide a été rendue franche par l'action de la polymérase de Kleenow.
- un fragment SamI-BamhI - symbolisé par ====== sur la figure 10 - contenant une version synthétique du promoteur du gène ADH2 ne différant de la version naturelle décrite par RUSSEL et SMITH (RUSSEL et al., 1983). J. Biol. Chem. 258, 2674-2682) que par quelques paires de bases destinées à introduire des sites de restriction. (La séquence naturelle pourrait être utilisée avec des résultats peu différents). La séquence de ce fragment est donnée ci-après :

```
     S    M
     m    l
     a    u
     I    I
     ▼GGGACGCGTCTCCTCTGCCGGAACACCGGGCATCTCCAACTTATAAGTTGGAG
       ------+---------+---------+---------+---------+------
       CCCTGCGCAGAGGAGACGGCCTTGTGGCCCGTAGAGGTTGAATATTCAACCTC

AAATAAGAGAATTTCAGATTGAGAGAATGAAAAAAAAAAAAAAAAAAAAGGCAGAGGAGA
---+---------+---------+---------+---------+---------+------
TTTATTCTCTTAAAGTCTAACTCTCTTACTTTTTTTTTTTTTTTTTTTTCCGTCTCCTCT
                                        S
                                        P
                                        h
                                        I
GCATAGAAATGGGGTTCACTTTTTGGTAAAGCTATAGCATGCCTATCACATATAAATAGA
---+---------+---------+---------+---------+---------+------
CGTATCTTTACCCCAAGTGAAAAACCATTTCGATATCGTACGGATAGTGTATATTTATCT

GTGCCAGTAGCGACTTTTTTCACACTCGAGATACTCTTACTACTGCTCTCTTGTTGTTTT
---+---------+---------+---------+---------+---------+------
CACGGTCATCGCTGAAAAAAGTGTGAGCTCTATGAGAATGATGACGAGAGAACAACAAAA

TATCACTTCTTGTTTCTTCTTGGTAAATAGAATATCAAGCTACAAAAAGCATACAATCAA
---+---------+---------+---------+---------+---------+------
ATAGTGAAGAACAAAGAAGAACCATTTATCTTATAGTTCGATGTTTTTCGTATGTTAGTT

                                                  B
                     C                            a
                     I                            m
                     a                            H
                     I                            I
                                                  ▼
CTATCAACTATTAACTATATCGATACCATATGGATCCGTCGACTCTAGAGGATCGTC
---+---------+---------+---------+---------+---------+---
GATAGTTGATAATTGATATAGCTATGGTATACCTAGGCAGCTGAGATCTCCTAGCAG
```

```
           B
           a
           m
           H
GACTCTAGAG▼
-----------+
CTGAGATCTCCTAG
```

- le fragment BglII-HindIII - symbolisé par ▬ sur la figure 10 - portant l'extrémité 3' du gène PGK de levure. Ce fragment provient de la digestion complète à l'aide de BglII du fragment HindIII de l'ADN chromosomique de levure, portant le gène PGK décrit par HITZEMAN et al. (1982 Nucleic Acids Res., 10, 7791-7808) lequel ne présente qu'un site BglII. Cette digestion permet d'obtenir deux fragments HindIII-BglII, dont le plus petit d'environ 0,4 Kb, qui porte l'extrémité 3' du gène PGK de levure est retenu. La séquence de ce dernier fragment est décrite par HITZEMANN et al. (référence citée ci-dessus). Le site BglII est cloné dans le site BamHI du fragment précédent (les sites BamHI et BglII disparaissent donc) et le site HindIII, rendu franc par action de la polymérase de Kleenow, est cloné dans le site PvuII du fragment PvuII-PstI de pBR322 décrit ci-après.
- le fragment PvuII-PstI - symbolisé par xxx sur la figure 10 - de pBR322 contenant l'origine de réplication et la partie aval du gène de résistance à l'ampicilline $Amp^R$.

Le plasmide pEMR414 ainsi constitué comporte donc les éléments suivants :

- une origine de réplication et un gène de résistance à l'ampicilline $Amp^R$ permettant la réplication et la sélection du plasmide dans les cellules E. coli. Ces éléments permettent la transformation dans les cellules de E. coli.
- une origine de réplication pour la levure (ARS), le locus STB et le gène LEU2 de S. cerevisiae sans promoteur et le gène URA3 avec son promoteur de S. cerevisiae. Ces éléments permettent la réplication et la sélection du plasmide dans les cellules de S. cerevisiae ainsi qu'une efficacité de partition suffisante dans les cellules contenant le plasmide 2µ endogène.

Le plasmide pEMR414 a été soumis à une digestion complète par les enzymes de restriction NheI et ClaI. Le petit fragment NheI-ClaI contenant le gène URA3, appelé ci-après fragment A, a été purifié.

Le plasmide pEMR414 a été soumis à une digestion complète par les enzymes NheI et BamHI. Le grand fragment NheI-BamHI contenant notamment le gène LEU2d et l'origine de réplication du plasmide pBR322, appelé ci-après fragment B, a été purifié.

On a préparé d'autre part le fragment synthétique ClaI-AccI contenant le début d'un gène codant pour la protéine déduite de la séquence de l'ADNc de l'urate oxydase (clone 9C). Ce fragment comporte des modifications par rapport au clone 9C, introduites dans le but de mettre en place des codons usuels dans la levure (Cf. SHARP et al., 1986, Nucl. Ac. Res. Vol. 14, 13, pp. 5125-5143) sans changement des acides aminés codés. La séquence de ce fragment, dénommé ci-après fragment C, est la suivante (les nucléotides soulignés sont ceux modifiés par rapport au clone 9C).

```
C                                                          A
l                                                          c
a                                                          c
I                                                          I
▼
CGATATACACAATGTCTGCTGTTAAGGCTGCTAGATACGGTAAGGACAACGTTAGAGT
---+---------+---------+---------+---------+---------+----
  TATATGTGTTACAGACGACAATTCCGACGATCTATGCCATTCCTGTTGCAATCTCAGA
```

On a soumis le plasmide du clone 9C (Cf. figure 3) à une digestion par les enzymes AccI et BamHI. Le fragment AccI-BamHI qui contient la fin de l'ADNc de l'urate oxydase, ci-après appelé fragment D, a été purifié. Ce fragment a la séquence suivante :

```
                                         AccI
                                              CTACAAGGTTCACAAGGACGAGAAG
                                              - --+---------+---------+
                                               TGTTCCAAGTGTTCCTGCTCTTC

ACCGGTGTCCAGACGGTGTACGAGATGACC GTCTGTGTGCTTCTGGAGGGTGAGATTGAG
---------+---------+---------+ ---------+---------+---------+
TGGCCACAGGTCTGCCACATGCTCTACTGG CAGACACACGAAGACCTCCCACTCTAACTC

ACCTCTTACACCAAGGCCGACAACAGCGTC ATTGTCGCAACCGACTCCATTAAGAACACC
---------+---------+---------+ ---------+---------+---------+
TGGAGAATGTGGTTCCGGCTGTTGTCGCAG TAACAGCGTTGGCTGAGGTAATTCTTGTGG

ATTTACATCACCGCCAAGCAGAACCCCGTT ACTCCTCCCGAGCTGTTCGGCTCCATCCTG
---------+---------+---------+ ---------+---------+---------+
TAAATGTAGTGGCGGTTCGTCTTGGGGCAA TGAGGAGGGCTCGACAAGCCGAGGTAGGAC

GGCACACACTTCATTGAGAAGTACAACCAC ATCCATGCCGCTCACGTCAACATTGTCTGC
---------+---------+---------+ ---------+---------+---------+
CCGTGTGTGAAGTAACTCTTCATGTTGGTG TAGGTACGGCGAGTGCAGTTGTAACAGACG

CACCGCTGGACCCGGATGGACATTGACGGC AAGCCACACCCTCACTCCTTCATCCGCGAC
---------+---------+---------+ ---------+---------+---------+
GTGGCGACCTGGGCCTACCTGTAACTGCCG TTCGGTGTGGGAGTGAGGAAGTAGGCGCTG

AGCGAGGAGAAGCGGAATGTGCAGGTGGAC GTGGTCGAGGGCAAGGGCATCGATATCAAG
---------+---------+---------+ ---------+---------+---------+
TCGCTCCTCTTCGCCTTACACGTCCACCTG CACCAGCTCCCGTTCCCGTAGCTATAGTTC

TCGTCTCTGTCCGGCCTGACCGTGCTGAAG AGCACCAACTCGCAGTTCTGGGGCTTCCTG
---------+---------+---------+ ---------+---------+---------+
AGCAGAGACAGGCCGGACTGGCACGACTTC TCGTGGTTGAGCGTCAAGACCCCGAAGGAC

CGTGACGAGTACACCACACTTAAGGAGACC TGGGACCGTATCCTGAGCACCGACGTCGAT
---------+---------+---------+ ---------+---------+---------+
GCACTGCTCATGTGGTGTGAATTCCTCTGG ACCCTGGCATAGGACTCGTGGCTGCAGCTA

GCCACTTGGCAGTGGAAGAATTTCAGTGGA CTCCAGGAGGTCCGCTCGCACGTGCCTAAG
---------+---------+---------+ ---------+---------+---------+
CGGTGAACCGTCACCTTCTTAAAGTCACCT GAGGTCCTCCAGGCGAGCGTGCACGGATTC

TTCGATGCTACCTGGGCCACTGCTCGCGAG GTCACTCTGAAGACTTTTGCTGAAGATAAC
---------+---------+---------+ ---------+---------+---------+
AAGCTACGATGGACCCGGTGACGAGCGCTC CAGTGAGACTTCTGAAAACGACTTCTATTG

AGTGCCAGCGTGCAGGCCACTATGTACAAG ATGGCAGAGCAAATCCTGGCGCGCCAGCAG
---------+---------+---------+ ---------+---------+---------+
TCACGGTCGCACGTCCGGTGATACATGTTC TACCGTCTCGTTTAGGACCGCGCGGTCGTC

CTGATCGAGACTGTCGAGTACTCGTTGCCT AACAAGCACTATTTCGAAATCGACCTGAGC
---------+---------+---------+ ---------+---------+---------+
GACTAGCTCTGACAGCTCATGAGCAACGGA TTGTTCGTGATAAAGCTTTAGCTGGACTCG

TGGCACAAGGGCCTCCAAAACACCGGCAAG AACGCCGAGGTCTTCGCTCCTCAGTCGGAC
---------+---------+---------+ ---------+---------+---------+
ACCGTGTTCCCGGAGGTTTTGTGGCCGTTC TTGCGGCTCCAGAAGCGAGGAGTCAGCCTG

CCCAACGGTCTGATCAAGTGTACCGTCGGC CGGTCCTCTCTGAAGTCTAAATTGTAAACC
---------+---------+---------+ ---------+---------+---------+
GGGTTGCCAGACTAGTTCACATGGCAGCCG GCCAGGAGAGACTTCAGATTTAACATTTGG

AACATGATTCTCACGTTCCGGAGTTTCCAA GGCAAACTGTATATAGTCTGGGATAGGGTA
---------+---------+---------+ ---------+---------+---------+
TTGTACTAAGAGTGCAAGGCCTCAAAGGTT CCGTTTGACATATATCAGACCCTATCCCAT

TAGCATTCATTCACTTGTTTTTTACTTCCA AAAAAAAAAAAAAAAAAAAAAAAAAAAAAA
---------+---------+---------+ ---------+---------+---------+
ATCGTAAGTAAGTGAACAAAAAATGAAGGT TTTTTTTTTTTTTTTTTTTTTTTTTTTTTT

AAAAAAAAAAAAAAAAAAAAAGGGCCCG         BamHI
---------+---------+--------
TTTTTTTTTTTTTTTTTTTTTCCCGGGCCT AG
```

Les fragments A, B, C et D ont été ligués de manière à obtenir le plasmide pEMR469 représenté sur la figure 11, dans laquelle les symboles ont la même signification que sur la figure 10, les nouveaux fragments ClaI-AccI et AccI-BamHI étant symbolisés par

2) Construction du plasmide pEMR473 :

Le plasmide pEMR469 a été soumis à une digestion complète par les enzymes MluI et SphI. Le grand fragment MluI-SphI contenant le gène de l'urate oxydase a ensuite été ligué au fragment synthétique, de séquence donnée ci-après, correspondant à une partie (200 pb) de la séquence en amont de l'élément TATA du promoteur GAL 7 de S. cerevisiae, laquelle comprend les séquences d'activation amont (upstream activation sequences : UAS).

```
  M
  l
  u
  I
  CGCGTCTATACTTCGGAGCACTGTTGAGCGAAGGCTCATTAGATATATTTTCTGTCAT
  --+---------+---------+---------+---------+---------+------
      AGATATGAAGCCTCGTGACAACTCGCTTCCGAGTAATCTATATAAAAGACAGTA

TTTCCTTAACCCAAAAATAAGGGAGAGGGTCCAAAAAGCGCTCGGACAACTGTTGACCGT
----+---------+---------+---------+---------+---------+------
AAAGGAATTGGGTTTTTATTCCCTCTCCCAGGTTTTTCGCGAGCCTGTTGACAACTGGCA

GATCCGAAGGACTGGCTATACAGTGTTCACAAAATAGCCAAGCTGAAAATAATGTGTAGC
----+---------+---------+---------+---------+---------+------
CTAGGCTTCCTGACCGATATGTCACAAGTGTTTTATCGGTTCGACTTTTATTACACATCG

                              S
                              p
                              h
                              I
CTTTAGCTATGTTCAGTTAGTTTGGCATG
----+---------+---------+-----
GAAATCGATACAAGTCAATCAAACC
```

Le plasmide pEMR473 ainsi obtenu est représenté sur la figure 12, dans laquelle les symboles ont la même signification que dans la figure 11, le nouveau fragment MluI-SphI introduit étant symbolisé par

3) Construction du plasmide pEMR515 :

Le plasmide pEMR473 a été soumis à une digestion partielle par l'enzyme XbaI et à une digestion totale par l'enzyme MluI. Le grand fragment XbaI-MluI a été purifié. Ce fragment contient notamment les séquences de l'origine de réplication et le locus STB du 2µ, le gène LEU2d, le gène de résistance à l'ampicilline $Amp^R$, l'origine de réplication du pBR322 et la cassette d'expression de l'urate oxydase. Il ne contient en revanche ni le gène URA3, ni la partie du 2µ comprise entre les sites XbaI et NheI.

Le grand fragment XbaI-MluI a été recircularisé via l'adaptateur de séquence suivant comportant des extrémités cohésives XbaI modifié et MluI :

```
XbaI modifié
 CTAGGCTAGCGGGCCCGCATGCA
         CGATCGCCCGGGCGTACGTGCGC
                                 MluI
```

Le plasmide pEMR515 ainsi obtenu ne possède qu'un seul des trois éléments du site FRT cible de la recombinase codée par le gène FLP du 2µ.

Les plasmides pEMR 469, pEMR473 et pEMR515 possèdent le gène codant pour l'urate oxydate de séquence

ci-après :

```
ATGTCTGCTG TTAAGGCTGC TAGATACGGT AAGGACAACG TTAGAGTCTA
CAAGGTTCAC AAGGACGAGA AGACCGGTGT CCAGACGGTG TACGAGATGA
CCGTCTGTGT GCTTCTGGAG GGTGAGATTG AGACCTCTTA CACCAAGGCC
GACAACAGCG TCATTGTCGC AACCGACTCC ATTAAGAACA CCATTTACAT
CACCGCCAAG CAGAACCCCG TTACTCCTCC CGAGCTGTTC GGCTCCATCC
TGGGCACACA CTTCATTGAG AAGTACAACC ACATCCATGC CGCTCACGTC
AACATTGTCT GCCACCGCTG GACCCGGATG GACATTGACG GCAAGCCACA
CCCTCACTCC TTCATCCGCG ACAGCGAGGA GAAGCGGAAT GTGCAGGTGG
ACGTGGTCGA GGGCAAGGGC ATCGATATCA AGTCGTCTCT GTCCGGCCTG
ACCGTGCTGA AGAGCACCAA CTCGCAGTTC TGGGGCTTCC TGCGTGACGA
GTACACCACA CTTAAGGAGA CCTGGGACCG TATCCTGAGC ACCGACGTCG
```

```
ATGCCACTTG GCAGTGGAAG AATTTCAGTG GACTCCAGGA GGTCCGCTCG
CACGTGCCTA AGTTCGATGC TACCTGGGCC ACTGCTCGCG AGGTCACTCT
GAAGACTTTT GCTGAAGATA ACAGTGCCAG CGTGCAGGCC ACTATGTACA
AGATGGCAGA GCAAATCCTG GCGCGCCAGC AGCTGATCGA GACTGTCGAG
TACTCGTTGC CTAACAAGCA CTATTTCGAA ATCGACCTGA GCTGGCACAA
GGGCCTCCAA AACACCGGCA AGAACGCCGA GGTCTTCGCT CCTCAGTCGG
ACCCCAACGG TCTGATCAAG TGTACCGTCG GCCGGTCCTC TCTGAAGTCT
AAATTG.
```

EXEMPLE 11 : Transformation de la souche de levure EMY761, par les plasmides pEMR469, pEMR473 et pEMR515 - Transformation des souches de levures EMY500 et GRF18 par le plasmide pEMR515 - Transformation avec sélection soit pour la prototrophie de l'uracile, soit pour la prototrophie de leucine

On a utilisé comme souches réceptrices trois souches de Saccharomyces cerevisiae non isogéniques :

- la souche EMY761 (Matα, leu2, ura3, his3, gal)
- la souche EMY500 (Mata, leu2, ura3, pep4)
- la souche GRF18 (Mata, leu2, his3);

La souche GRF18 est bien connue de l'homme de l'art (Gerry FINK, MIT, USA). Les souches EMY761 et EMY500 sont apparentées à la souche GRF18. Elles ont été obtenues par croisements successifs de la souche GRF18 avec une souche ura3 dérivée de la souche FL100 (déposée à l'ATCC sous le n° 28 383) et avec la souche 20B12 (Mata, tspl, pep4) décrite par E.W. JONES (E.W. JONES et al. (1977) Genetics, 85, 23).

On peut obtenir la souche GRF18 par curage du plasmide pEMR515 de la souche GRF18 pEMR515 ($leu^+$) déposée à la CNCM sous la référence n° I-920, le 28 décembre 1989 et la souche EMY500 par curage du plasmide pEMR515 de la souche EMY500 pEMR515 ($leu^+$) déposée à la CNCM sous la référence n° I-919, le 28 décembre 1989.

Ces souches contiennent des mutations (Leu2 et ura3), susceptibles d'être complémentées par le marqueur de sélection défectif LEU2d et le marqueur de sélection URA3, présents dans chacun des plasmides pEMR469 et pEMR473.

1) Transformation avec sélection pour la prototrophie de l'uracile :

Une colonie de la souche EMY761 a servi à ensemencer 100 ml d'un milieu appelé milieu YPG liquide (Cf. tableau III ci-après). Lors de l'obtention d'une densité cellulaire de $10^7$ cellules par ml, les cellules ont été traitées à l'acétate de lithium 0,2 M pour la transformation selon une technique bien connue de l'homme de l'art, décrite par ITO et al. (ITO et al., 1983, J. Bacteriology 153, 163-168).

Les cellules EMY761 ont été transformées en parallèle avec environ 1 µg de chacun des plasmides pEMR469 et pEMR473. Les cellules transformées sont sélectionnées pour le caractère d'auxotrophie d'uracile ($ura^+$) sur un milieu appelé milieu solide sans uracile, (Cf. tableau III ci-après). On a ainsi retenu un transformé EMY761 pEMR469 ($ura^+$) et un transformé EMY761 pEMR473 ($ura^+$).

2) Transformation avec sélection pour la prototrophie de leucine :

La technique de transformation utilisée est une variante de celle décrite par Beggs et al. (Beggs et al. (1978), Nature 275, 104 - 109). Elle consiste à soumettre les levures à un traitement de protoplastisation en présence d'un stabilisant osmotique, le sorbitol en concentration 1 M.

Le protocole précis de transformation est précisé ci-après :

a) 200 ml du milieu YPG liquide (Cf. tableau III) sont inoculés avec environ 5 x $10^6$ cellules d'une culture en phase stationnaire et la culture ainsi inoculée est placée une nuit sous agitation à 30°C.
b) Lorsque la culture atteint environ $10^7$ cellules par ml, les cellules sont centrifugées à 4 000 tr/min pendant 5 min et le culot est lavé avec du sorbitol 1 M.
c) Les cellules sont suspendues dans 5 ml de solution de sorbitol 1 M contenant 25 mM EDTA et 50 mM de dithiothréitol et incubées pendant 10 min à 30°C.
d) Les cellules sont lavées une fois avec 10 ml de sorbitol 1 M et suspendues dans 20 ml de sorbitol. De la zymolyase-100T (préparation obtenue par purification partielle sur colonne d'affinité du surnageant de culture d'Arthobacter luteus et contenant de la β-1,3-glucane-laminaripentahydrolase, commercialisée par SEYKAGAKU KOGYO Co. Ltd) est ajoutée à une concentration finale de 20 µg/ml et on incube la suspension à température ambiante pendant environ 15 min.
e) Les cellules sont resuspendues dans 20 ml d'un milieu contenant du sorbitol appelé milieu YPG sorbitol (Cf. tableau III ci-après) et incubées pendant 20 min à 30°C sous agitation douce.
f) On centrifuge pendant 3 min à 2 500 tr/min.
g) On resuspend dans 9 ml de tampon de transformation (sorbitol 1 M, tris-HCl de pH 7,5 10 mM et $CaCl_2$ 10 mM).
h) On ajoute 0,1 ml de cellules et 5 µl de solution d'ADN (environ 5 µg) et on laisse la suspension obtenue pendant 10 à 15 min à température ambiante.
i) On ajoute 1 ml de la solution : polyéthylène glycol PEG 4000 20 %, tris-HCl de pH 7,5 10 mM et $CaCl_2$ 10 mM.
j) On verse 0,1 ml de la suspension obtenue en i) dans un tube contenant du milieu solide de régénération sans leucine (Cf. tableau III ci-après) préalablement fondu et maintenu liquide à environ 45°C. On verse la suspension sur une boîte de Pétri contenant une couche solidifiée de 15 ml de milieu de régénération solide sans leucine.
k) On répète l'étape j) avec le reste de la suspension cellulaire obtenue en i).

Les transformés commencent à apparaître au bout de trois jours.

On a ainsi retenu les transformés EMY761 pEMR469 ($leu^+$), EMY761 pEMR473 ($leu^+$), EMY761 pEMR515 ($leu^+$), GRF18 pEMR515 ($leu^+$) et EMY500 pEMR515 ($leu^+$).

TABLEAU III

Principaux milieux utilisés dans les exemples 11, 12, 13 et 14

---

- milieu solide sans uracile

6,7 g de base azotée de levure sans acides aminés
(Yeast nitrogen base without Amino Acids de DIFCO)
5,0 g d'hydrolysat de caséine (Casamino acids de DIFCO)
10 g de glucose
20 g d'agar
mélanger tous les ingrédients dans de l'eau distillée, compléter le volume final à 1 l avec de l'eau distillée. Autoclaver 15 min à 120°C.

---

- milieu liquide sans uracile

Utiliser la formule du milieu solide sans uracile en omettant l'agar - Autoclaver 15 min à 120°C.

---

- milieu solide sans leucine

6,7 g de base azotée de levure sans acides aminés
(Yeast nitrogen base without Amino Acids de DIFCO)
20 mg d'adénine
20 mg d'uracile
20 mg de l-tryptophane
20 mg de l-histidine
20 mg de l-arginine
20 mg de l-méthionine
30 mg de l-tyrosine
30 mg de l-isoleucine
30 mg de l-lysine
50 mg de l-phénylalanine
100 mg de l-acide glutamique
150 mg de l-valine
400 mg de l-leucine

20 g de glucose

20 g d'agar

mélanger tous les ingrédients dans l'eau distillée. Compléter le volume final à 1 l avec de l'eau distillée - Autoclaver 15 min à 120°C. Après autoclavage, ajouter 200 mg de l-thréonine et 100 mg d'acide l-aspartique.

---

- <u>milieu solide de régénération sans leucine</u>

utiliser la formule du milieu solide sans leucine en mélangeant 30 g d'agar au lieu de 20 et en ajoutant au mélange 182 g de sorbitol.

---

- <u>milieu liquide sans leucine</u>

utiliser la formule du milieu solide sans leucine en omettant l'agar - Autoclaver 15 min à 120°C. Après autoclavage, ajouter 200 mg de l-thréonine et 100 mg d'acide l-aspartique.

---

- <u>milieu YP liquide</u>

10 g d'extrait de levure (Bacto-yeast extract de DIFCO)

20 g de peptone (Bacto-peptone de DIFCO)

mélanger les ingrédients dans de l'eau distillée. Compléter le volume final à 1 l avec de l'eau distillée - Autoclaver 15 min à 120°C.

---

- <u>milieu YPG liquide</u>

utiliser la formule du milieu YP liquide auquel on ajoute, après autoclavage, du glucose à une concentration de 20 g/l.

---

- milieu YPG sorbitol

  utiliser la formule du milieu YPG liquide auquel on ajoute, après autoclavage, du sorbitol à une concentration de 1 M.

---

- milieu YP éthanol-glycérol

  utiliser la formule du milieu YP liquide. Après autoclavage, ajouter 10 ml d'éthanol 100 % (1 % final) et 30 g de glycérol.

---

- milieu YP éthanol-glycérol-galactose

  utiliser la formule du milieu YP liquide. Après autoclavage, ajouter 10 ml d'éthanol 100 %, 30 g de glycérol et 30 g de galactose.

---

EXEMPLE 12 : Expression en erlenmeyer de l'ADNc de l'urate oxydase par les souches EMY761 pEMR469 ($ura^+$), EMY761 pEMR473 ($ura^+$), EMY761 pEMR469 ($leu^+$) et EMY761 pEMR473 ($leu^+$) - Immunodétection par Western Blot, dosage de l'activité urate oxydase et des protéines solubles

1) Expression de l'ADNc de l'urate oxydase :

a) Souches sélectionnées sur milieu sans uracile

Une colonie de chacune des souches EMY761 pEMR469 ($ura^+$) et EMY761 pEMR473 ($ura^+$) a été mise en culture dans 20 ml de milieu liquide sans uracile (Cf. tableau III, exemple 11). Après une nuit à 30°C sous agitation, les deux cultures ont été centrifugées pendant 10 min à 7 000 tr/min. Les culots ont été repris dans 10 ml d'eau distillée stérile et de nouveaux centrifugés pendant 10 min à 7 000 tr/min. L'expression de l'urate oxydase a été induite en reprenant les cellules dans 20 ml de milieu YP éthanol-glycérol (Cf. tableau III, exemple 11) pour la souche EMY761 pEMR469 ($ura^+$) et 20 ml de milieu YP éthanol-glycérol-galactose (Cf. tableau III, exemple 11) pour la souche EMY761 pEMR473 ($ura^+$). Les cultures ont été replacées à 30°C sous agitation pendant 22 h.

b) Souches sélectionnées sur milieu sans leucine

Dans un premier temps, une colonie de chacune des souches EMY761 pEMR469 ($leu^+$) et EMY761 pEMR473 ($leu^+$) a été mise en culture dans 20 ml de milieu liquide sans leucine (Cf. tableau III, exemple 11). Ceci a permis d'obtenir et de maintenir un nombre de copies de plasmides élevé en pratiquant la sélection pour la complémentation de la mutation Leu2 par le gène LEU2d porté par les plasmides pEMR469 et pEMR473.

Après une nuit à 30°C sous agitation, les deux cultures ont été centrifugées pendant 10 min à 7 000 tr/min. Les culots ont été repris dans 10 ml d'eau distillée stérile et de nouveau centrifugés pendant 10 min à 7 000 tr/min. L'expression de l'urate oxydase a été induite en reprenant les cellules dans 20 ml de milieu YP éthanol-glycérol pour la souche EMY761 pEMR469 ($leu^+$) et 20 ml de milieu YP éthanol-glycérol-galactose (Cf. tableau III, exemple 11) pour la souche EMY761 pEMR473 ($leu^+$). Les cultures ont été replacées à 30°C sous agitation pendant 22 h.

c) Souche témoin

La souche EMY761 non transformée, c'est-à-dire sans plasmide, a été cultivée comme ci-dessus. Elle a subi d'une part l'induction dans 10 ml de milieu liquide YP éthanol-glycérol et, d'autre part, l'induction dans 10 ml de milieu YP éthanol-glycérol-galactose.

2) Préparation des échantillons :

a) Les cellules cultivées en 1a), 1b) et 1c) ont été centrifugées et le surnageant a été éliminé. Les culots ont été repris dans 10 ml d'eau distillée et centrifugés pendant 10 min à 7 000 tr/min. Les culots ainsi lavés ont été repris dans environ 1 ml de tampon de triéthanolamine TEA de pH 8,9. Environ 300 µl de cellules ainsi reprises ont été lisées en présence de billes de verre (de 400 à 500 µm de diamètre) représentant environ la moitié du volume final. Ce mélange a été vortexé vigoureusement pendant 1 min, 4 fois, les échantillons étant placés pendant 30 s dans la glace entre chaque broyage. Le liquide a été retiré des tubes avec une pipette pasteur et transféré dans un microtube. Les billes de verre ont été lavées 1 fois avec environ 200 µl de tampon TEA de pH 8,9. Les billes ont été vortexées pendant 1 min, 1 fois, et le liquide a été retiré à la pipette pasteur pour être ajouté au lysat précédent. Le lysat a été ensuite centrifugé dans un microtube pendant 5 min à 7 000 tr/min. Le surnageant a été précautionneusement retiré et conservé à -20°C pour le Western Blot, le dosage de l'activité urate oxydase et le dosage des protéines. Le culot des cellules lysées a été conservé séparément à -20°C pour le Western Blot (Cf. 3) ci-dessous).

D'autre part, des prélèvements des cultures réalisées en 1a) et 1b) ont été réalisés comme suit avant l'induction : 2 ml de culture ont été centrifugés pendant 10 min à 7 000 tr/min. Les culots ont été repris dans 500 µl d'eau distillée et de nouveau centrifugés pendant 5 min à 7 000 tr/min. Les culots ont été repris dans environ 200 µl de tampon TEA de pH 8,9 et lysés comme ci-dessus en présence de billes de verre. Les surnageants et les culots des cellules lysées ont été conservés à -20°C séparément.

3) Immunodétection de l'urate oxydase par Western Blot :

a) Mode opératoire

Les culots et les surnageants des différents échantillons ont été soumis à un Western Blot, technique bien connue de l'homme de l'art, qui comprend les étapes suivantes :

- solubilisation du culot par ébullition pendant 10 min dans un tampon dénommé tampon de charge constitué de tris-HCl 0,125 M pH 6,8 SDS 4 %, bleu de bromophénol 0,002 %, glycérol 20 %, β-mercaptoéthanol 10 % (selon le protocole décrit par LAEMMLI (U.K. LAEMMLI, Nature, 227 (1970), 680-685)), (étape mise en oeuvre uniquement pour les culots),
- séparation électrophorétique des différentes protéines contenues dans le solubilisat selon le protocole décrit par LAEMMLI (U.K. LAEMMLI, Nature, 227 (1970), 680-685),
- transfert desdites protéines contenues dans le gel sur un filtre de nitrocellulose (selon la technique de H. TOWBIN et al. Proc. Natl. Acad. Sci. USA 76 (1979) 4350-4354),

L'immunodétection, réalisée selon la technique de BURNETTE (W.W. BURNETTE Ana. Biochem. 112 (1981) 195-203), implique successivement :

. Le rinçage du filtre de nitrocellulose pendant 10 min avec un tampon A (tris-HCl 10 mM, NaCl 170 mM, KI 1 mM).
. La mise en contact du filtre de nitrocellulose pendant 30 min à 37°C avec un tampon B (tampon A additionné de sérum-albumine bovine à raison de 3 g pour 100 ml).
. La mise en contact du filtre de nitrocellulose pendant 1 h à 37°C avec un immunsérum (des anticorps polyclonaux reconnaissant l'urate oxydase d'A. flavus).
. Le rinçage du filtre de nitrocellulose avec le tampon B.
. La mise en contact du filtre de nitrocellulose pendant 1 h à 37°C avec une solution de protéine G marquée à l'iode 125 à 0,1 microcurie/ml.
. Le rinçage du filtre avec le tampon A.
. Le séchage du filtre entre deux feuilles absorbantes.
. La mise en contact d'un film radiographique.
. La révélation du film.

b) Résultats

On constate que les souches EMY761 pEMR469 ($ura^+$), EMY761 pEMR473 ($ura^+$), EMY761 pEMR469 ($leu^+$) et EMY761 pEMR473 ($leu^+$) produisent une protéine de poids moléculaire apparent d'environ 33 KDa qui est reconnue par les anticorps dirigés contre l'urate oxydase d'A. flavus et qui est absente de la souche témoin.

On constate également que les souches non induites ne produisent pas ou très peu de la protéine décrite ci-dessus.

La comparaison entre les quantités de cette protéine pour les culots et les surnageants permet de déduire qu'environ 80 % de celle-ci est sous forme soluble dans le lysat.

4) Dosage de l'activité urate oxydase :

L'activité urate oxydase a été mesurée sur les surnageants des cellules lysées selon le mode opératoire décrit à l'exemple 9 ci-dessus.

Les résultats obtenus sont rassemblés dans le tableau (IV) ci-après. Celui-ci précise pour chaque souche induite au glycérol-éthanol, induite au glycérol-éthanol-galactose ou non induite, l'activité urate oxydase en U/ml.

TABLEAU IV

| Souche / Inducteur | Activité urate oxydase (U/ml) |
|---|---|
| EMY761/YP éthanol-glycérol-galactose | < 0,1 |
| EMY761/YP éthanol-glycérol | < 0,1 |
| EMY761 pEMR469 ($ura^+$)/(non induit) | 0,4 |
| EMY761 pEMR469 ($ura^+$)/YP éthanol-glycérol | 12 |
| EMY761 pEMR469 ($leu^+$)/(non induit) | 0,17 |
| EMY761 pEMR469 ($leu^+$)/YP éthanol-glycérol | 36 |
| EMY761 pEMR473 ($ura^+$)/(non induit) | < 0,1 |
| EMY761 pEMR473 ($ura^+$)/YP éthanol-glycérol-galactose | 12,5 |
| EMY761 pEMR473 ($leu^+$)/(non induit) | < 0,1 |
| EMY761 pEMR473 ($leu^+$)/YP éthanol-glycérol-galactose | 15,3 |

Il apparaît clairement sur le tableau ci-dessus que les cellules levure transformées par ces plasmides pEMR469 et pEMR473 sont capables après induction de produire une activité urate oxydase.

5) Dosage des protéines totales solubles dans les lysats :

Le kit protein assay de BIORAD a été utilisé pour doser les protéines totales présentes dans le surnageant des cellules lysées. Il est basé sur l'observation que l'absorbance maximum pour une solution acide de bleu brillant de Coomassie g-250 passe de 465 nm à 595 nm lorsque des protéines viennent s'y fixer (Cf. Reisner et al., Anal Biochem, 64, 509-(1975)).

a) Mode opératoire

On introduit dans la cuve d'un spectrophotomètre réglé à 595 nm les volumes suivants :

- 10 µl d'échantillon auxquels ont été rajoutés 790 µl d'eau distillée
- 200 µl de "Dye reagent" concentré (Biorad).

On mélange et on lit la densité optique à 595 nm. Une gamme étalon avec des concentrations croissantes de BSA (Bovine Serum Albumine) a été réalisée ainsi. On lit la concentration inconnue des protéines totales des lysats sur la courbe étalon obtenue.

b) Résultats

Les principaux résultats obtenus sont rassemblés dans le tableau V ci-après. Celui-ci précise pour chaque souche induite au glycérol-éthanol, induite au glycérol-éthanol-galactose ou non induite, la quantité (en mg/ml) de protéines totales solubles ainsi que le pourcentage d'urate oxydase dans les protéines totales solubles (on admet ici que l'activité spécifique de la protéine recombinante est identique à celle de l'urate oxydase obtenue à partir d'A. flavus : 30 U/mg).

TABLEAU V

| Souche / Inducteur | Protéines totales solubles mg/ml | % d'urate oxydase dans les protéines totales solubles |
|---|---|---|
| EMY761/glycérol-éthanol | 5,3 | < 0,05 |
| EMY761/glycérol-éthanol-galactose | 5,8 | < 0,05 |

TABLEAU V (suite)

| Souche / Inducteur | Protéines totales solubles mg/ml | % d'urate oxydase dans les protéines totales solubles |
|---|---|---|
| EMY761 pEMR469 ($ura^+$)/non induit | 8,5 | 0,25 |
| EMY761 pEMR469 ($ura^+$)/glycérol-éthanol | 5,3 | 4,7 |
| EMY761 pEMR69 (leu)/non induit | 1,7 | 0,3 |
| EMY761 pEMR469 ($leu^+$)/glycérol-éthanol | 5,9 | 20 |
| EMY761 pEMR473 ($ura^+$)/non induit | 10,3 | < 0,05 |
| EMY761 pEMR473 ($ura^+$)/glycérol-éthanol-galactose | 6,5 | 6,4 |
| EMY761 pEMR473 ($leu^+$)/non induit | 0,5 | < 0,05 |
| EMY761 pEMR473 ($leu^+$)/glycérol-éthanol-galactose | 3,9 | 13 |

On constate que le taux de production de l'urate oxydase varie de 5 à 20 % selon les transformants et le mode de sélection des transformés ($leu^+$).

EXEMPLE 13 : Expression en fermenteur de 2,5 l de l'ADNc de l'urate oxydase pour la souche EMY761 pEMR473 ($ura^+$)

1) Protocole de fermentation :

a) Milieux

Milieu inoculum

Une colonie de la souche EMY761 pEMR473 ($ura^+$) a été mise en culture dans 200 ml de milieu liquide sans uracile (Cf. tableau III, exemple 11). La culture est poursuivie pendant une nuit sous agitation jusqu'à une DO d'environ 3.

Milieu de culture A

| | pour 1 l d'eau purifiée sur un appareil de type Milli-q |
|---|---|
| glucose | 30 g |
| glycérol | 30 g |
| hydrolysat de caséine (Casamino-acids de DIFCO) | 30 g |
| base azotée de levure (Yeast Nitrogen Base de DIFCO) | 15 g |
| extrait de levure (Yeast extract de DIFCO) | 2,5 g |
| $K_2HPO_4$ | 3 g |
| $MgSO_4,7H_2O$ | 0,5 g |

Milieu additionnel B

| | pour 100 ml d'eau purifiée sur un appareil de type Milli-Q |
|---|---|
| glycérol | 30 g |
| hydrolysat de peptone (le Primatone de G. Sheffield) | 30 g |
| base azotée de levure (Yeast Nitrogen Base de DIFCO) | 15 g |
| extrait de levure (Yeast extract de DIFCO) | 5 g |
| $K_2HPO_4$ | 3 g |
| $MgSO_4,7H_2O$ | 0,5 g |

b) Paramètres de fermentation

Bioréacteur de 2,5 l de volume total équipé de deux turbines
température = 30°C
pH = 5
pression partielle en oxygène = 30 mmHg
débit d'air = 1 l/min.

Le bioréacteur est rempli avec 1,5 l du milieu A et est ensemencé avec 150 ml de l'inoculum.

Une fois que le glucose est épuisé à DO 2,5 vers DO 17, l'induction a lieu par addition d'un volume de 150 ml de galactose à 20 % poids/volume. La croissance est poursuivie, puis le milieu additionnel B est ajouté aux environs de DO 30.

La croissance se prolonge une quinzaine d'heures et la récolte a été effectuée à une DO 104.

2) Préparation et analyse des échantillons :

Les échantillons ont été préparés comme décrit dans l'exemple 9 2) a) à partir de la culture en fermenteur. Deux prélèvements ont été réalisés : le premier après 7 h d'induction, le second après 22 h d'induction.

Sur ces deux lysats obtenus après lyse des cellules, les tests suivants décrits dans l'exemple 9 ont été réalisés :

- immunodétection par Western Blot
- dosage de l'activité biologique
- dosage des protéines totales.

Les résultats obtenus sont les suivants.

a) Immunodétection par Western Blot

On constate que la souche EMY761 pEMR473 ($ura^+$) cultivée en fermenteur de 2 l produit une protéine de poids moléculaire apparent de 33 KDa qui est reconnue par les anticorps dirigés contre l'urate oxydase d'A. flavus : (préparés chez le lapin selon des techniques bien connues de l'homme de l'art : Cf. VAITUKAITIS et al. (1981) "Methods in Enzymology" Academic Press, New York, vol. 73, p. 46) et qui est absente dans la souche témoin.

b) Dosage de l'activité biologique

Les résultats obtenus sont rassemblés dans le tableau VI ci-après :

TABLEAU VI

| Souche / Temps d'induction | U/ml |
|---|---|
| EMY761 pEMR473 ($ura^+$)/7 h | 9 |
| EMY761 pEMR473 ($ura^+$)/22 h | 12,5 |

On constate que la souche EMY761 pEMR473 ($ura^+$), cultivée en fermenteur, est capable, après induction, de produire une activité urate oxydase.

c) Dosage des protéines totales solubles

Les résultats sont rassemblés dans le tableau VII ci-après :

TABLEAU VII

| Souche / Temps d'induction | Protéines totales solubles mg/ml | % d'urate oxydase dans les protéines totales solubles |
|---|---|---|
| EMY761 pEMR473 ($ura^+$)/7 h | 5,2 | 5,7 |
| EMY761 pEMR473 ($ura^+$)/21 h | 6,2 | 6,6 |

Ces résultats indiquent que le taux de synthèse en urate oxydase de la souche EMY761 pEMR473 ($ura^+$) cultivée en fermenteur est d'environ 5 % de protéines totales de la cellule après 7 h et 21 h d'induction.

EXEMPLE 14 : Expression en erlenmeyer de l'ADNc de l'urate oxydase par les souches EMY761 pEMR515 ($leu^+$), EMY500 pEMR515 ($leu^+$) et GRF18 pEMR515 ($leu^+$)

Une colonie de chacune des trois souches ci-dessus a été mise en culture dans 20 ml de milieu liquide sans leucine.

Après une nuit à 30°C sous agitation, les trois cultures ont été centrifugées pendant 10 min à 7 000 tr/min. Les culots cellulaires ont été repris dans 10 ml d'eau distillée stérile et de nouveau centrifugés pendant 10 min. L'expression de l'urate oxydase a été induite en reprenant les cellules dans 20 ml de milieu YP éthanol-glycérol-galactose (Cf. tableau I, exemple 8). Les cultures ont été replacées à 30°C sous agitation pendant environ 20 h. En témoin, une culture de chaque souche hôte, non transformée, a été effectuée.

Les cellules de chacune des six cultures sont reculotées par centrifugation et le surnageant est éliminé. Les culots ont été repris dans 10 ml d'eau distillée et centrifugés pendant 10 min à 7 000 tr/min. Les culots ainsi lavés ont été repris dans environ 1 ml de tampon TEA de pH 8,9 et le broyage ainsi que l'élimination des particules par centrifugation ont été réalisés comme décrit dans l'exemple 9, 2). Le surnageant de chaque culture sert comme précédemment à un dosage de l'urate oxydase et des protéines totales. Les principaux résultats obtenus sont rassemblés dans le tableau VIII ci-après :

TABLEAU VIII

| Souche/Conditions de culture | Activité urate oxydase (U/ml) | Protéines totales solubles (mg/ml) | % d'urate oxydase dans les protéines solubles |
|---|---|---|---|
| GRF18 pEMR515 ($leu^+$)/a) | < 0,1 | 2,2 | < 0,05 |
| EMY500 pEMR515 ($leu^+$)/a) | < 0,1 | 0,9 | < 0,05 |
| EMY761 pEMR515 ($leu^+$)/a) | < 0,1 | 1,8 | < 0,05 |
| GRF18 pEMR515 ($leu^+$)/b) | 38 | 5,4 | 23 % |
| EMY500 pEMR515 ($leu^+$)/b) | 20 | 2,5 | 26 % |

a) : les souches sont cultivées en présence de glucose (conditions de non-induction)

b) : les souches sont cultivées en absence de glucose et en présence de galactose (induction).

TABLEAU VIII (suite)

| Souche/Conditions de culture | Activité urate oxydase (U/ml) | Protéines totales solubles (mg/ml) | % d'urate oxydase dans les protéines solubles |
|---|---|---|---|
| EMY761 pEMR515 (leu+)/b) | 33 | 4,2 | 26 % |

b) : les souches sont cultivées en absence de glucose et en présence de galactose (induction).

Ces résultats montrent que l'on peut obtenir un haut niveau d'expression d'urate oxydase avec trois souches réceptrices non isogéniques transformées par le vecteur d'expression selon l'invention.

EXEMPLE 15 : Expression en fermenteur de 2,5 l de l'ADNc de l'urate oxydase pour la souche EMY500 pEMR515. Purification et caractérisation partielle de l'urate oxydase recombinante

1) Culture en fermenteur de 2,5 l de la souche EMY500 pEMR515 :

La culture de la souche EMY500 pEMR515 se fait en fermenteur et se déroule de la manière suivante :

a) Phase de préculture en erlenmeyer

A partir de 1 ml de solution dans un milieu contenant 20 % de glycérol de la souche EMY500 pEMR515 avec un nombre de cellules correspondant à une DO de 2,35, on ensemence un erlenmeyer de 500 ml contenant 90 ml de milieu de croissance phase autoclave MCPA complémenté par 1,28 g de MES (2/N-morpholino/-ethanesulfonic acid : Sigma n° M 8250) et 10 ml de milieu de croissance phase filtrée MCPF. Les compositions des milieux MCPA et MCPF sont précisées ci-après. Après 24 heures d'incubation, sous agitation à 30°C, la densité optique de la culture est d'environ 7.

b) Phases de culture en fermenteur

La culture ci-dessus est utilisée pour ensemencer un fermenteur 2,5 l, contenant le milieu de culture de composition suivante :

900 ml de MCPA
\+ 200 ml de MCPF

Le pH de la culture est régulé par le fermenteur à la valeur de consigne qui est 5,5. Après 6 à 7 h de culture à 30°C, on ajoute de manière linéaire et pendant 9 h, 72 ml d'une solution de glucose à 500 g/l soit en tout 36 g de glucose.

c) Phase d'expression

Au mélange précédemment décrit, on ajoute 100 ml de milieu d'expression phase autoclavable MEPA et 150 ml de milieu d'expression phase filtrée MEPF (dont les compositions sont précisées ci-après). La culture est alors continuée pendant 5 h. Puis, on ajoute de façon linéaire, pendant 20 h, 150 ml d'une solution contenant 30 g de galactose, 15 g de glycérol et 36 g d'éthanol. On obtient alors une D.O. voisine de 160.

COMPOSITION CHIMIQUE DES MILIEUX DE CROISSANCE ET D'EXPRESSION

- Milieu de croissance phase autoclavable MCPA

| | pour 900 ml final |
|---|---|
| NTA (acide nitrilotriacétique) | 1,2 g |
| extrait de levure (DIFCO) | 6 g |
| $K_2SO_4$ | 1,2 g |
| NaCl | 0,6 g |
| $MgSO_4$, $7H_2O$ | 1,2 g |
| $CaCl_2$, $2H_2O$ | 840 mg |
| $FeCl_3$ | 108 mg |
| acide glutamique | 4,44 g |
| HYCASE SF (Sheffield Products) | 30 g |
| leucine | 2,16 g |
| histidine | 600 mg |
| méthionine | 1,2 g |
| oligoéléments I (Cf. ci-après) | 5 ml |
| uracile | 1,2 g |

Liste des oligoéléments 1

| | pour 1 l d'eau ultrapurifiée |
|---|---|
| $CUSO_4$, $5H_2O$ | 780 mg |
| $H_3BO_3$ | 5 g |
| $ZuSO_4$, $7H_2O$ | 3 g |
| KI | 1 g |
| $MnSO_4$, $2H_2O$ | 3,5 g |
| $Na_2MO_4$, $2H_2O$ | 2 g |
| $FeCl_3$, $6H_2O$ | 4,8 g |

Ajouter à la solution 100 ml d'acide chlorhydrique concentré. Compléter à 1 000 ml.

- Milieu de croissance phase filtrée MCPF

| | pour 200 ml final d'eau ultrapurifiée |
|---|---|
| $KH_2PO_4$ | 4,8 g |
| tryptophane | 420 mg |
| vitamine I (Cf. ci-après) | 5 ml |
| glucose | 36 g |

Chauffer pour dissoudre, tempérer, ajouter les vitamines I et filtrer à 0,2 µ.

Liste des vitamines I

| | pour 100 ml final d'eau ultrapurifiée |
|---|---|
| biotine | 1,2 mg |
| acide folique | 1 mg |
| niacine | 144 mg |
| (acide nicotinique pyridoxine. HCl | 60 mg |
| thiamine. HCl | 240 mg |
| pantothénate de calcium | 1,2 g |
| mésoinositol | 2,4 g |

Compléter à 100 ml après dissolution.
Filtrer à froid stérilement à 0,2 µ.

- Milieu d'expression phase autoclavable MEPA

| | pour 100 ml d'eau ultrapurifiée |
|---|---|
| NTA | 1,2 g |
| $K_2SO_4$ | 2,08 g |
| acide glutamique | 6 g |
| HYCASE SF (Sheffield Products) | 24 g |
| leucine | 2,16 g |
| histidine | 600 mg |
| méthionine | 1,2 g |
| $MgSO_4$, $7H_2O$ | 720 g |
| $CaCl_2$, $2H_2O$ | 840 mg |
| $FeCl_3$, $6H_2O$ | 108 mg |
| oligoéléments liste 1 | 5 ml |
| uracile | 1,2 g |

Ajuster le pH à 5,5 avec $H_2SO_4$ concentré ou KOH concentré.
Autoclaver 20 min à 120°C.

- Milieu d'expression phase filtrée MEPF

| | pour 150 ml final d'eau ultrapurifiée |
|---|---|
| $KH_2PO_4$ | 2,4 g |
| tryptophane | 420 mg |
| vitamines I | 5 ml |
| glycérol | 36 g |
| galactose | 45 g |

Chauffer pour dissoudre, tempérer, ajouter les vitamines et filtrer.

2) Broyage des cellules

Après 20 heures d'induction, la densité optique, mesurée à 600 nm, de la culture est de 98. 800 g de moût fermentation sont centrifugés 5 min à 10 000 g et le culot cellulaire est repris dans 80 ml de tampon de lyse (glycine 20 mM pH 8,5). Les cellules sont alors broyées à 4°C, 2 fois pendant 2,5 min dans un broyeur (Vibrogen Zellmühle V14) en présence d'un volume de billes de 0,5 mm de diamètre, égal au volume de la solution de cellules à lyser. Après broyage, le surnageant est repris et les billes sont lavées deux fois par 80 ml de tampon de lyse. On récupère 210 ml de lysat possédant un taux de protéines totales d'environ 3 mg/ml et une activité urate oxydase d'environ 7,7 U/ml (soit un pourcentage d'urate oxydase par rapport aux protéines totales d'environ 8,5 % en supposant une activité spécifique de cette dernière de 30 U/mg).

3) Purification de l'urate oxydase recombinante

a) Protocole de purification

On soumet le lysat précédent au protocole de purification à deux étapes décrit ci-après.

ETAPE 1 :

Chromatographie anionique :

Support :

DEAE (diéthylaminosulfate) sépharose fast flow (Pharmacia réf. 17.07.09.91).
Le gel une fois tassé occupe un volume de 70 ml.
La séparation est effectuée à température ambiante, les fractions récoltées, conservées à 0°C.

Conditions de séparation :

On utilise un gradient de force ionique de chlorure entre un tampon 1 (borate de sodium 10 mM, pH 9,2) et un tampon 2 (borate de sodium 10 mM, chlorure de sodium 1 M). Les tampons sont préalablement dégazés et lors de l'élution sont conservés à 0°C. Dans chaque tampon il a été ajouté l'équivalent de 0,02 % d'azide.

L'extrait brut est déposé (10 ml) et élué avec le tampon 1 jusqu'à la collecte complète de l'urate oxydase (par fractions de 10 ml), qui n'est pas retenue sur la colonne.

Les pigments et les protéines contaminantes sont ensuite éliminés en éluant avec le tampon 2.

La purification est suivie par mesure de la densité optique de l'éluat à 214 nm.

ETAPE 2 :

Chromatographie liquide haute pression sur phase inverse :

Support :

Colonne à base de silice greffée C8, l'Aquapore OD-300 (100 x 2,1 mm) (Brownlee-Applied Biosystems)

Conditions opératoires :

Eluant 1 : Eau ultrapurifiée (passée à travers un système Millipore) avec 0,1 % d'acide trifluoroacétique.
Eluant 2 : Acétonitrile (de qualité spectrophotométrique ou équivalent) avec 0,08 % d'acide trifluoroacétique.
Débit : 0,3 ml/min.
Le gradient est de 35 % acétonitrile/TFA à 70 % acétonitrile/TFA en 20 min, on maintient à 70 % pendant 5 min. La quantité injectée est de 1 ml par run.

Collecte des fractions :

Le déroulement de la séparation est suivi par mesure de la densité optique à 218 nm. L'acétonitrile est évaporé lors de la centrifugation sous vide.

b) Résultats :

L'échantillon avant et après la première étape de purification a été analysé par chromatographie liquide sur une colonne de silice greffée C8, l'Aquapore OD-300 décrite précédemment avec le même gradient, avec une quantité injectée de 50 µl. On utilise comme témoin externe l'urate oxydase d'A. flavus purifiée.

Dans le lysat de départ, l'urate oxydase représente 63 % des protéines totales. Après la première étape de purification l'urate oxydase représente 84 % des protéines totales.

La totalité de l'échantillon obtenu à l'issue de la seconde étape, lequel contient certainement plus de 84 % d'urate oxydase, a été utilisée pour la caractérisation partielle décrite ci-après.

4) Caractérisation partielle de l'urate oxydase recombinante.

a) Analyse d'acides aminés

L'analyse des acides aminés de l'hydrolysat acide de l'urate oxydase recombinante purifiée a été effectuée sur un analyseur d'Applied Biosystems modèle 420-130A. La répartition des acides aminés quantifiés est compatible (pas de différence significative) avec la séquence supposée. Le même résultat a été observé pour l'urate oxydate d'A. flavus extractive purifiée (obtenue à l'exemple 4).

b) Carte peptidique tryptique

Une carte peptidique tryptique a été établie pour l'urate oxydase recombinante purifiée et pour l'urate oxydase extractive purifiée (obtenue à l'exemple 4) dans les conditions suivantes :

On prépare une solution d'urage oxydase à une concentration d'environ 1 mg/ml et extemporanément une solution de trypsine à une concentration de 1 mg/ml.

Les deux solutions sont mises en présence dans une proportion enzyme/substrat de 1/30 pendant 8 heures à température ambiante. L'hydrolysat tryptique est ensuite soumis à une chromatographie en phase liquide sur une colonne à base de silice greffée C18,5 µm, lichrosorb (250 x 4,6 mm) (Hichrom-réf.RP 18-5-250A), équipé d'un détecteur UV à 218 couplé à un enregistreur. Le gradient appliqué est de 1 % acétonitrile/TFA à 60 % acétonitrile/TFA en 120 min, puis on maintient à 60 % pendant 5 min.

Les cartes peptidiques obtenues ont un profil très proche.

3) Mise en évidence du caractère bloqué de la séquence amino-terminale :

La séquence amino-terminale a été analysée à l'aide d'un séquenceur Applied Biosystem modèle 470A, couplé à un analyseur de dérivés phénylthioidantoïques Applied Biosystem modèle 120A. L'urate oxydase recombinante purifiée (200 pmoles contrôlées par analyse d'acides aminés) a été déposée sur le séquenceur en présence de 20 pmoles de β-lactoglobuline, protéine témoin.

Aucune séquence amino-terminale correspondant à une séquence de l'urate oxydase n'a été détectée (par contre la séquence amino-terminale de la protéine témoin a été détectée).

L'urate oxydase recombinante a donc, comme l'urate oxydase extractive, l'extrémité amino-terminale bloquée.

<u>Exemple 16</u> : <u>Construction d'un vecteur d'expression de l'ADNc de l'urate oxydase dans les cellules animales, le plasmide PSV860.</u>

Ce vecteur a été obtenu par :

- ligation du petit fragment AccI-SnaBI contenant une séquence codant pour l'urate oxydase à l'exception des 16 premiers acides aminés, issu du plasmide p466 : vecteur d'expression de l'urate oxydase d'A. flavus dans E. coli disponible au laboratoire et décrit ci-après, à un fragment synthétique HindIII-AccI, ce qui a permis d'obtenir un fragment HindIII-SnaBI contenant une séquence complète codant pour l'urate oxydase d'A. flavus et une séquence 5′ non traduite favorable à l'expression dans les cellules animales.
- Insertion du fragment HindIII-SnaBI entre les sites HindIII et SnaBI du polysite de clonage (également appelé polylinker) du vecteur d'expression pour les cellules animales, le plasmide $pSE_1$.

L'exposé ci-après présentera successivement la construction du plasmide p466, celle du plasmide $pSE_1$ et l'assemblage du plasmide pSV860.

1) Construction du plasmide p466

On a préparé le plasmide p466, vecteur d'expression de l'ADNc de l'urate oxydase dans E. coli. Ce dernier comprend un fragment de pBR327 incluant l'origine de réplication et le gène de résistance à l'ampicilline, il comprend également un promoteur synthétique d'E. coli (R. RODRIGUEZ et M.CHAMBERLIN "Promoters-Structure and function (1982) Preager), une séquence de Shine-Dalgarno, suivie d'un polylinker présentant les sites uniques NdeI et KpnI, un terminateur de transcription (dérivé du phage fd) et le gène lac i.

Ce plasmide a été construit à partir d'un plasmide d'expression de l'hGH dans E. coli (p462) par substitution d'un fragment portant le gène de l'hGH par l'ADNc de l'urate oxydase.

La construction du plasmide p466 a été décrite en détail dans l'exemple 7 ci-dessus.

2) Construction d'un vecteur d'expression pour les cellules animales, le plasmide $pSE_1$

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. MANIATIS, EF. FRITSCH et J. SAMBROOK dans "Molecular Cloning, a laboratory manual" (Cold Spring Harbor Laboratory, 1984). La synthèse des oligonucléotides est réalisée à l'aide d'un synthétiseur d'ADN Biosearch 4 600.

La description ci-après sera mieux comprise en référence à la figure 13, qui représente une carte d'assemblage du plasmide $pSE_1$, les sites ayant disparu par ligation étant notés entre parenthèses. Les symboles utilisés dans cette figure seront précisés dans l'exposé ci-après.

Ce plasmide a été construit par ligations successives des éléments ci-après :

1) - un fragment PvuII-PvuII - symbolisé par ++++++ sur la figure 13 - de 2525 pb, obtenu par digestion complète du plasmide pTZ18R (Pharmacia) à l'aide de l'enzyme de restriction PvuII. Ce fragment contient l'origine de réplication du phage F1 (notée ORI F1 sur la figure 13), un gène (noté $Amp^R$ sur la figure 13) portant la résistance à l'ampicilline et l'origine de réplication (notée ORI pBR322 sur la figure 13) permettant la réplication de ce plasmide dans E. coli. Le premier site franc PvuII disparaît par ligation avec le site franc EcoRV (qui disparaît également) du fragment décrit en 7).

2) - Un fragment PvuII-HpaI - symbolisé par ▬ sur la figure 13 - de 1060 pb de l'ADN d'adénovirus type 5 entre les positions 11299 (site de restriction PvuII) et 10239 (site de restriction HpaI) (DEKKER * VAN ORMONDT, Gene 27, 1984, 115-120) contenant l'information pour les ARN VA-I et VA-II. Le site franc HpaI disparaît par ligation avec le site franc PvuII (qui disparaît également) du fragment décrit en 3).

3) - Un fragment PvuII-HindIII - symbolisé par ZZZ sur la figure 13 - de 344 pb, issu de l'ADN du virus SV40 obtenu par digestion complète à l'aide des enzymes de restriction PvuII et HindIII. Ce fragment comporte l'origine de réplication et le promoteur précoce de l'ADN du virus SV40 (réf. B.J. BYRNE et al. PNAS-USA (1983), 80, 721-725).

Le site HindIII disparaît par ligation avec le site liant à HindIII du fragment décrit en 4).

4) - Un fragment synthétique "site liant à HindIII"-HindIII - symbolisé par ═══ sur la figure 13 - de 419 pb dont la séquence donnée ci-après est proche de la séquence 5' non traduite du virus HTLV1 (réf. WEISS et al, "Molecular Biology of Tumor Viruses - part 2-2e ed - 1985 - Cold Spring Harbor Laboratory - p. 1057).

```
site liant à HindIII
▼AGCTGGCTCGCATCTCTCCTTCACGCGCCCGCCGCCCTACCTGAGGCCGCCATCCACGCC
1 ---------+---------+---------+---------+---------+---------+ 60
     CCGAGCGTAGAGAGGAAGTGCGCGGGCGGCGGGATGGACTCCGGCGGTAGGTGCGG

GGTGAGTCGCGTTCTGCCGCCTCCCGCCTGTGGTGCCTCCTGAACTGCGTCCGCCGTCTA
61 ---------+---------+---------+---------+---------+---------+120
CCACTCAGCGCAAGACGGCGGAGGGCGGACACCACGGAGGACTTGACGCAGGCGGCAGAT

GGTAGGCTCCAAGGGAGCCGGACAAAGGCCCGGTCTCGACCTGAGCTCTAAACTTACCTA
121 ---------+---------+---------+---------+---------+---------+180
CCATCCGAGGTTCCCTCGGCCTGTTTCCGGGCCAGAGCTGGACTCGAGATTTGAATGGAT

GACTCAGCCGGCTCTCCACGCTTTGCCTGACCCTGCTTGCTCAACTCTACGTCTTTGTTT
181 ---------+---------+---------+---------+---------+---------+240
CTGAGTCGGCCGAGAGGTGCGAAACGGACTGGGACGAACGAGTTGAGATGCAGAAACAAA

CGTTTTCTGTTCTGCGCCGTTACAACTTCAAGGTATGCGCTGGGACCTGGCAGGCGGCAT
241 ---------+---------+---------+---------+---------+---------+300
GCAAAAGACAAGACGCGGCAATGTTGAAGTTCCATACGCGACCCTGGACCGTCCGCCGTA

CTGGGACCCCTAGGAAGGGCTTGGGGGTCCTCGTGCCCAAGGCAGGGAACATAGTGGTCC
301 ---------+---------+---------+---------+---------+---------+360
GACCCTGGGGATCCTTCCCGAACCCCCAGGAGCACGGGTTCCGTCCCTTGTATCACCAGG

CAGGAAGGGGAGCAGAGGCATCAGGGTGTCCACTTTGTCTCCGCAGCTCCTGAGCCTGCA
361 ---------+---------+---------+---------+---------+---------+420
GTCCTTCCCCTCGTCTCCGTAGTCCCACAGGTGAAACAGAGGCGTCGAGGACTCGGACGT

GA
------
CTTCGA▲
      HindIII
```

5) - Un fragment synthétique HindIII "site liant à BamHI" - symbolisé par XXXX sur la figure 13 - contenant le promoteur de l'ARN-polymérase du phage T7 ainsi qu'un polylinker contenant le site de clonage SmaI.

```
AGCTTGTCGACTAATACGACTCACTATAGGGCGGCCGCGGGCCCCTGCAGGAATTC
    ACAGCTGATTATGCTGAGTGATATCCCGCCGGCGCCCGGGGACGTCCTTAAG
    ▲
    HindIII

          SmaI          site liant à BamHI
          ▼
GGATCCCCCGGGTGACTGACT  ▼

CCTAGGGGGCCCACTGACTGACTAG
```

6) - Un fragment BamHI-BclI de 240 pb - représenté par sur la figure 13 -, petit fragment obtenu par digestion complète à l'aide des enzymes BclI et BamHI du virus SV40 qui contient le site de polyadénylation tardif de ce dernier. (M. FITZGERALD et al. Cell, 24, 1981, 251-260). Les sites BamHI et BclI disparaissent par ligations avec respectivement le site liant à BamHI du fragment décrit en 5) et le site BamHI (qui disparaît également du fragment décrit en 7).

7) - Un fragment BamHI-EcoRV - symbolisé par sur la figure 13 - de 190 pb, petit fragment issu du plasmide pBR322 après digestion complète à l'aide des enzymes EcoRV et BamHI.

3) Construction du plasmide pSV860

Le plasmide p466 (cf. figure 9) a été soumis à une digestion complète à l'aide des enzymes AccI et SnaBI. Le petit fragment AccI-SnaBI, qui contient une séquence d'ADN codant pour l'urate oxydase à l'exception des 16 premiers acides aminoterminaux, a été purifié et ligué au fragment synthétique HindIII-AccI de séquence suivante :

```
HindIII                                                      AccI
▼                                                               ▼
 AGCTTGCCGCCACTATGTCCGCAGTAAAAGCAGCCCGCTACGGCAAGGACAATGTCCGCGT
 ---------+---------+---------+---------+---------+---------+
     ACGGCGGTGATACAGGCGTCATTTTCGTCGGGCGATGCCGTTCCTGTTACAGGCGCAGA
```

Cette ligation permet d'obtenir le fragment HindIII-SnaBI, qui contient une séquence codant pour l'urate oxydase identique à celle du clone 9C et une séquence 5′ non traduite favorable à l'expression dans les cellules animales (KOZAK, M., Nucl. Acids Res., 12, 2, 1984, 857-872).

Le fragment HindIII-SnaBI contient la séquence :

```
5' -AGCTTGCCG  CCACTATGTC  CGCAGTAAAA  GCAGCCCGCT  ACGGCAAGGA
   CAATGTCCGC  GTCTACAAGG  TTCACAAGGA  CGAGAAGACC  GGTGTCCAGA
   CGGTGTACGA  GATGACCGTC  TGTGTGCTTC  TGGAGGGTGA  GATTGAGACC
   TCTTACACCA  AGGCCGACAA  CAGCGTCATT  GTCGCAACCG  ACTCCATTAA
   GAACACCATT  TACATCACCG  CCAAGCAGAA  CCCCGTTACT  CCTCCCGAGC
   TGTTCGGCTC  CATCCTGGGC  ACACACTTCA  TTGAGAAGTA  CAACCACATC
   CATGCCGCTC  ACGTCAACAT  TGTCTGCCAC  CGCTGGACCC  GGATGGACAT
   TGACGGCAAG  CCACACCCTC  ACTCCTTCAT  CCGCGACAGC  GAGGAGAAGC
   GGAATGTGCA  GGTGGACGTG  GTCGAGGGCA  AGGGCATCGA  TATCAAGTCG
   TCTCTGTCCG  GCCTGACCGT  GCTGAAGAGC  ACCAACTCGC  AGTTCTGGGG
   CTTCCTGCGT  GACGAGTACA  CCACACTTAA  GGAGACCTGG  GACCGTATCC
   TGAGCACCGA  CGTCGATGCC  ACTTGGCAGT  GGAAGAATTT  CAGTGGACTC
   CAGGAGGTCC  GCTCGCACGT  GCCTAAGTTC  GATGCTACCT  GGGCCACTGC
   TCGCGAGGTC  ACTCTGAAGA  CTTTTGCTGA  AGATAACAGT  GCCAGCGTGC
   AGGCCACTAT  GTACAAGATG  GCAGAGCAAA  TCCTGGCGCG  CCAGCAGCTG
   ATCGAGACTG  TCGAGTACTC  GTTGCCTAAC  AAGCACTATT  TCGAAATCGA
   CCTGAGCTGG  CACAAGGGCC  TCCAAAACAC  CGGCAAGAAC  GCCGAGGTCT
   TCGCTCCTCA  GTCGGACCCC  AACGGTCTGA  TCAAGTGTAC  CGTCGGCCGG
   TCCTCTCTGA  AGTCTAAATT  G
```

Le fragment HindIII-SnaBI a ensuite été inséré dans le vecteur $pSE_1$ préalablement incubé avec les enzymes HindIII et SmaI. On a ainsi obtenu le plasmide pSV860, représenté sur la figure 14, dans laquelle les symboles ont la même signification que dans la figure 13, le nouveau fragment HindIII-SnaBI étant symbolisé par (Les sites SnaBI et SmaI ont disparu par ligation).

Exemple 17 : Expression transitoire de l'ADNc de l'urate oxydase dans les cellules COS. Dosage de l'activité urate oxydase dans le lysat cellulaire.

Les cellules COS sont des cellules de reins de singe exprimant l'antigène T du virus SV40 (Gluzman, Y. Cell 23, 1981, 175-182). Ces cellules, qui permettent la réplication des vecteurs contenant l'origine de réplication de l'ADN du virus SV40, constituent des hôtes de choix pour l'étude de l'expression de gènes dans les cellules animales.

1) Transfection des cellules COS et expression transitoire de l'ADNc de l'urate oxydase.

$4.10^5$ cellules COS sont étalées dans une boîte de Pétri de 6 cm de diamètre (Corning) dans 5 ml de milieu modifié d'Eagle selon Dulbecco ci-après appelé DMEM (le Dulbecco's modified Eagles medium de Gibco), lequel contient 0,6 g/l glutamine, 3,7 g/l $NaHCO_3$ et est complémenté avec du sérum de veau foetal (GIBCO) à raison de 5%. Après environ 16 h de culture à 37°C dans une atmosphère contenant 5% de dioxyde de carbone, le milieu de culture est enlevé par aspiration et les cellules sont lavées avec 3 ml de tampon PBS (le Phosphate Buffer Saline de GIBCO). On y ajoute alors le mélange suivant : 1 000 µl de (DMEM + 10% sérum de veau foetal (GIBCO)), 110 µl de diéthylaminoéthyl-dextrane de poids moléculaire moyen 500 000, à une concentration de 2 mg/ml (Pharmacia), 1,1 µl de chloroquine 100mM (Sigma) et 3 µg d'ADN soit du plasmide pSV860, soit du plasmide $pSE_1$ (pour le témoin). Après 5 h d'incubation à 37°C dans une atmosphère contenant 5% de dioxyde de carbone, le mélange est retiré des cellules. On y ajoute alors 2 ml de tampon PBS contenant 10% de diméthyl sulfoxyde (qualité Spectroscopie, Merck). Après 1 min d'incubation à la température ambiante, le mélange est retiré et les cellules sont lavées deux fois avec du tampon PBS. On y ajoute 5 ml de DMEM complémenté avec du sérum de veau foetal à raison de 2%. L'incubation est poursuivie pendant 4 jours à 37°C sous atmosphère contenant 5% de dioxyde de carbone.

2) Préparation des échantillons.

Le milieu de culture est aspiré et les cellules COS sont rincées deux fois avec 3 ml de tampon PBS. Les cellules sont alors recueillies par grattage avec une spatule en caoutchouc (un "Policeman") dans 1 ml de tampon PBS. Après grattage, la boîte est rincée avec 1 ml de tampon PBS. Les deux suspensions cellulaires sont combinées et centrifugées pendant 10 min à 1 000 tr/min. Le surnageant est enlevé et le culot cellulaire est remis en suspension dans 1 ml de tampon de triéthanolamine (TEA) 0,05 M de pH 8,9/EDTA.

Les cellules sont lysées par sonication (sur de la glace) par des pulsations de 10 s avec un sonicateur (le Vibra Cell de Sonics and Materials Inc. USA) réglé à une puissance de 12 W. Le lysat cellulaire est centrifugé pendant 10 min à 10 000 tr/min et le surnageant est récupéré pour le dosage de l'urate oxydase.

3) Dosage de l'activité urate oxydase.

Le dosage de l'activité urate oxydase a été effectué comme décrit à l'exemple 9.

Les résultats sont rassemblés dans le tableau ci-après :

| Cellules COS transfectées par | Activité urate oxydase U/ml |
|---|---|
| pSV860 | 0,105 |
| $pSE_1$ | <0,01 |

On constate que les cellules COS transfectées par le plasmide pSV860, porteur de l'ADNc de l'urate oxydase, expriment un niveau appréciable d'activité urate oxydase, alors qu'aucune activité urate oxydase n'est détectable sur le témoin. Il y a donc expression de l'ADNc de l'urate oxydase.

## Revendications

1. Protéine recombinante, caractérisée en ce qu'elle présente une activité urate oxydase spécifique d'au moins 16 U/mg et en ce qu'elle a la séquence ci-après :

| | | | | | | | | | | | | | | | |
|---|---|---|---|---|---|---|---|---|---|---|---|---|---|---|---|
| Ser | Ala | Val | Lys | Ala | Ala | Arg | Tyr | Gly | Lys | Asp | Asn | Val | Arg | Val | Tyr |
| 1 | | | | 5 | | | | | 10 | | | | | 15 | |
| Lys | Val | His | Lys | Asp | Glu | Lys | Thr | Gly | Val | Gln | Thr | Val | Tyr | Glu | Met |
| | | | 20 | | | | | 25 | | | | | 30 | | |
| Thr | Val | Cys | Val | Leu | Leu | Glu | Gly | Glu | Ile | Glu | Thr | Ser | Tyr | Thr | Lys |
| | | 35 | | | | | 40 | | | | | 45 | | | |
| Ala | Asp | Asn | Ser | Val | Ile | Val | Ala | Thr | Asp | Ser | Ile | Lys | Asn | Thr | Ile |
| | 50 | | | | | 55 | | | | | 60 | | | | |
| Tyr | Ile | Thr | Ala | Lys | Gln | Asn | Pro | Val | Thr | Pro | Pro | Glu | Leu | Phe | Gly |
| 65 | | | | | 70 | | | | | 75 | | | | | 80 |
| Ser | Ile | Leu | Gly | Thr | His | Phe | Ile | Glu | Lys | Tyr | Asn | His | Ile | His | Ala |
| | | | | 85 | | | | | 90 | | | | | 95 | |
| Ala | His | Val | Asn | Ile | Val | Cys | His | Arg | Trp | Thr | Arg | Met | Asp | Ile | Asp |
| | | | 100 | | | | | 105 | | | | | 110 | | |
| Gly | Lys | Pro | His | Pro | His | Ser | Phe | Ile | Arg | Asp | Ser | Glu | Glu | Lys | Arg |
| | | 115 | | | | | 120 | | | | | 125 | | | |
| Asn | Val | Gln | Val | Asp | Val | Val | GIu | Gly | Lys | Gly | Ile | Asp | Ile | Lys | Ser |
| | 130 | | | | | 135 | | | | | 140 | | | | |
| Ser | Leu | Ser | Gly | Leu | Thr | Val | Leu | Lys | Ser | Thr | Asn | Ser | Gln | Phe | Trp |
| 145 | | | | | 150 | | | | | 155 | | | | | 160 |
| Gly | Phe | Leu | Arg | Asp | Glu | Tyr | Thr | Thr | Leu | Lys | Glu | Thr | Trp | Asp | Arg |
| | | | | 165 | | | | | 170 | | | | | 175 | |
| Ile | Leu | Ser | Thr | Asp | Val | Asp | Ala | Thr | Trp | Gln | Trp | Lys | Asn | Phe | Ser |
| | | | 180 | | | | | 185 | | | | | 190 | | |
| Gly | Leu | Gln | Glu | Val | Arg | Ser | His | Val | Pro | Lys | Phe | Asp | Ala | Thr | Trp |
| | | 195 | | | | | 200 | | | | | 205 | | | |
| Ala | Thr | Ala | Arg | Glu | Val | Thr | Leu | Lys | Thr | Phe | Ala | Glu | Asp | Asn | Ser |
| | 210 | | | | | 215 | | | | | 220 | | | | |
| Ala | Ser | Val | Gln | Ala | Thr | Met | Tyr | Lys | Met | Ala | Glu | Gln | Ile | Leu | Ala |
| 225 | | | | | 230 | | | | | 235 | | | | | 240 |
| Arg | Gln | Gln | Leu | Ile | Glu | Thr | Val | Glu | Tyr | Ser | Leu | Pro | Asn | Lys | His |
| | | | | 245 | | | | | 250 | | | | | 255 | |
| Tyr | Phe | Glu | Ile | Asp | Leu | Ser | Trp | His | Lys | Gly | Leu | Gln | Asn | Thr | Gly |
| | | | 260 | | | | | 265 | | | | | 270 | | |
| Lys | Asn | Ala | Glu | Val | Phe | Ala | Pro | Gln | Ser | Asp | Pro | Asn | Gly | Leu | Ile |
| | | 275 | | | | | 280 | | | | | 285 | | | |
| Lys | Cys | Thr | Val | Gly | Arg | Ser | Ser | Leu | Lys | Ser | Lys | Leu | | | |
| | 290 | | | | | 295 | | | | | 300 | | | | |

précédée éventuellement d'une méthionine.

2. Protéine recombinante selon la revendication 1, caractérisée en ce qu'elle présente une activité urate oxydase spécifique d'environ 30 U/mg.

3. Protéine recombinante selon l'une des revendications 1 et 2, caractérisée en ce que, par analyse sur gel bidimensionnel, elle présente un spot de masse moléculaire d'environ 33,5 kDa, représentant au moins 90 % de la masse

protéique.

4. Protéine recombinante selon l'une quelconque des revendications 1 à 3, caractérisée en ce qu'elle a un taux de pureté, déterminé par chromatographie liquide sur une colonne de silice greffée C8, supérieur à 80 %.

5. Protéine recombinante selon l'une quelconque des revendications 1 à 4, caractérisée en ce qu'elle a un point isoélectrique voisin de 8,0.

6. Protéine recombinante selon l'une quelconque des revendications 1 à 4, caractérisée en ce qu'elle porte un groupement bloquant, de préférence de masse moléculaire voisine de 43 unités de masse atomique, sur la sérine amino-terminale.

7. Médicament, caractérisé en ce qu'il contient la protéine recombinante selon l'une quelconque des revendications 1 à 6.

8. Gène recombinant, caractérisé en ce qu'il comporte une séquence d'ADN codant pour la protéine de séquence ci-après :

```
Met Ser Ala Val Lys Ala Ala Arg Tyr Gly Lys Asp Asn Val Arg Val
1               5                   10                  15
Tyr Lys Val His Lys Asp Glu Lys Thr Gly Val Gln Thr Val Tyr Glu
            20                  25                  30
Met Thr Val Cys Val Leu Leu Glu Gly Glu Ile Glu Thr Ser Tyr Thr
        35                  40                  45
Lys Ala Asp Asn Ser Val Ile Val Ala Thr Asp Ser Ile Lys Asn Thr
    50                  55                  60
Ile Tyr Ile Thr Ala Lys Gln Asn Pro Val Thr Pro Pro Glu Leu Phe
65                  70                  75                  80
Gly Ser Ile Leu Gly Thr His Phe Ile Glu Lys Tyr Asn His Ile His
                85                  90                  95
Ala Ala His Val Asn Ile Val Cys His Arg Trp Thr Arg Met Asp Ile
```

```
                100                 105                 110
Asp Gly Lys Pro His Pro His Ser Phe Ile Arg Asp Ser Glu Glu Lys
        115                 120                 125
Arg Asn Val Gln Val Asp Val Val Glu Gly Lys Gly Ile Asp Ile Lys
    130                 135                 140
Ser Ser Leu Ser Gly Leu Thr Val Leu Lys Ser Thr Asn Ser Gln Phe
145                 150                 155                 160
Trp Gly Phe Leu Arg Asp Glu Tyr Thr Thr Leu Lys Glu Thr Trp Asp
                165                 170                 175
Arg Ile Leu Ser Thr Asp Val Asp Ala Thr Trp Gln Trp Lys Asn Phe
            180                 185                 190
Ser Gly Leu Gln Glu Val Arg Ser His Val Pro Lys Phe Asp Ala Thr
        195                 200                 205
Trp Ala Thr Ala Arg Glu Val Thr Leu Lys Thr Phe Ala Glu Asp Asn
    210                 215                 220
Ser Ala Ser Val Gln Ala Thr Met Tyr Lys Met Ala Glu Gln Ile Leu
225                 230                 235                 240
Ala Arg Gln Gln Leu Ile Glu Thr Val Glu Tyr Ser Leu Pro Asn Lys
                245                 250                 255
His Tyr Phe Glu Ile Asp Leu Ser Trp His Lys Gly Leu Gln Asn Thr
            260                 265                 270
Gly Lys Asn Ala Glu Val Phe Ala Pro Gln Ser Asp Pro Asn Gly Leu
        275                 280                 285
Ile Lys Cys Thr Val Gly Arg Ser Ser Leu Lys Ser Lys Leu
    290                 295                 300
```

9. Gène recombinant selon la revendication 8, caractérisé en ce qu'il permet une expression dans les micro-organismes procaryotes.

10. Gène recombinant selon la revendication 9, caractérisé en ce que ladite séquence d'ADN contient la séquence ci-après :

```
ATGTCTGCGG TAAAAGCAGC GCGCTACGGC AAGGACAATG TTCGCGTCTA CAAGGTTCAC    60
AAGGACGAGA AGACCGGTGT CCAGACGGTG TACGAGATGA CCGTCTGTGT GCTTCTGGAG   120
GGTGAGATTG AGACCTCTTA CACCAAGGCC GACAACAGCG TCATTGTCGC AACCGACTCC   180
ATTAAGAACA CCATTTACAT CACCGCCAAG CAGAACCCCG TTACTCCTCC CGAGCTGTTC   240
GGCTCCATCC TGGGCACACA CTTCATTGAG AAGTACAACC ACATCCATGC CGCTCACGTC   300
AACATTGTCT GCCACCGCTG GACCCGGATG GACATTGACG GCAAGCCACA CCCTCACTCC   360
TTCATCCGCG ACAGCGAGGA GAAGCGGAAT GTGCAGGTGG ACGTGGTCGA GGGCAAGGGC   420
ATCGATATCA AGTCGTCTCT GTCCGGCCTG ACCGTGCTGA AGAGCACCAA CTCGCAGTTC   480
TGGGGCTTCC TGCGTGACGA GTACACCACA CTTAAGGAGA CCTGGGACCG TATCCTGAGC   540
```

ACCGACGTCG ATGCCACTTG GCAGTGGAAG AATTTCAGTG GACTCCAGGA GGTCCGCTCG 600
CACGTGCCTA AGTTCGATGC TACCTGGGCC ACTGCTCGCG AGGTCACTCT GAAGACTTTT 660
GCTGAAGATA ACAGTGCCAG CGTGCAGGCC ACTATGTACA AGATGGCAGA GCAAATCCTG 720
GCGCGCCAGC AGCTGATCGA GACTGTCGAG TACTCGTTGC CTAACAAGCA CTATTTCGAA 780
ATCGACCTGA GCTGGCACAA GGGCCTCCAA AACACCGGCA AGAACGCCGA GGTCTTCGCT 840
CCTCAGTCGG ACCCCAACGG TCTGATCAAG TGTACCGTCG GCCGGTCCTC TCTGAAGTCT 900
AAATTG 906

11. Gène recombinant selon la revendication 8, caractérisé en ce qu'il permet une expression dans les cellules eucaryotes.

12. Gène recombinant selon la revendication 11, caractérisé en ce que ladite séquence d'ADN contient la séquence ci-après ;

ATGTCTGCTG TTAAGGCTGC TAGATACGGT AAGGACAACG TTAGAGTCTA CAAGGTTCAC 60
AAGGACGAGA AGACCGGTGT CCAGACGGTG TACGAGATGA CCGTCTGTGT GCTTCTGGAG 120
GGTGAGATTG AGACCTCTTA CACCAAGGCC GACAACAGCG TCATTGTCGC AACCGACTCC 180
ATTAAGAACA CCATTTACAT CACCGCCAAG CAGAACCCCG TTACTCCTCC CGAGCTGTTC 240
GGCTCCATCC TGGGCACACA CTTCATTGAG AAGTACAACC ACATCCATGC CGCTCACGTC 300
AACATTGTCT GCCACCGCTG GACCCGGATG GACATTGACG GCAAGCCACA CCCTCACTCC 360
TTCATCCGCG ACAGCGAGGA GAAGCGGAAT GTGCAGGTGG ACGTGGTCGA GGGCAAGGGC 420
ATCGATATCA AGTCGTCTCT GTCCGGCCTG ACCGTGCTGA AGAGCACCAA CTCGCAGTTC 480
TGGGGCTTCC TGCGTGACGA GTACACCACA CTTAAGGAGA CCTGGGACCG TATCCTGAGC 540
ACCGACGTCG ATGCCACTTG GCAGTGGAAG AATTTCAGTG GACTCCAGGA GGTCCGCTCG 600
CACGTGCCTA AGTTCGATGC TACCTGGGCC ACTGCTCGCG AGGTCACTCT GAAGACTTTT 660
GCTGAAGATA ACAGTGCCAG CGTGCAGGCC ACTATGTACA AGATGGCAGA GCAAATCCTG 720
GCGCGCCAGC AGCTGATCGA GACTGTCGAG TACTCGTTGC CTAACAAGCA CTATTTCGAA 780
ATCGACCTGA GCTGGCACAA GGGCCTCCAA AACACCGGCA AGAACGCCGA GGTCTTCGCT 840
CCTCAGTCGG ACCCCAACGG TCTGATCAAG TGTACCGTCG GCCGGTCCTC TCTGAAGTCT 900
AAATTG 906

13. Gène recombinant selon la revendication 8, caractérisé en ce qu'il permet une expression dans les cellules animales.

14. Gène recombinant selon la revendication 13, caractérisé en ce que ladite séquence d'ADN comprend la séquence ci-après ;

ATGTCCGCAG TAAAAGCAGC CCGCTACGGC AAGGACAATG TCCGCGTCTA CAAGGTTCAC 60
AAGGACGAGA AGACCGGTGT CCAGACGGTG TACGAGATGA CCGTCTGTGT GCTTCTGGAG 120
GGTGAGATTG AGACCTCTTA CACCAAGGCC GACAACAGCG TCATTGTCGC AACCGACTCC 180
ATTAAGAACA CCATTTACAT CACCGCCAAG CAGAACCCCG TTACTCCTCC CGAGCTGTTC 240

```
GGCTCCATCC TGGGCACACA CTTCATTGAG AAGTACAACC ACATCCATGC CGCTCACGTC   300
AACATTGTCT GCCACCGCTG GACCCGGATG GACATTGACG GCAAGCCACA CCCTCACTCC   360
TTCATCCGCG ACAGCGAGGA GAAGCGGAAT GTGCAGGTGG ACGTGGTCGA GGGCAAGGGC   420
ATCGATATCA AGTCGTCTCT GTCCGGCCTG ACCGTGCTGA AGAGCACCAA CTCGCAGTTC   480
TGGGGCTTCC TGCGTGACGA GTACACCACA CTTAAGGAGA CCTGGGACCG TATCCTGAGC   540
ACCGACGTCG ATGCCACTTG GCAGTGGAAG AATTTCAGTG GACTCCAGGA GGTCCGCTCG   600
CACGTGCCTA AGTTCGATGC TACCTGGGCC ACTGCTCGCG AGGTCACTCT GAAGACTTTT   660
GCTGAAGATA ACAGTGCCAG CGTGCAGGCC ACTATGTACA AGATGGCAGA GCAAATCCTG   720
GCGCGCCAGC AGCTGATCGA GACTGTCGAG TACTCGTTGC CTAACAAGCA CTATTTCGAA   780
ATCGACCTGA GCTGGCACAA GGGCCTCCAA AACACCGGCA AGAACGCCGA GGTCTTCGCT   840
CCTCAGTCGG ACCCCAACGG TCTGATCAAG TGTACCGTCG GCCGGTCCTC TCTGAAGTCT   900
AAATTG                                                              906
```

précédée d'une séquence 5' non traduite favorable à l'expression dans les cellules animales.

**15.** Gène recombinant selon la revendication 14, caractérisé en ce que la séquence 5' non traduite favorable à l'expression dans les cellules animales comprend la séquence : AGCTTGCCGCCACT située immédiatement en amont de la séquence explicitée dans la revendication 14.

**16.** Vecteur d'expression, caractérisé en ce qu'il porte, avec les moyens nécessaires à son expression, un gène recombinant selon l'une quelconque des revendications 8 à 15.

**17.** Vecteur d'expression selon la revendication 16, caractérisé en ce qu'il porte au moins un marqueur de sélection.

**18.** Vecteur d'expression selon la revendication 17, caractérisé en ce qu'il a les caractéristiques de l'un des plasmides pEMR469, pEMR473 décrits dans les figures 11 et 12 ou de pEMR515 dérivé de pEMR473 par délétion du gène URA3 et du fragment XbaI-NheI du 2 micron.

**19.** Micro-organismes procaryotes, caractérisés en ce qu'ils sont transformés par un vecteur d'expression selon la revendication 16, portant un gène recombinant selon la revendication 9.

**20.** Cellules eucaryotes, caractérisées en ce qu'elles sont transformées par l'un des vecteurs d'expression selon l'une quelconque des revendications 16 à 18 portant le gène recombinant selon la revendication 11.

**21.** Souche de Saccharomyces cerevisiae, caractérisée en ce qu'elle est transformée par l'un des vecteurs d'expression selon l'une quelconque des revendications 16 à 18.

**22.** Souche selon la revendication 21, caractérisée en ce qu'elle porte une mutation sur au moins l'un des gènes responsables de la synthèse de la leucine ou de l'uracile.

**23.** Souche selon la revendication 22, caractérisée en ce qu'elle porte une mutation sur au moins l'un des gènes LEU2 et URA3.

**24.** Procédé pour obtenir l'urate oxydase recombinante selon la revendication 1, caractérisé en ce qu'il comprend les étapes ci-après :

1/ mise en culture d'une souche selon l'une quelconque des revendications 21 à 23 ;
2/ lyse de cellules ;
3/ isolement et purification de l'urate oxydase recombinante contenue dans le lysat.

**25.** Cellules animales, caractérisées en ce qu'elles contiennent, avec les moyens nécessaires à son expression, un gène recombinant selon la revendication 13.

**26.** Cellules animales, caractérisées en ce qu'elles contiennent un vecteur d'expression selon la revendication 16, portant un gène recombinant selon la revendication 14.

## Patentansprüche

**1.** Rekombinantes Protein, dadurch gekennzeichnet, daß es eine spezifische Uratoxidase-Aktivität von zumindest 16 U/mg hat, und daß es die nachstehende Sequenz aufweist:

```
Ser Ala Val Lys Ala Ala Arg Tyr Gly Lys Asp Asn Val Arg Val Tyr
1               5                   10                  15
Lys Val His Lys Asp Glu Lys Thr Gly Val Gln Thr Val Tyr Glu Met
            20                  25                  30
Thr Val Cys Val Leu Leu Glu Gly Glu Ile Glu Thr Ser Tyr Thr Lys
        35                  40                  45
Ala Asp Asn Ser Val Ile Val Ala Thr Asp Ser Ile Lys Asn Thr Ile
    50                  55                  60
Tyr Ile Thr Ala Lys Gln Asn Pro Val Thr Pro Pro Glu Leu Phe Gly
65                  70                  75                  80
Ser Ile Leu Gly Thr His Phe Ile Glu Lys Tyr Asn His Ile His Ala
                85                  90                  95
Ala His Val Asn Ile Val Cys His Arg Trp Thr Arg Met Asp Ile Asp
            100                 105                 110
Gly Lys Pro His Pro His Ser Phe Ile Arg Asp Ser Glu Glu Lys Arg
        115                 120                 125
Asn Val Gln Val Asp Val Val Glu Gly Lys Gly Ile Asp Ile Lys Ser
    130                 135                 140
Ser Leu Ser Gly Leu Thr Val Leu Lys Ser Thr Asn Ser Gln Phe Trp
145                 150                 155                 160
Gly Phe Leu Arg Asp Glu Tyr Thr Thr Leu Lys Glu Thr Trp Asp Arg
                165                 170                 175
Ile Leu Ser Thr Asp Val Asp Ala Thr Trp Gln Trp Lys Asn Phe Ser
            180                 185                 190
Gly Leu Gln Glu Val Arg Ser His Val Pro Lys Phe Asp Ala Thr Trp
        195                 200                 205
Ala Thr Ala Arg Glu Val Thr Leu Lys Thr Phe Ala Glu Asp Asn Ser
    210                 215                 220
Ala Ser Val Gln Ala Thr Met Tyr Lys Met Ala Glu Gln Ile Leu Ala
225                 230                 235                 240
Arg Gln Gln Leu Ile Glu Thr Val Glu Tyr Ser Leu Pro Asn Lys His
                245                 250                 255
Tyr Phe Glu Ile Asp Leu Ser Trp His Lys Gly Leu Gln Asn Thr Gly
            260                 265                 270
Lys Asn Ala Glu Val Phe Ala Pro Gln Ser Asp Pro Asn Gly Leu Ile
        275                 280                 285
Lys Cys Thr Val Gly Arg Ser Ser Leu Lys Ser Lys Leu
    290                 295                 300
```

der gegebenenfalls ein Methionin vorausgeht.

2. Rekombinantes Protein nach Anspruch 1, dadurch gekennzeichnet, daß es eine spezifische Uratoxidase-Aktivität von ungefähr 30 U/mg hat.

3. Rekombinantes Protein nach einem der Ansprüche 1 und 2, dadurch gekennzeichnet, daß es gemäß zweidimensionaler Gelanalyse einen Molmassen-Spot von ungefähr 33,5 kDA zeigt, welcher zumindest 90 % der Proteinmasse darstellt.

4. Rekombinantes Protein nach einem der Ansprüche 1 bis 3, dadurch gekennzeichnet, daß es einen Reinheitsgrad, bestimmt durch Flüssigchromatographie auf einer $C_8$-pfropfte Kieselerdesäule, von mehr als 80 % aufweist.

5. Rekombinantes Protein nach einem der Ansprüche 1 bis 4, dadurch gekennzeichnet, daß es einen isoelektrischen Punkt von etwa 8,0 aufweist.

6. Rekombinantes Protein nach einem der Ansprüche 1 bis 4, dadurch gekennzeichnet, daß es eine Blockierungsgruppe, vorzugsweise mit einer Molmasse von etwa 43 Atommasseeinheiten, am Amino-terminalen Serin trägt.

7. Medikament, dadurch gekennzeichnet, daß es das rekombinante Protein nach einem der Ansprüche 1 bis 6 enthält.

8. Rekombinantes Gen, dadurch gekennzeichnet, daß es eine DNS-Sequenz umfaßt, die für das Protein mit der nachstehenden Sequenz codiert:

```
Met Ser Ala Val Lys Ala Ala Arg Tyr Gly Lys Asp Asn Val Arg Val
1               5                   10                  15
Tyr Lys Val His Lys Asp Glu Lys Thr Gly Val Gln Thr Val Tyr Glu
            20                  25                  30
Met Thr Val Cys Val Leu Leu Glu Gly Glu Ile Glu Thr Ser Tyr Thr
        35                  40                  45
Lys Ala Asp Asn Ser Val Ile Val Ala Thr Asp Ser Ile Lys Asn Thr
    50                  55                  60
Ile Tyr Ile Thr Ala Lys Gln Asn Pro Val Thr Pro Pro Glu Leu Phe
65                  70                  75                  80
Gly Ser Ile Leu Gly Thr His Phe Ile Glu Lys Tyr Asn His Ile His
                85                  90                  95
Ala Ala His Val Asn Ile Val Cys His Arg Trp Thr Arg Met Asp Ile
            100                 105                 110
Asp Gly Lys Pro His Pro His Ser Phe Ile Arg Asp Ser Glu Glu Lys
        115                 120                 125
Arg Asn Val Gln Val Asp Val Val Glu Gly Lys Gly Ile Asp Ile Lys
    130                 135                 140
```

```
Ser Ser Leu Ser Gly Leu Thr Val Leu Lys Ser Thr Asn Ser Gln Phe
145                 150                 155                 160
Trp Gly Phe Leu Arg Asp Glu Tyr Thr Thr Leu Lys Glu Thr Trp Asp
                165                 170                 175
Arg Ile Leu Ser Thr Asp Val Asp Ala Thr Trp Gln Trp Lys Asn Phe
            180                 185                 190
Ser Gly Leu Gln Glu Val Arg Ser His Val Pro Lys Phe Asp Ala Thr
        195                 200                 205
Trp Ala Thr Ala Arg Glu Val Thr Leu Lys Thr Phe Ala Glu Asp Asn
    210                 215                 220
Ser Ala Ser Val Gln Ala Thr Met Tyr Lys Met Ala Glu Gln Ile Leu
225                 230                 235                 240
Ala Arg Gln Gln Leu Ile Glu Thr Val Glu Tyr Ser Leu Pro Asn Lys
                245                 250                 255
His Tyr Phe Glu Ile Asp Leu Ser Trp His Lys Gly Leu Gln Asn Thr
            260                 265                 270
Gly Lys Asn Ala Glu Val Phe Ala Pro Gln Ser Asp Pro Asn Gly Leu
        275                 280                 285
Ile Lys Cys Thr Val Gly Arg Ser Ser Leu Lys Ser Lys Leu
    290                 295                 300
```

**9.** Rekombinantes Gen nach Anspruch 8, dadurch gekennzeichnet, daß es eine Expression in prokaryotischen Mikroorganismen ermöglicht.

**10.** Rekombinantes Gen nach Anspruch 9, dadurch gekennzeichnet, daß die DNS-Sequenz die nachstehende Sequenz enthält:

```
ATGTCTGCGG TAAAAGCAGC GCGCTACGGC AAGGACAATG TTCGCGTCTA CAAGGTTCAC    60
AAGGACGAGA AGACCGGTGT CCAGACGGTG TACGAGATGA CCGTCTGTGT GCTTCTGGAG   120
GGTGAGATTG AGACCTCTTA CACCAAGGCC GACAACAGCG TCATTGTCGC AACCGACTCC   180
ATTAAGAACA CCATTTACAT CACCGCCAAG CAGAACCCCG TTACTCCTCC CGAGCTGTTC   240
GGCTCCATCC TGGGCACACA CTTCATTGAG AAGTACAACC ACATCCATGC CGCTCACGTC   300
AACATTGTCT GCCACCGCTG GACCCGGATG GACATTGACG GCAAGCCACA CCCTCACTCC   360
TTCATCCGCG ACAGCGAGGA GAAGCGGAAT GTGCAGGTGG ACGTGGTCGA GGGCAAGGGC   420
ATCGATATCA AGTCGTCTCT GTCCGGCCTG ACCGTGCTGA AGAGCACCAA CTCGCAGTTC   480
TGGGGCTTCC TGCGTGACGA GTACACCACA CTTAAGGAGA CCTGGGACCG TATCCTGAGC   540
ACCGACGTCG ATGCCACTTG GCAGTGGAAG AATTTCAGTG GACTCCAGGA GGTCCGCTCG   600
CACGTGCCTA AGTTCGATGC TACCTGGGCC ACTGCTCGCG AGGTCACTCT GAAGACTTTT   660
GCTGAAGATA ACAGTGCCAG CGTGCAGGCC ACTATGTACA AGATGGCAGA GCAAATCCTG   720
GCGCGCCAGC AGCTGATCGA GACTGTCGAG TACTCGTTGC CTAACAAGCA CTATTTCGAA   780
ATCGACCTGA GCTGGCACAA GGGCCTCCAA AACACCGGCA AGAACGCCGA GGTCTTCGCT   840
CCTCAGTCGG ACCCCAACGG TCTGATCAAG TGTACCGTCG GCCGGTCCTC TCTGAAGTCT   900
AAATTG                                                              906
```

**11.** Rekombinantes Gen nach Anspruch 8, dadurch gekennzeichnet, daß es eine Expression in eukaryotischen Zellen ermöglicht.

**12.** Rekombinantes Gen nach Anspruch 11, dadurch gekennzeichnet, daß die DNS-Sequenz die nachstehende Sequenz enthält:

```
ATGTCTGCTG TTAAGGCTGC TAGATACGGT AAGGACAACG TTAGAGTCTA CAAGGTTCAC   60
AAGGACGAGA AGACCGGTGT CCAGACGGTG TACGAGATGA CCGTCTGTGT GCTTCTGGAG  120
GGTGAGATTG AGACCTCTTA CACCAAGGCC GACAACAGCG TCATTGTCGC AACCGACTCC  180
ATTAAGAACA CCATTTACAT CACCGCCAAG CAGAACCCCG TTACTCCTCC CGAGCTGTTC  240
GGCTCCATCC TGGGCACACA CTTCATTGAG AAGTACAACC ACATCCATGC CGCTCACGTC  300
AACATTGTCT GCCACCGCTG GACCCGGATG GACATTGACG GCAAGCCACA CCCTCACTCC  360
TTCATCCGCG ACAGCGAGGA GAAGCGGAAT GTGCAGGTGG ACGTGGTCGA GGGCAAGGGC  420
ATCGATATCA AGTCGTCTCT GTCCGGCCTG ACCGTGCTGA AGAGCACCAA CTCGCAGTTC  480
TGGGGCTTCC TGCGTGACGA GTACACCACA CTTAAGGAGA CCTGGGACCG TATCCTGAGC  540
ACCGACGTCG ATGCCACTTG GCAGTGGAAG AATTTCAGTG GACTCCAGGA GGTCCGCTCG  600
CACGTGCCTA AGTTCGATGC TACCTGGGCC ACTGCTCGCG AGGTCACTCT GAAGACTTTT  660
GCTGAAGATA ACAGTGCCAG CGTGCAGGCC ACTATGTACA AGATGGCAGA GCAAATCCTG  720
GCGCGCCAGC AGCTGATCGA GACTGTCGAG TACTCGTTGC CTAACAAGCA CTATTTCGAA  780
ATCGACCTGA GCTGGCACAA GGGCCTCCAA AACACCGGCA AGAACGCCGA GGTCTTCGCT  840
CCTCAGTCGG ACCCCAACGG TCTGATCAAG TGTACCGTCG GCCGGTCCTC TCTGAAGTCT  900
AAATTG                                                             906
```

**13.** Rekombinantes Gen nach Anspruch 8, dadurch gekennzeichnet, daß es eine Expression in tierischen Zellen ermöglicht.

**14.** Rekombinantes Gen nach Anspruch 13, dadurch gekennzeichnet, daß die DNS-Sequenz die nachstehende Sequenz enthält:

```
ATGTCCGCAG TAAAAGCAGC CCGCTACGGC AAGGACAATG TCCGCGTCTA CAAGGTTCAC   60
AAGGACGAGA AGACCGGTGT CCAGACGGTG TACGAGATGA CCGTCTGTGT GCTTCTGGAG  120
GGTGAGATTG AGACCTCTTA CACCAAGGCC GACAACAGCG TCATTGTCGC AACCGACTCC  180
ATTAAGAACA CCATTTACAT CACCGCCAAG CAGAACCCCG TTACTCCTCC CGAGCTGTTC  240
GGCTCCATCC TGGGCACACA CTTCATTGAG AAGTACAACC ACATCCATGC CGCTCACGTC  300
AACATTGTCT GCCACCGCTG GACCCGGATG GACATTGACG GCAAGCCACA CCCTCACTCC  360
TTCATCCGCG ACAGCGAGGA GAAGCGGAAT GTGCAGGTGG ACGTGGTCGA GGGCAAGGGC  420
ATCGATATCA AGTCGTCTCT GTCCGGCCTG ACCGTGCTGA AGAGCACCAA CTCGCAGTTC  480
TGGGGCTTCC TGCGTGACGA GTACACCACA CTTAAGGAGA CCTGGGACCG TATCCTGAGC  540
ACCGACGTCG ATGCCACTTG GCAGTGGAAG AATTTCAGTG GACTCCAGGA GGTCCGCTCG  600
CACGTGCCTA AGTTCGATGC TACCTGGGCC ACTGCTCGCG AGGTCACTCT GAAGACTTTT  660
GCTGAAGATA ACAGTGCCAG CGTGCAGGCC ACTATGTACA AGATGGCAGA GCAAATCCTG  720
GCGCGCCAGC AGCTGATCGA GACTGTCGAG TACTCGTTGC CTAACAAGCA CTATTTCGAA  780
ATCGACCTGA GCTGGCACAA GGGCCTCCAA AACACCGGCA AGAACGCCGA GGTCTTCGCT  840
CCTCAGTCGG ACCCCAACGG TCTGATCAAG TGTACCGTCG GCCGGTCCTC TCTGAAGTCT  900
AAATTG                                                             906
```

der eine 5'-nicht-translatierte Sequenz vorausgeht, die für die Expression in tierischen Zellen vorteilhaft ist.

**15.** Rekombinantes Gen nach Anspruch 14, dadurch gekennzeichnet, daß die 5'-nicht-translatierte Sequenz, die für die Expression in tierischen Zellen vorteilhaft ist, die Sequenz umfaßt: AGCTTGCCGCCACT, die unmittelbar

stromaufwärts von der in Anspruch 14 angegebenen Sequenz angeordnet ist.

**16.** Expressionvektor, dadurch gekennzeichnet, daß er, mit den für seine Expression notwendigen Mitteln, ein rekombinantes Gen nach einem der Ansprüche 8 bis 15 trägt.

**17.** Expressionsvektor nach Anspruch 16, dadurch gekennzeichnet, daß er zumindest einen Selektionsmarker trägt.

**18.** Expressionsvektor nach Anspruch 17, dadurch gekennzeichnet, daß er die Eigenschaften eines der Plasmide pEMR469, pEMR473, die in Fig.11 und 12 beschrieben sind, oder pEMR515, das von pEMR473 durch Deletion des Gens URA3 und des 2 µm XbaI-NheI-Fragments abgeleitet wird, hat.

**19.** Prokaryotische Mikroorganismen, dadurch gekennzeichnet, daß sie mit einem Expressionvektor nach Anspruch 16, der ein rekombinantes Gen nach Anspruch 9 trägt, transformiert sind.

**20.** Eukaryotische Zellen, dadurch gekennzeichnet, daß sie mit einem Expressionsvektor nach einem der Ansprüche 16 bis 18, der ein rekombinantes Gen nach Anspruch 11 trägt, transformiert sind.

**21.** Saccharomyces cerevisiae-Stamm, dadurch gekennzeichnet, daß er mit einem Expressionsvektor nach einem der Ansprüche 16 bis 18 transformiert ist.

**22.** Stamm nach Anspruch 21, dadurch gekennzeichnet, daß er eine Mutation zumindest an einem der für die Synthese von Leucin oder Uracil verantwortlichen Gene trägt.

**23.** Stamm nach Anspruch 22, dadurch gekennzeichnet, daß er eine Mutation zumindest an einem der Gene LEU2 und URA3 trägt.

**24.** Verfahren zum Erhalten rekombinanter Uratoxydase nach Anspruch 1, dadurch gekennzeichnet, daß es die nachstehenden Schritte umfaßt:

1) Kultivieren eines Stamms nach einem der Ansprüche 21 bis 23;
2) Lyse der Zellen;
3) Isolierung und Reinigung der rekombinanten Uratoxydase, die im Lysat enthalten ist.

**25.** Tierische Zellen, dadurch gekennzeichnet, daß sie, mit den für seine Expression notwendigen Mitteln, ein rekombinantes Gen nach Anspruch 13 enthalten.

**26.** Tierische Zellen, dadurch gekennzeichnet, daß sie einen Expressionsvektor nach Anspruch 16, der ein rekombinantes Gen nach Anspruch 14 trägt, enthalten.

## Claims

**1.** Recombinant protein, characterized in that it has a specific urate oxidase activity of at least 16 U/mg and in that it has the following sequence:

Ser Ala Val Lys Ala Ala Arg Tyr Gly Lys Asp Asn Val Arg Val Tyr
1 5 10 15
Lys Val His Lys Asp Glu Lys Thr Gly Val Gln Thr Val Tyr Glu Met
20 25 30
Thr Val Cys Val Leu Leu Glu Gly Glu Ile Glu Thr Ser Tyr Thr Lys
35 40 45
Ala Asp Asn Ser Val Ile Val Ala Thr Asp Ser Ile Lys Asn Thr Ile
50 55 60
Tyr Ile Thr Ala Lys Gln Asn Pro Val Thr Pro Pro Glu Leu Phe Gly
65 70 75 80
Ser Ile Leu Gly Thr His Phe Ile Glu Lys Tyr Asn His Ile His Ala
85 90 95
Ala His Val Asn Ile Val Cys His Arg Trp Thr Arg Met Asp Ile Asp
100 105 110
Gly Lys Pro His Pro His Ser Phe Ile Arg Asp Ser Glu Glu Lys Arg
115 120 125
Asn Val Gln Val Asp Val Val Glu Gly Lys Gly Ile Asp Ile Lys Ser
130 135 140
Ser Leu Ser Gly Leu Thr Val Leu Lys Ser Thr Asn Ser Gln Phe Trp
145 150 155 160
Gly Phe Leu Arg Asp Glu Tyr Thr Thr Leu Lys Glu Thr Trp Asp Arg
165 170 175
Ile Leu Ser Thr Asp Val Asp Ala Thr Trp Gln Trp Lys Asn Phe Ser
180 185 190
Gly Leu Gln Glu Val Arg Ser His Val Pro Lys Phe Asp Ala Thr Trp
195 200 205
Ala Thr Ala Arg Glu Val Thr Leu Lys Thr Phe Ala Glu Asp Asn Ser
210 215 220
Ala Ser Val Gln Ala Thr Met Tyr Lys Met Ala Glu Gln Ile Leu Ala
225 230 235 240
Arg Gln Gln Leu Ile Glu Thr Val Glu Tyr Ser Leu Pro Asn Lys His
245 250 255
Tyr Phe Glu Ile Asp Leu Ser Trp His Lys Gly Leu Gln Asn Thr Gly
260 265 270
Lys Asn Ala Glu Val Phe Ala Pro Gln Ser Asp Pro Asn Gly Leu Ile
275 280 285
Lys Cys Thr Val Gly Arg Ser Ser Leu Lys Ser Lys Leu
290 295 300

preceded, optionally, by a methionine.

2. Recombinant protein according to claim 1, characterized in that it has a specific urate oxidase activity of approximately 30 U/mg.

3. Recombinant protein according to one of claims 1 and 2, characterized in that it has, by bidimensional gel analysis, a spot of molecular mass of approximately 33.5 kDa which represents at least 90% of the proteic weight.

**4.** Recombinant protein according to any one of claims 1 to 3, characterized in that its purity degree, determined by liquid chromatography on a C8 grafted silica column, is higher than 80%.

**5.** Recombinant protein according to any one of claims 1 to 4, characterized in that it has an isoelectric point around 8.0.

**6.** Recombinant protein according to any one of claims 1 to 4, characterized in that it carries a blocking group, preferably of molecular mass around 43 units of atomic mass, on the amino-terminal serine.

**7.** Medicament, characterized in that it contains the recombinant protein according to any one of claims 1 to 6.

**8.** Recombinant gene, characterized in that it comprises a DNA sequence coding for the protein which has the following sequence:

```
Met Ser Ala Val Lys Ala Ala Arg Tyr Gly Lys Asp Asn Val Arg Val
1               5                   10                  15
Tyr Lys Val His Lys Asp Glu Lys Thr Gly Val Gln Thr Val Tyr Glu
            20                  25                  30
Met Thr Val Cys Val Leu Leu Glu Gly Glu Ile Glu Thr Ser Tyr Thr
        35                  40                  45
Lys Ala Asp Asn Ser Val Ile Val Ala Thr Asp Ser Ile Lys Asn Thr
    50                  55                  60
Ile Tyr Ile Thr Ala Lys Gln Asn Pro Val Thr Pro Pro Glu Leu Phe
65                  70                  75                  80
Gly Ser Ile Leu Gly Thr His Phe Ile Glu Lys Tyr Asn His Ile His
                85                  90                  95
Ala Ala His Val Asn Ile Val Cys His Arg Trp Thr Arg Met Asp Ile
```

```
                    100                     105                     110
Asp Gly Lys Pro His Pro His Ser Phe Ile Arg Asp Ser Glu Glu Lys
            115                     120                     125
Arg Asn Val Gln Val Asp Val Val Glu Gly Lys Gly Ile Asp Ile Lys
        130                     135                     140
Ser Ser Leu Ser Gly Leu Thr Val Leu Lys Ser Thr Asn Ser Gln Phe
145                     150                     155                     160
Trp Gly Phe Leu Arg Asp Glu Tyr Thr Thr Leu Lys Glu Thr Trp Asp
                165                     170                     175
Arg Ile Leu Ser Thr Asp Val Asp Ala Thr Trp Gln Trp Lys Asn Phe
            180                     185                     190
Ser Gly Leu Gln Glu Val Arg Ser His Val Pro Lys Phe Asp Ala Thr
        195                     200                     205
Trp Ala Thr Ala Arg Glu Val Thr Leu Lys Thr Phe Ala Glu Asp Asn
    210                     215                     220
Ser Ala Ser Val Gln Ala Thr Met Tyr Lys Met Ala Glu Gln Ile Leu
225                     230                     235                     240
Ala Arg Gln Gln Leu Ile Glu Thr Val Glu Tyr Ser Leu Pro Asn Lys
                245                     250                     255
His Tyr Phe Glu Ile Asp Leu Ser Trp His Lys Gly Leu Gln Asn Thr
            260                     265                     270
Gly Lys Asn Ala Glu Val Phe Ala Pro Gln Ser Asp Pro Asn Gly Leu
        275                     280                     285
Ile Lys Cys Thr Val Gly Arg Ser Ser Leu Lys Ser Lys Leu
    290                     295                     300
```

**9.** Recombinant gene according to claim 8, characterized in that it permits expression in prokaryotic microorganisms.

**10.** Recombinant gene according to claim 9, characterized in that said DNA sequence contains the following sequence:

```
ATGTCTGCGG TAAAAGCAGC GCGCTACGGC AAGGACAATG TTCGCGTCTA CAAGGTTCAC    60
AAGGACGAGA AGACCGGTGT CCAGACGGTG TACGAGATGA CCGTCTGTGT GCTTCTGGAG   120
GGTGAGATTG AGACCTCTTA CACCAAGGCC GACAACAGCG TCATTGTCGC AACCGACTCC   180
ATTAAGAACA CCATTTACAT CACCGCCAAG CAGAACCCCG TTACTCCTCC CGAGCTGTTC   240
GGCTCCATCC TGGGCACACA CTTCATTGAG AAGTACAACC ACATCCATGC CGCTCACGTC   300
AACATTGTCT GCCACCGCTG GACCCGGATG GACATTGACG GCAAGCCACA CCCTCACTCC   360
TTCATCCGCG ACAGCGAGGA GAAGCGGAAT GTGCAGGTGG ACGTGGTCGA GGGCAAGGGC   420
ATCGATATCA AGTCGTCTCT GTCCGGCCTG ACCGTGCTGA AGAGCACCAA CTCGCAGTTC   480
TGGGGCTTCC TGCGTGACGA GTACACCACA CTTAAGGAGA CCTGGGACCG TATCCTGAGC   540
```

ACCGACGTCG ATGCCACTTG GCAGTGGAAG AATTTCAGTG GACTCCAGGA GGTCCGCTCG 600
CACGTGCCTA AGTTCGATGC TACCTGGGCC ACTGCTCGCG AGGTCACTCT GAAGACTTTT 660
GCTGAAGATA ACAGTGCCAG CGTGCAGGCC ACTATGTACA AGATGGCAGA GCAAATCCTG 720
GCGCGCCAGC AGCTGATCGA GACTGTCGAG TACTCGTTGC CTAACAAGCA CTATTTCGAA 780
ATCGACCTGA GCTGGCACAA GGGCCTCCAA AACACCGGCA AGAACGCCGA GGTCTTCGCT 840
CCTCAGTCGG ACCCCAACGG TCTGATCAAG TGTACCGTCG GCCGGTCCTC TCTGAAGTCT 900
AAATTG 906

**11.** Recombinant gene according to claim 8, characterized in that it permits expression in eukaryotic cells.

**12.** Recombinant gene according to claim 11, characterized in that said DNA sequence contains the following sequence:

ATGTCTGCTG TTAAGGCTGC TAGATACGGT AAGGACAACG TTAGAGTCTA CAAGGTTCAC 60
AAGGACGAGA AGACCGGTGT CCAGACGGTG TACGAGATGA CCGTCTGTGT GCTTCTGGAG 120
GGTGAGATTG AGACCTCTTA CACCAAGGCC GACAACAGCG TCATTGTCGC AACCGACTCC 180
ATTAAGAACA CCATTTACAT CACCGCCAAG CAGAACCCCG TTACTCCTCC CGAGCTGTTC 240
GGCTCCATCC TGGGCACACA CTTCATTGAG AAGTACAACC ACATCCATGC CGCTCACGTC 300
AACATTGTCT GCCACCGCTG GACCCGGATG GACATTGACG GCAAGCCACA CCCTCACTCC 360
TTCATCCGCG ACAGCGAGGA GAAGCGGAAT GTGCAGGTGG ACGTGGTCGA GGGCAAGGGC 420
ATCGATATCA AGTCGTCTCT GTCCGGCCTG ACCGTGCTGA AGAGCACCAA CTCGCAGTTC 480
TGGGGCTTCC TGCGTGACGA GTACACCACA CTTAAGGAGA CCTGGGACCG TATCCTGAGC 540
ACCGACGTCG ATGCCACTTG GCAGTGGAAG AATTTCAGTG GACTCCAGGA GGTCCGCTCG 600
CACGTGCCTA AGTTCGATGC TACCTGGGCC ACTGCTCGCG AGGTCACTCT GAAGACTTTT 660
GCTGAAGATA ACAGTGCCAG CGTGCAGGCC ACTATGTACA AGATGGCAGA GCAAATCCTG 720
GCGCGCCAGC AGCTGATCGA GACTGTCGAG TACTCGTTGC CTAACAAGCA CTATTTCGAA 780
ATCGACCTGA GCTGGCACAA GGGCCTCCAA AACACCGGCA AGAACGCCGA GGTCTTCGCT 840
CCTCAGTCGG ACCCCAACGG TCTGATCAAG TGTACCGTCG GCCGGTCCTC TCTGAAGTCT 900
AAATTG 906

**13.** Recombinant gene according to claim 8, characterized in that it permits expression in animal cells.

**14.** Recombinant gene according to claim 13, characterized in that said DNA sequence comprises the following sequence:

ATGTCCGCAG TAAAAGCAGC CCGCTACGGC AAGGACAATG TCCGCGTCTA CAAGGTTCAC 60
AAGGACGAGA AGACCGGTGT CCAGACGGTG TACGAGATGA CCGTCTGTGT GCTTCTGGAG 120
GGTGAGATTG AGACCTCTTA CACCAAGGCC GACAACAGCG TCATTGTCGC AACCGACTCC 180
ATTAAGAACA CCATTTACAT CACCGCCAAG CAGAACCCCG TTACTCCTCC CGAGCTGTTC 240

```
GGCTCCATCC TGGGCACACA CTTCATTGAG AAGTACAACC ACATCCATGC CGCTCACGTC   300
AACATTGTCT GCCACCGCTG GACCCGGATG GACATTGACG GCAAGCCACA CCCTCACTCC   360
TTCATCCGCG ACAGCGAGGA GAAGCGGAAT GTGCAGGTGG ACGTGGTCGA GGGCAAGGGC   420
ATCGATATCA AGTCGTCTCT GTCCGGCCTG ACCGTGCTGA AGAGCACCAA CTCGCAGTTC   480
TGGGGCTTCC TGCGTGACGA GTACACCACA CTTAAGGAGA CCTGGGACCG TATCCTGAGC   540
ACCGACGTCG ATGCCACTTG GCAGTGGAAG AATTTCAGTG GACTCCAGGA GGTCCGCTCG   600
CACGTGCCTA AGTTCGATGC TACCTGGGCC ACTGCTCGCG AGGTCACTCT GAAGACTTTT   660
GCTGAAGATA ACAGTGCCAG CGTGCAGGCC ACTATGTACA AGATGGCAGA GCAAATCCTG   720
GCGCGCCAGC AGCTGATCGA GACTGTCGAG TACTCGTTGC CTAACAAGCA CTATTTCGAA   780
ATCGACCTGA GCTGGCACAA GGGCCTCCAA AACACCGGCA AGAACGCCGA GGTCTTCGCT   840
CCTCAGTCGG ACCCCAACGG TCTGATCAAG TGTACCGTCG GCCGGTCCTC TCTGAAGTCT   900
AAATTG                                                              906
```

preceded by a 5'-non-translated sequence favoring expression in animal cells.

**15.** Recombinant gene according to claim 14, characterized in that the 5'-non-translated sequence favoring expression in animal cells comprises the sequence AGCTTGCCGCCACT, located immediately upstream from the sequence described in claim 14.

**16.** Expression vector, characterized in that it carries a recombinant gene according to any one of claims 8 to 15 with the means necessary for its expression.

**17.** Expression vector according to claim 16, characterized in that it carries at least one selection marker.

**18.** Expression vector according to claim 17, characterized in that it has the characteristics of one of plasmids pEMR469, pEMR473, described in Figures 11 and 12, or pEMR515 derived from pEMR473 by deletion of the gene URA3 and of the fragment XbaI-NheI of the 2 micron.

**19.** Prokaryotic microorganisms, characterized in that they are transformed by an expression vector according to claim 16, carrying a recombinant gene according to claim 9.

**20.** Eukaryotic cells, characterized in that they are transformed by one of the expression vectors according to any one of claims 16 to 18, carrying the recombinant gene according to claim 11.

**21.** Strain of <u>Saccharomyces cerevisiae</u>, characterized in that it is transformed by one of the expression vectors according to any one of claims 16 to 18.

**22.** Strain according to claim 21, characterized in that it carries a mutation on at least one of the genes responsible for the synthesis of leucine or uracil.

**23.** Strain according to claim 22, characterized in that it carries a mutation on at least one of the LEU2 and URA3 genes.

**24.** Method for obtaining the recombinant urate oxidase according to claim 1, characterized in that it comprises the following steps:

1/ culturing of a strain according to any one of claims 21 to 23;
2/ lysis of cells;
3/ isolation and purification of the recombinant urate oxidase contained in the lyzate.

**25.** Animal cells, characterized in that they contain a recombinant gene according to claim 13 with the means necessary for its expression.

**26.** Animal cells, characterized in that they contain an expression vector according to claim 16, carrying a recombinant

gene according to claim 14.

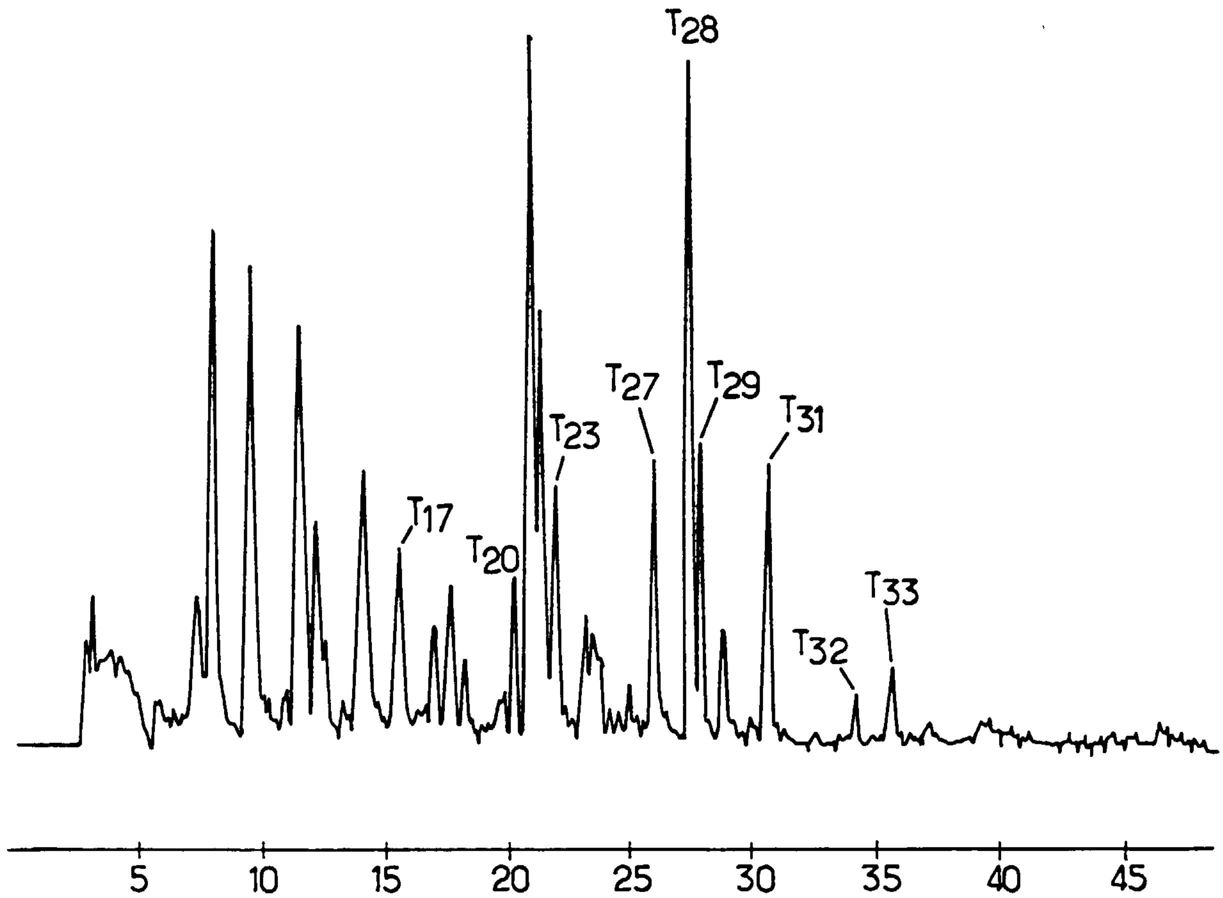


FIG.1

Profil d'élution par mesure de la densité optique à 218 nm
du produit de la digestion tryptique de l'urat oxydase

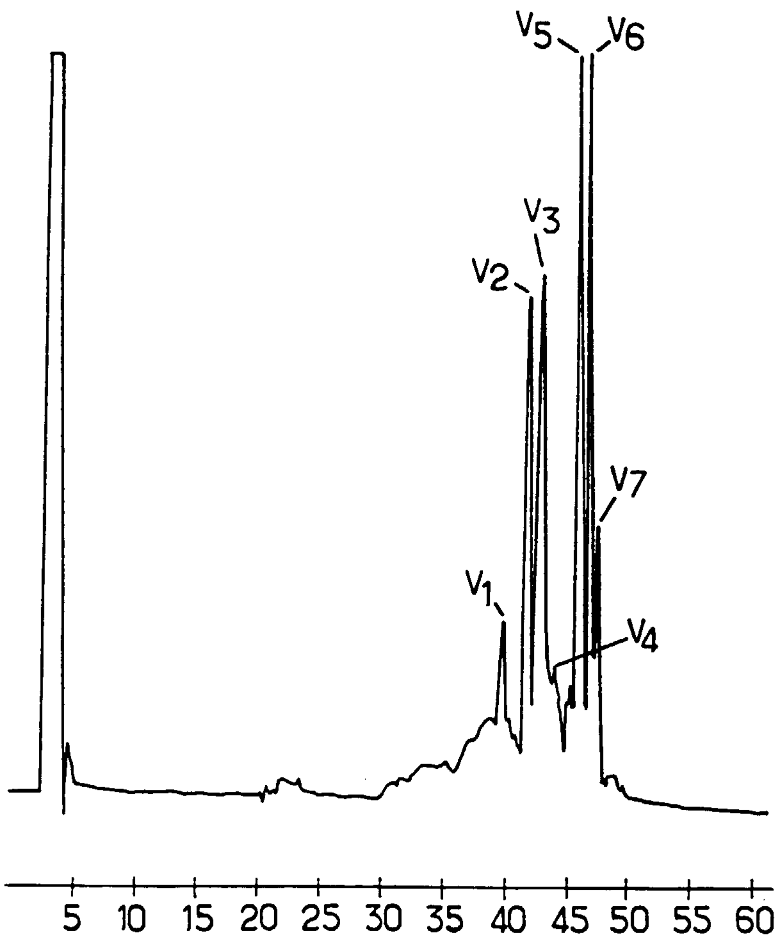


FIG. 2

Profil d'élution par mesure de la densité optique à 218 nm
du produit de la digestion par la protéase V8 de l'urate oxydase

1 AAACCCTCACTGCCTCTCTCATTCCTTCCG GTGCCCCCGATCCTCAATCCAACTTGTACA 60

61 TACTTCTCCCAACTCTCTGCTATATCCTTC ATATTCCCATACTACAAGATGTCCGCAGTA 120

121 AAAGCAGCCCGCTACGGCAAGGACAATGTC CGCGTCTACAAGGTTCACAAGGACGAGAAG 180

181 ACCGGTGTCCAGACGGTGTACGAGATGACC GTCTGTGTGCTTCTGGAGGGTGAGATTGAG 240

241 ACCTCTTACACCAAGGCCGACAACAGCGTC ATTGTCGCAACCGACTCCATTAAGAACACC 300

↓

301 ATTTACATCACCGCCAAGCAGAACCCCGTT ACTCCTCCCGAGCTGTTCGGCTCCATCCTG 360

361 GGCACACACTTCATTGAGAAGTACAACCAC ATCCATGCCGCTCACGTCAACATTGTCTGC 420

421 CACCGCTGGACCCGGATGGACATTGACGGC AAGCCACACCCTCACTCCTTCATCCGCGAC 480

A*

481 AGCGAGGAGAAGCGGAATGTGCAGGTGGAC GTGGTCGAGGGCAAGGGCATCGATATCAAG 540

541 TCGTCTCTGTCCGGCCTGACCGTGCTGAAG AGCACCAACTCGCAGTTCTGGGGCTTCCTG 600

601 CGTGACGAGTACACCACACTTAAGGAGACC TGGGACCGTATCCTGAGCACCGACGTCGAT 660

661 GCCACTTGGCAGTGGAAGAATTTCAGTGGA CTCCAGGAGGTCCGCTCGCACGTGCCTAAG 720

721 TTCGATGCTACCTGGGCCACTGCTCGCGAG GTCACTCTGAAGACTTTTGCTGAAGATAAC 780

781 AGTGCCAGCGTGCAGGCCACTATGTACAAG ATGGCAGAGCAAATCCTGGCGCGCCAGCAG 840

841 CTGATCGAGACTGTCGAGTACTCGTTGCCT AACAAGCACTATTTCGAAATCGACCTGAGC 900

G*

901 TGGCACAAGGGCCTCCAAAACACCGGCAAG AACGCCGAGGTCTTCGCTCCTCAGTCGGAC 960

961 CCCAACGGTCTGATCAAGTGTACCGTCGGC CGGTCCTCTCTGAAGTCTAAATTGTAAACC 1020

1021 AACATGATTCTCACGTTCCGGAGTTTCCAA GGCAAACTGTATATAGTCTGGGATAGGGTA 1080

1081 TAGCATTCATTCACTTGTTTTTACTTCCA AAAAAAAAAAA...

FIG.3

Séquence nucléotidique du clone 9C et d'une partie du clone 9A

↓ : début du clone 9A

```
109  ATGTCCGCAGTAAAAGCAGCCCGCTACGGC AAGGACAATGTCCGCGTCTACAAGGTTCAC  168
1    MetSerAlaValLysAlaAlaArgTyrGly LysAspAsnValArgValTyrLysValHis  20

169  AAGGACGAGAAGACCGGTGTCCAGACGGTG TACGAGATGACCGTCTGTGTGCTTCTGGAG  228
21   LysAspGluLysThrGlyValGlnThrVal TyrGluMetThrValCysValLeuLeuGlu  40

229  GGTGAGATTGAGACCTCTTACACCAAGGCC GACAACAGCGTCATTGTCGCAACCGACTCC  288
41   GlyGluIleGluThrSerTyrThrLysAla AspAsnSerValIleValAlaThrAspSer  60
                                   V3
289  ATTAAGAACACCATTTACATCACCGCCAAG CAGAACCCCGTTACTCCTCCCGAGCTGTTC  348
61   IleLysAsnThrIleTyrIleThrAlaLys GlnAsnProValThrProProGluLeuPhe  80
                                             T32/T35
349  GGCTCCATCCTGGGCACACACTTCATTGAG AAGTACAACCACATCCATGCCGCTCACGTC  408
81   GlySerIleLeuGlyThrHisPheIleGlu LysTyrAsnHisIleHisAlaAlaHisVal  100

409  AACATTGTCTGCCACCGCTGGACCCGGATG GACATTGACGGCAAGCCACACCCTCACTCC  468
101  AsnIleValCysHisArgTrpThrArgMet AspIleAspGlyLysProHisProHisSer  120

469  TTCATCCGCGACAGCGAGGAGAAGCGGAAT GTGCAGGTGGACGTGGTCGAGGGCAAGGGC  528
121  PheIleArgAspSerGluGluLysArgAsn ValGlnValAspValValGluGlyLysGly  140
                                        T17
529  ATCGATATCAAGTCGTCTCTGTCCGGCCTG ACCGTGCTGAAGAGCACCAACTCGCAGTTC  588
141  IleAspIleLysSerSerLeuSerGlyLeu ThrValLeuLysSerThrAsnSerGlnPhe T31 160
                V5/V6
589  TGGGGCTTCCTGCGTGACGAGTACACCACA CTTAAGGAGACCTGGGACCGTATCCTGAGC  648
161  TrpGlyPheLeuArgAspGluTyrThrThr LeuLysGluThrTrpAspArgIleLeuSer  180

649  ACCGACGTCGATGCCACTTGGCAGTGGAAG AATTTCAGTGGACTCCAGGAGGTCCGCTCG  708
181  ThrAspValAspAlaThrTrpGlnTrpLys AsnPheSerGlyLeuGlnGluValArgSer  200
               T28                              T10
709  CACGTGCCTAAGTTCGATGCTACCTGGGCC ACTGCTCGCGAGGTCACTCTGAAGACTTTT  768
201  HisValProLysPheAspAlaThrTrpAla ThrAlaArgGluValThrLeuLysThrPhe  220
                  T13
769  GCTGAAGATAACAGTGCCAGCGTGCAGGCC ACTATGTACAAGATGGCAGAGCAAATCCTG  828
221  AlaGluAspAsnSerAlaSerValGlnAla ThrMetTyrLysMetAlaGluGlnIleLeu  240
                V2
829  GCGCGCCAGCAGCTGATCGAGACTGTCGAG TACTCGTTGCCTAACAAGCACTATTTCGAA  888
241  AlaArgGlnGlnLeuIleGluThrValGlu TyrSerLeuProAsnLysHisTyrPheGlu T21/ 260
                                             V1
889  ATCGACCTGAGCTGGCACAAGGGCCTCCAA AACACCGGCAAGAACGCCGAGGTCTTCGCT  948
261  IleAspLeuSerTrpHisLysGlyLeuGln AsnThrGlyLysAsnAlaGluValPheAla  280
T11
949  CCTCAGTCGGACCCCAACGGTCTGATCAAG TGTACCGTCGGCCGGTCCTCTCTGAAGTCT  1008
281  ProGlnSerAspProAsnGlyLeuIleLys CysThrValGlyArgSerSerLeuLysSer  300

1009 AAATTGTAA
301  LysLeuEnd
```

# FIG.4

Séquence d'ADN ouverte par l'ATG en position 109 sur la figure 3 et polypeptide codé.

Les peptides séquencés obtenus par hydrolyse de l'urate oxydase d'A. flavus à l'aide de la trypsine et de la protéase V8 sont représentés par des flèches en vis-à-vis du polypeptide codé selon

←——————→ : peptide tryptique

←— - ——— - —→ : peptide obtenu par hydrolyse à l'aide de la protéase V8.

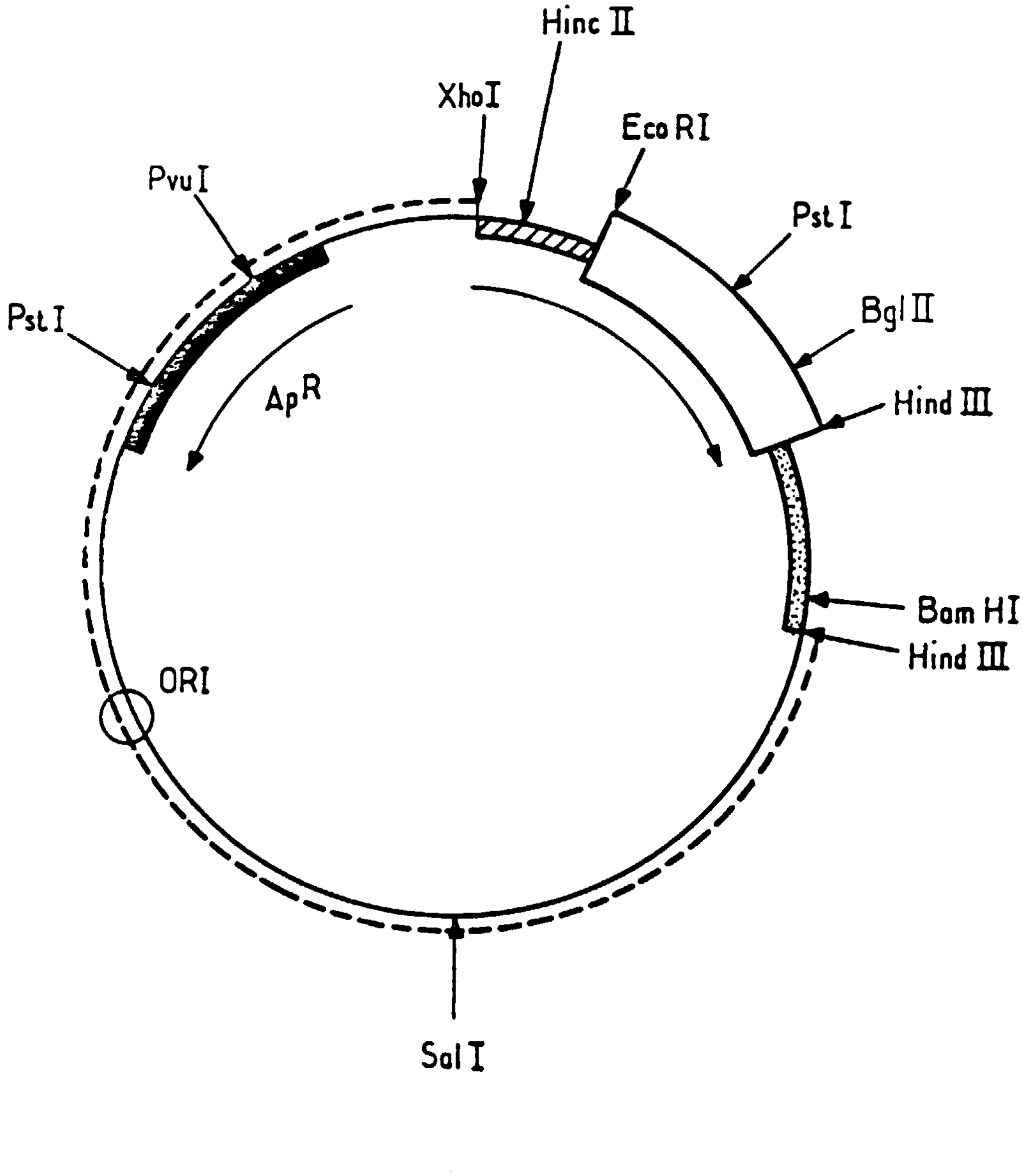


FIG. 5

*Plasmide p 163,1*

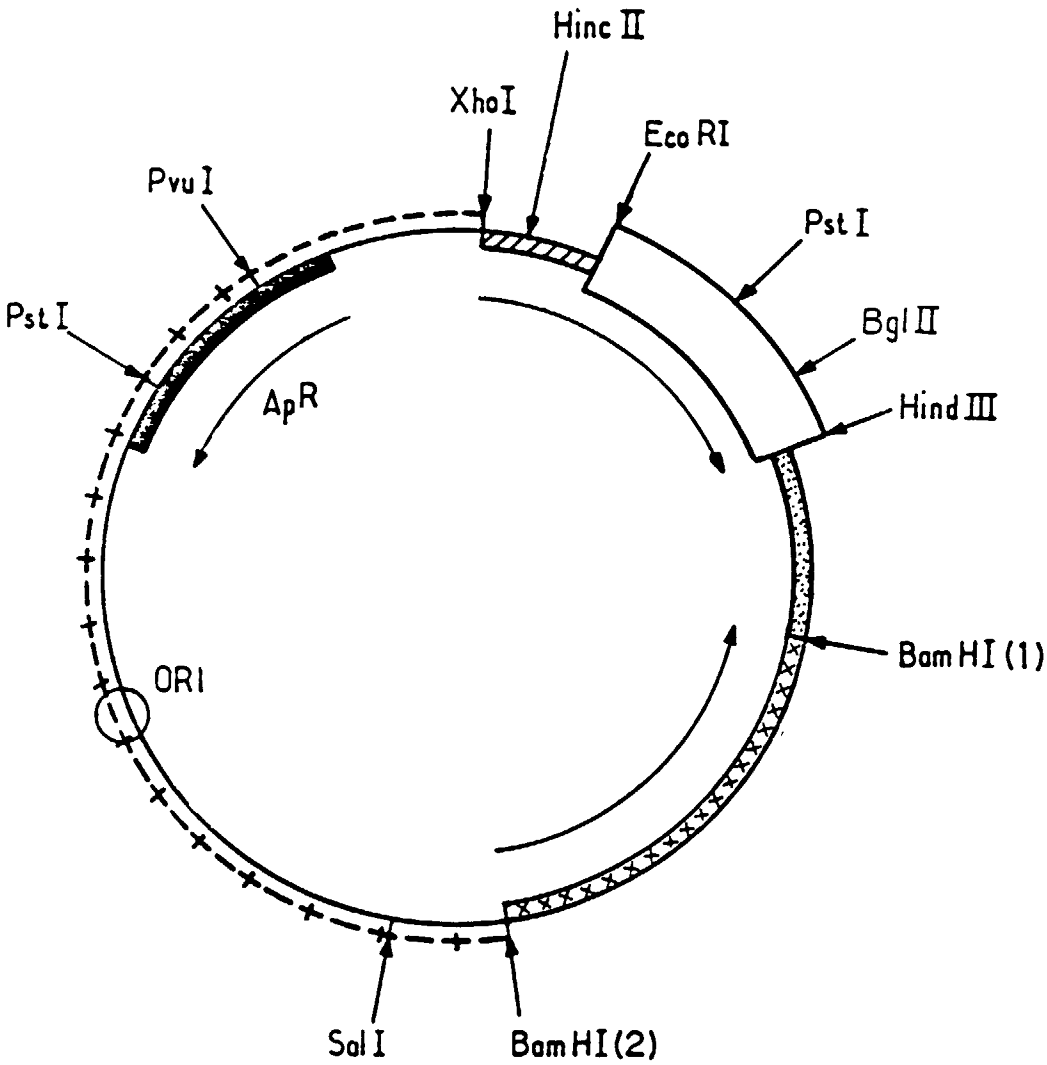


FIG. 6

*Plasmide p 160*

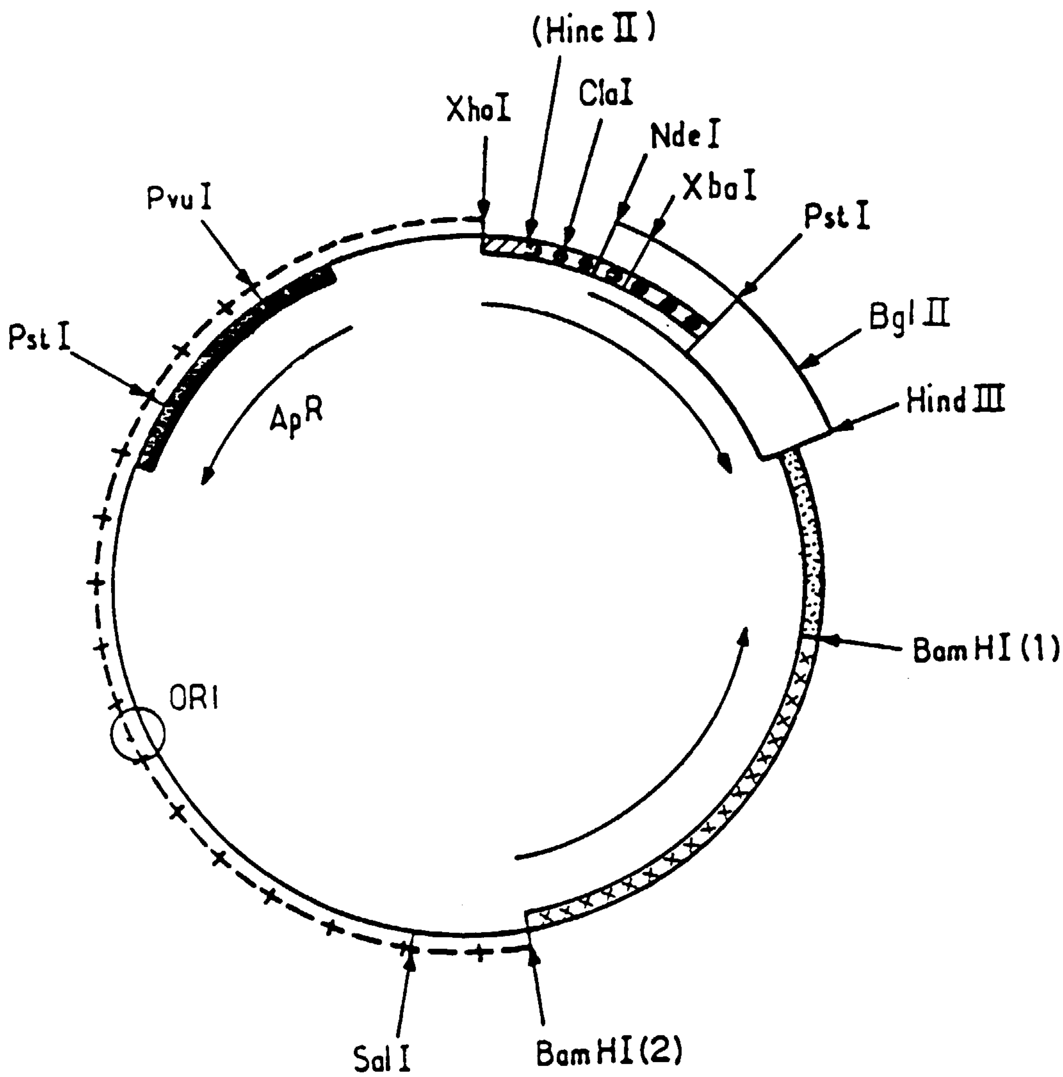


FIG. 7

*Plasmide p 373,2*

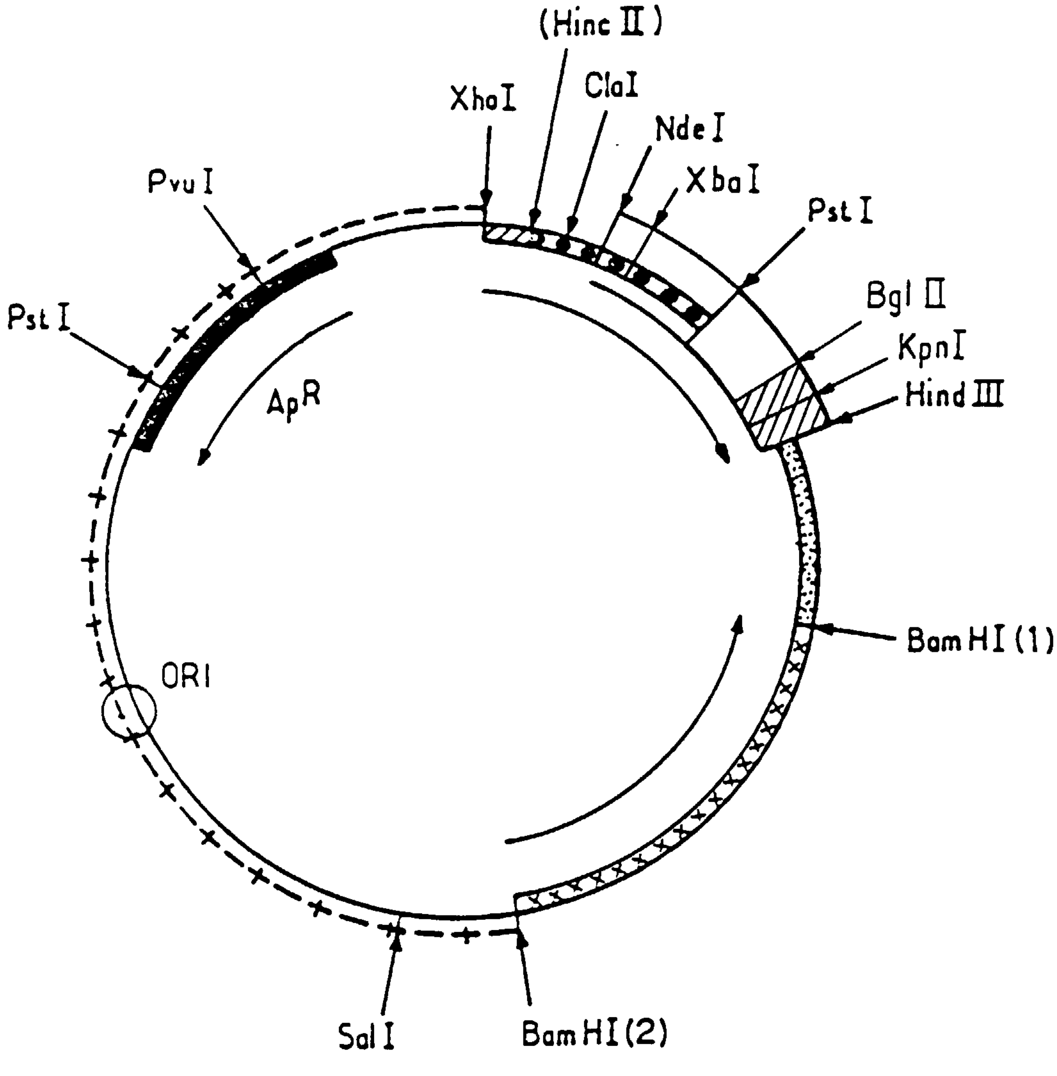


FIG.8

*Plasmide p 462*

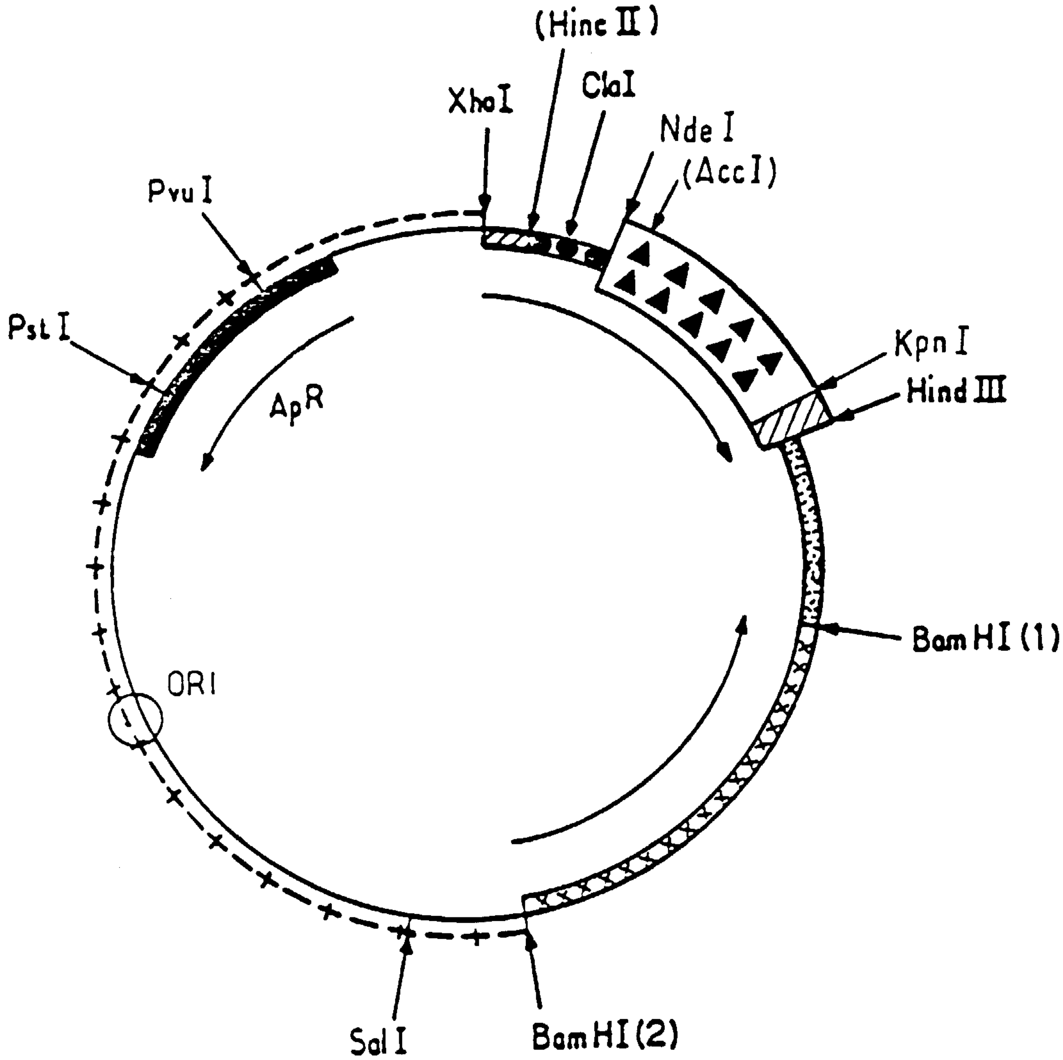


FIG.9

*Plasmide p 466*

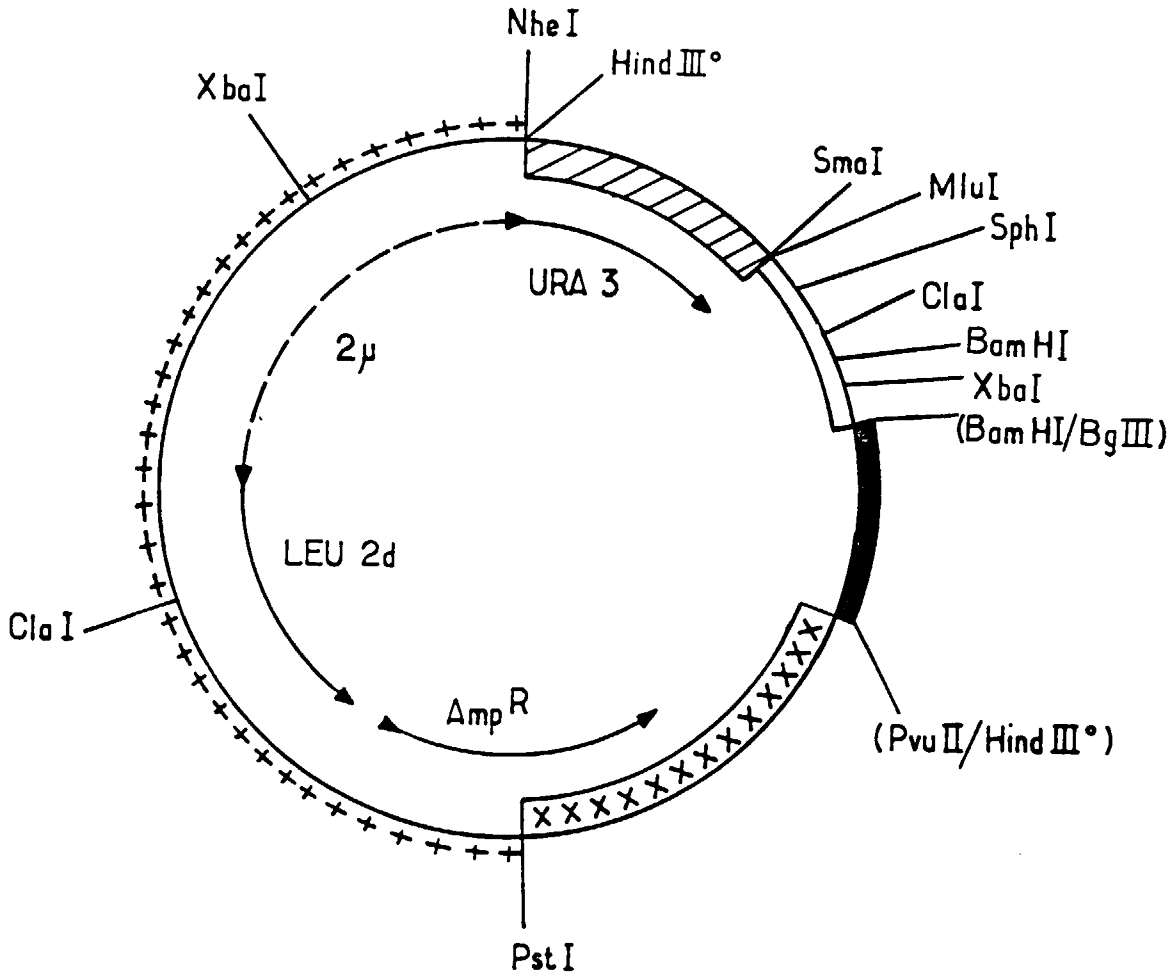


FIG.10

Plasmide pEMR 414

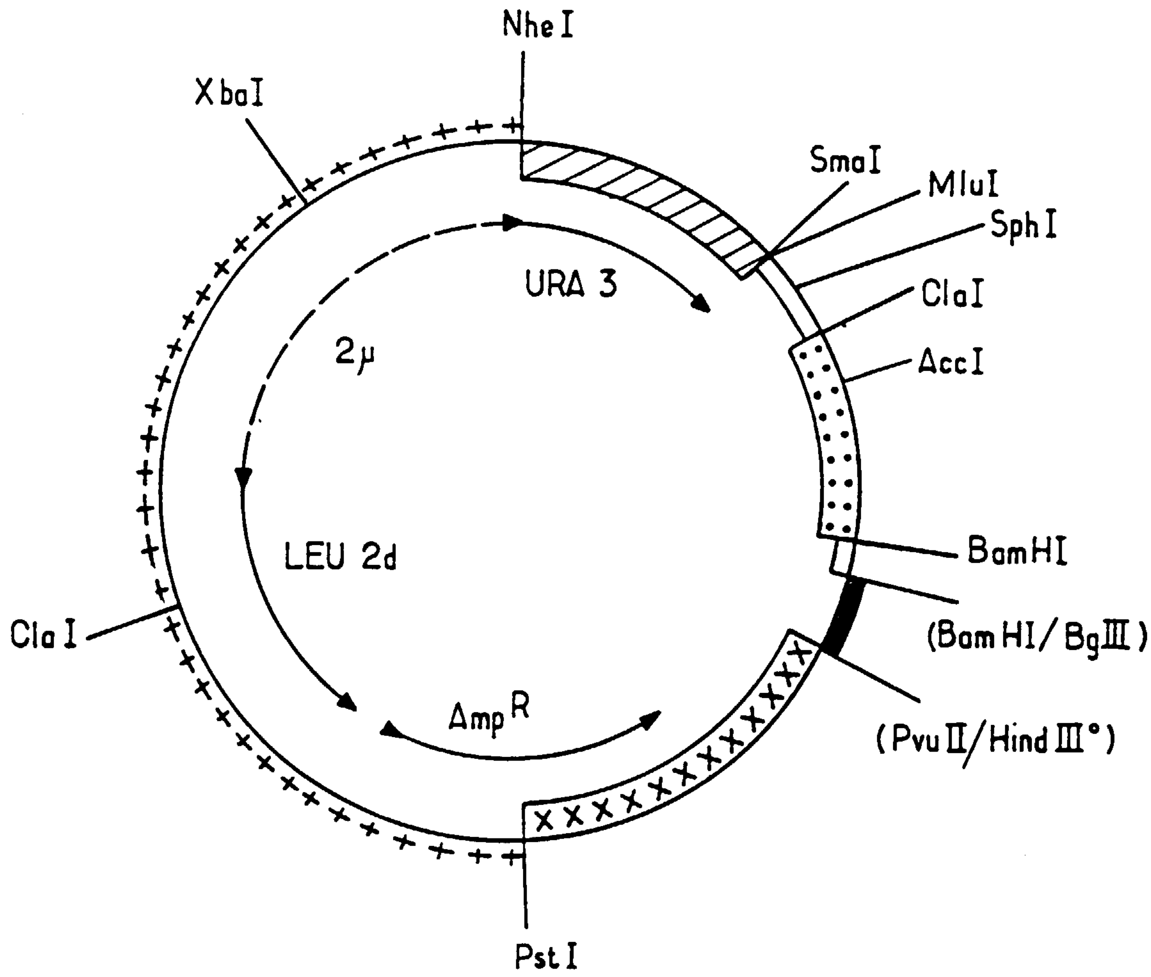


FIG.11

Plasmide pEMR 469

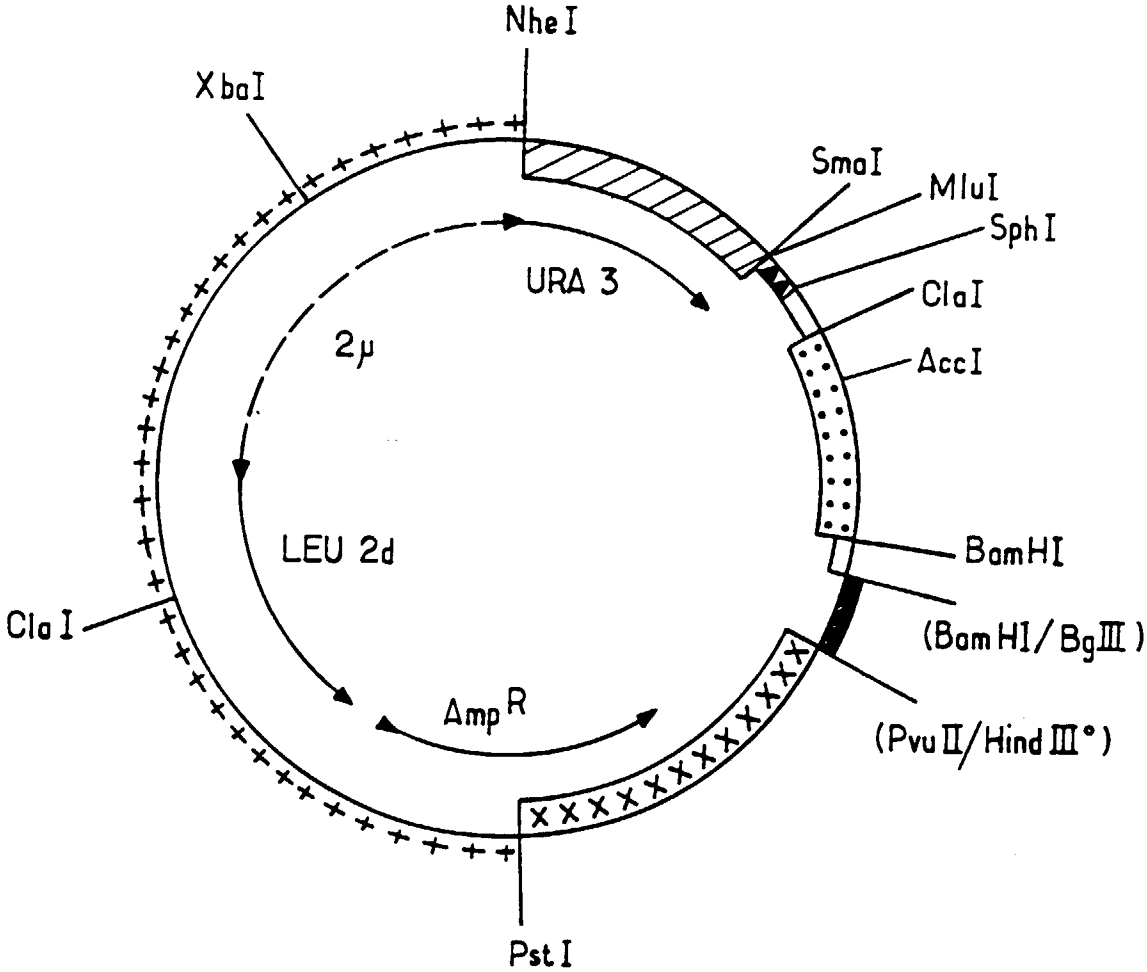


FIG.12

Plasmide pEMR 473

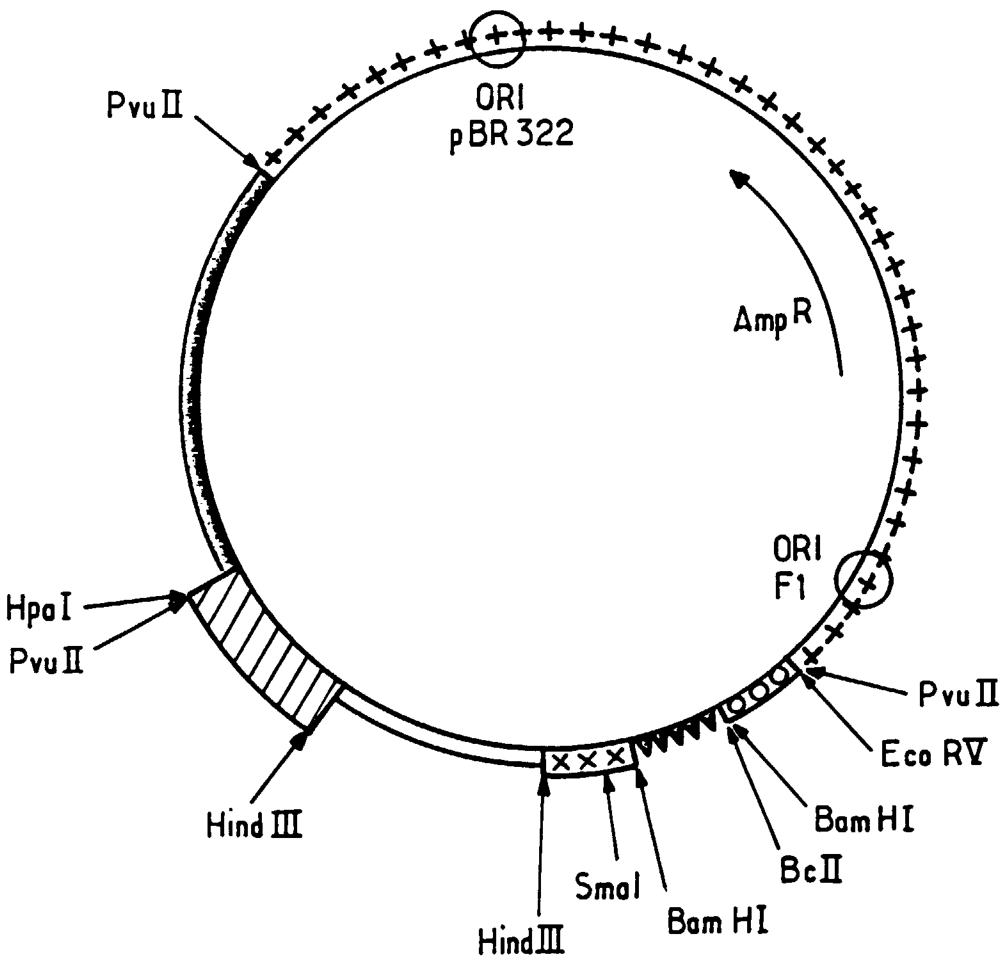


FIG.13

Plasmide PSE 1

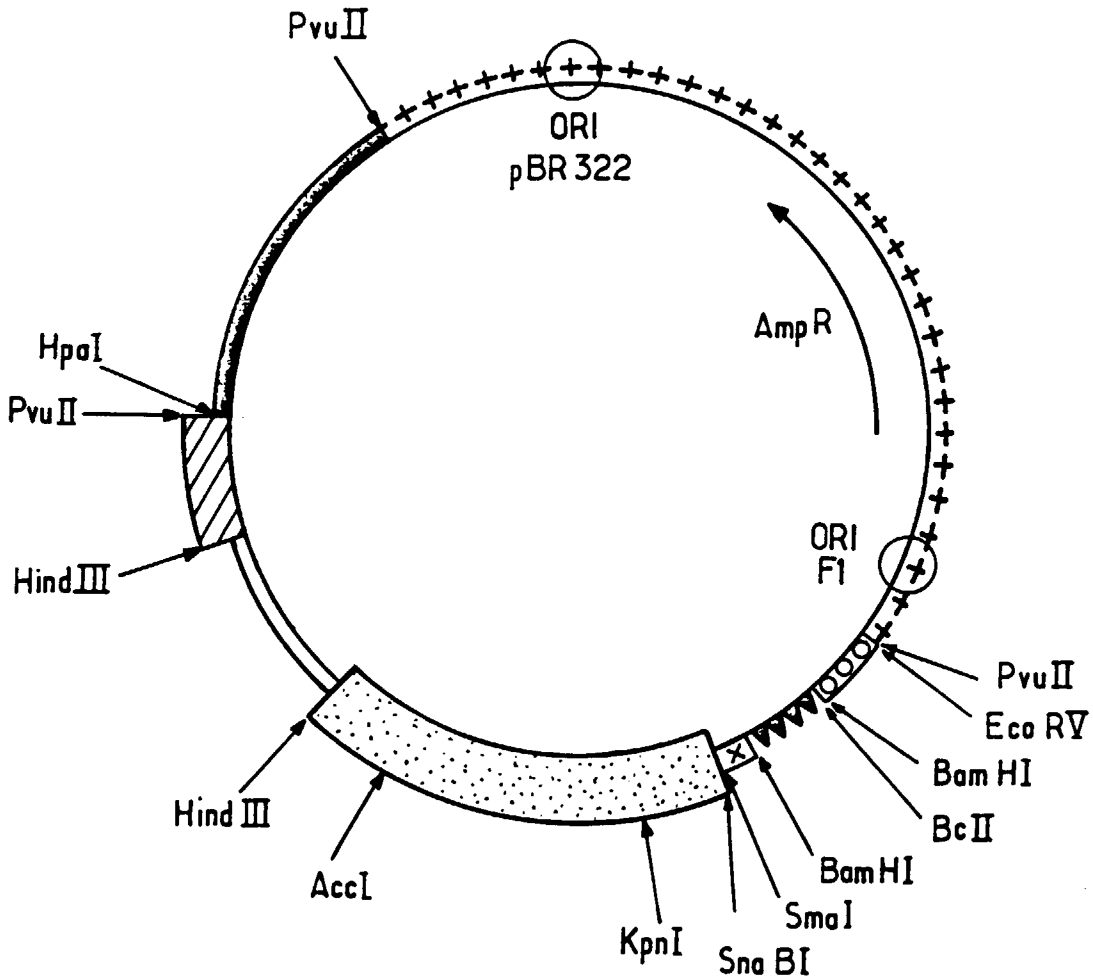


FIG.14

Plasmide pSV860